(19) Europäisches Patentamt
European Patent Office
Office européen des brevets

(11) Veröffentlichungsnummer: **0 168 342 B1**

# (12) EUROPÄISCHE PATENTSCHRIFT

(45) Veröffentlichungstag der Patentschrift: **03.07.91**

(21) Anmeldenummer: **85810268.4**

(22) Anmeldetag: **10.06.85**

(51) Int. Cl.⁵: **C12N 15/15**, C12P 19/34, C12P 21/02, C07K 7/10, A61K 37/64, G01N 33/94, //C12R1/19,C12R1/125, C12R1/865

## (54) Verfahren zur Herstellung von Thrombin-Inhibitoren.

(30) Priorität: **14.06.84 CH 2882/84**

(43) Veröffentlichungstag der Anmeldung:
**15.01.86 Patentblatt 86/03**

(45) Bekanntmachung des Hinweises auf die Patenterteilung:
**03.07.91 Patentblatt 91/27**

(84) Benannte Vertragsstaaten:
**AT BE CH DE FR GB IT LI LU NL SE**

(56) Entgegenhaltungen:
**EP-A- 158 564**

**FEBS LETTERS, vol. 165, no. 2, January 9, 1984, Amsterdam J. DODT et al. "The complete amino acid sequence of hirudin, a thrombin specific inhibitor" pages 180-184**

(73) Patentinhaber: **CIBA-GEIGY AG**
**Klybeckstrasse 141**
**CH-4002 Basel(CH)**

Patentinhaber: **UCP GEN-PHARMA AG**
**Solothurnstrasse 24**
**CH-3422 Kirchberg(CH)**

(72) Erfinder: **Liersch, Manfred, Dr.**
**Rütiring 26**
**CH-4125 Riehen(CH)**
Erfinder: **Rink, Hans**
**Rebenstrasse 10**
**CH-4125 Riehen(CH)**
Erfinder: **Märki, Walter, Dr.**
**Bremenstallstrasse 30**
**CH-4313 Möhlin(CH)**
Erfinder: **Grütter, Markus Gerhard, Dr.**
**Nettenbergweg**
**CH-4146 Hochwald(CH)**
Erfinder: **Meyhack, Bernd, Dr.**
**Höhenweg 9**
**CH-4312 Magden(CH)**

**EP 0 168 342 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Expressionsvektoren enthaltend DNA-Sequenzen, welche für die Aminosäuresequenz des Thrombin-Inhibitors Hirudin kodieren, mit solchen Expressionsvektoren transformierte Wirtszellen, Verfahren zur Herstellung dieser Expressionsvektoren, und transformierten Wirtszellen und Verfahren zur Herstellung des Thrombin-Inhibitors Desulfatohirudin mit Hilfe der transformierten Wirtszellen.

Im Plasma des gesunden Säugetierorganismus besteht ein dynamisches Gleichgewicht zwischen dem fibrinolytischen System und dem Koagulationssystem, wodurch ein funktionstüchtiges Gefässnetz aufrechterhalten wird. Bei Gefässverletzungen lagert das Koagulationssystem eine Fibrin-Matrix ab, welche nach Erreichung des hämostatischen Zustands durch das fibrinolytische System wieder abgebaut wird. In Fällen, in denen das fibrinolytische Potential des Organismus nicht ausreicht, um gebildete intravaskuläre Thrombi abzubauen, z.B. bei Patienten, die unter Thromboembolien oder postoperativen Komplikationen leiden, erweist es sich als unerlässlich, den Organismus durch Verabreichung von thrombolytischen Mitteln oder Antikogulantien zu unterstützen.

In der herkömmlichen Antikoagulationstherapie wurden bisher vor allem Heparin, ferner auch 4-Hydroxycumarin- und 1,3-Indandion-Derivate eingesetzt. Obwohl mit diesen Mitteln bemerkenswerte Erfolge erzielt worden sind, weisen sie doch einige Nachteile auf. So wirkt Heparin nicht direkt auf den Blutgerinnungsvorgang ein, sondern entfaltet seine koagulationshemmende Wirkung indirekt durch Beschleunigung der Hemmung von Thrombin und des Faktors X durch Antithrombin III. Heparin geht im Organismus weiterhin zahlreiche unspezifische Reaktionen ein und wird vom Platelet-Faktor 4 neutralisiert, was insbesondere seine Anwendbarkeit bei der Verbrauchs-Koagulopathie bzw. disseminierten intravasalen Gerinnung (DIC) beeinträchtigt. Es besteht daher ein Bedarf nach alternativen und spezifischeren Mitteln zur Antikoagulationstherapie.

Seit langem ist ein natürliches im Blutegel (Hirudo medicinalis) vorkommendes Antikoagulans, Hirudin, bekannt. Hirudin ist ein aus 65 Aminosäuren bestehendes Polypeptid mit vollständig aufgeklärter Primärstruktur (1) und weist als strukturelle Charakteristika eine Anhäufung hydrophober Aminosäuren am N-Terminus und polarer Aminosäuren am C-Terminus, einen als Sulfatmonoester vorliegenden Tyrosin-Rest ($Tyr^{63}$) und drei Disulfidbrücken, deren exakte Anordnung allerdings noch nicht bekannt ist, auf.

Hirudin ist mit einem $K_i$-Wert (Komplex-Dissoziationskonstante) von $6 \cdot 10^{-11}$ M der stärkste bekannte Thrombin-Inhibitor und durch eine spezifische Affinität zu Thrombin charakterisiert; andere Enzyme der Blutgerinnungskaskade werden nicht inhibiert. Im Gegensatz zu Heparin übt Hirudin seinen inhibierenden Einfluss auf Thrombin direkt aus und wirkt nicht, wie jenes, über Antithrombin III. Der einzige pharmakologisch fassbare Effekt von gereinigtem Hirudin ist die Hemmung der Blutkoagulation bzw. die Prophylaxe von Thrombose. Bei intravenöser Verabreichung an Hunden var selbst bei hohen Dosen kein Einfluss auf Herzschlagfrequenz, Atmung, Blutdruck, Thrombocytenzahl, Fibrinogen und Hämoglobin zu beobachten. Bei Versuchen an der Ratte, am Schwein und am Hund erwies sich Hirudin als wirksam bei experimenteller Thrombose (hervorgerufen entweder durch Stasis oder durch Injektion von Thrombin), beim Endotoxin-Schock sowie bei DIC (disseminierter intravasaler Gerinnung). Bei direkten Vergleichaversuchen mit Heparin, wo immer sie durchgeführt wurden, zeigte sich Hirudin diesem überlegen.

Obwohl seit langem bekannt, erlangte Hirudin bis heute nicht die breite therapeutische Anwendung, die man aufgrund seiner ausgezeichneten biologischen Eigenschaften erwarten durfte. Seiner allgemeinen Anwendung in der Medizin steht nämlich als ein schwerwiegender Nachteil seine äusserst beschränkte Zugänglichkeit im Wege. Bis jetzt waren Hirudin-Präparate ausschliesslich aus teurem und schwer zugänglichem Naturmaterial, ausgehend von Blutegel-Extrakten durch zeit- und kostenaufwendige Isolier- und Reinigungsverfahren, erhältlich (vgl. Ref. 2). Die relativ lange Sequenz von 65 Aminosäuren liess auch die klassische Peptid-Synthese aus ökonomischen Gesichtspunkten wenig erfolgsversprechend erscheinen. Zur Herstellung ausreichender Mengen an Hirudin, welche detaillierte klinische Untersuchungen über dessen therapeutisches Potential und seine breite therapeutische Anwendung in der Antikoagulations-Therapie erst ermöglichen, müssen daher neue Wege beschritten werden.

Hierzu bietet sich insbesondere die rekombinierte DNA-Technologie an. Mit ihrer Hilfe ist es möglich, die unterschiedlichsten physiologisch aktiven Polypeptide durch Kultivierung entsprechend genetisch modifizierter Mikroorganismen oder Säugetierzellkulturen herzustellen. Ein entsprechendes auf rekombinante Gentechnik beruhendes Verfahren zur Herstellung von Hirudin, insbesondere einer als HV2 bezeichneten Hirudin-Variante, die vom Desulfatohirudin gemäss vorliegender Erfindung verschieden ist, zu diesem Zweck bereitgestellte Expressionsvektoren und mit solchen Expressionsvektoren transformierte E. coli-Wirte werden in der nicht vorpublizierten Europäischen Patentanmeldung Nr. 158564 beschrieben.

Der vorliegenden Erfindung liegt die Aufgabe zugrunde, mit Hilfe von gentechnologischen Mitteln ein Expressionssystem zur Verfügung zu stellen, welches die mikrobiologische Herstellung von biologisch

aktivem Desulfatohirudin der Formel

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly (I)
ThrProLysProGlnSerHisAsnAspGlyAspPheGluGluIleProGluGluTyrLeuGln

im industriellen Massstab erlaubt. Diese Aufgabe wird in der vorliegenden Erfindung gelöst durch die Bereitstellung eines Expressionsvektors, welcher eine für besagtes Desulfatohirudin kodierende DNA-Sequenz enthält, die von einer Expressionskontrollsequenz derart kontrolliert wird, dass in einem mit diesem Expressionsvektor transformierten Mikroorganismus besagtes Desulfatohirudin exprimiert wird.

Es war zu erwarten, dass die Expression derartiger DNA-Sequenzen in transformierten Wirtszellen (mit Ausnahme von transformierten Egelzellen) in Abwesenheit eines zusätzlich eingebauten Sulfat-übertragenden Enzym-Systems zu einem Produkt führen würde, dass sich von natürlichem Hirudin im wesentlichen durch das Fehlen des Sulfatrestes am Tyrosin-63 unterscheidet. Die biologische Funktion des Sulfatrestes in Proteinen im allgemeinen und im Hirudin im besonderen ist im einzelnen nicht definitiv geklärt; jedoch soll der Sulfat-Rest nach dem jetzigen Kenntnisstand die physiologischen Eigenschaften des Proteins nicht unwesentlich beeinflussen. So soll der Sulfat-Rest folgende Funktionen ausüben:

(1) Positive Beeinflussung der biologischen Aktivität des Proteins;

(2) Beteiligung an regulatorischen Zellprozessen (wie es bei der reversiblen Phosphorylierung bekannt ist);

(3) Stimulierung der Sekretion, d.h. die Sulfatierung dient als Markierung für die Erkennung als sekretorisches Protein: Alle bekannten sulfatierten Proteine sind sekretorische oder transmembranale Proteine.

Ueberraschenderweise wurde jetzt gefunden, dass entgegen den theoretischen Vorstellungen die wertvollen biologischen Eigenschaften des Hirudins auch dann erhalten bleiben, wenn die charakteristische Sulfatmonoester-Gruppe vom phenolischen Hydroxyl des $Tyr^{63}$-Restes entfernt wird. Die mit Hilfe der erfindungsgemässen transformierten Mikroorganismen hergestellte Hirudin-Verbindung ohne Sulfatrest ("Desulfatohirudin") erweist sich nämlich gegenüber dem natürlichen Hirudin in ihren biologischen Eigenschaften, insbesondere in ihrer antikoagulativen Wirkung, qualitativ und quantitativ als zumindest ebenbürtig.

Herstellung von DNA-Sequenzen, die für die Aminosäuresequenz von Hirudin kodieren

Das Verfahren zur Herstellung von DNA-Sequenzen, die für die Aminosäuresequenz von Desulfatohirudin der Formel I kodieren und von Fragmenten davon ist dadurch gekennzeichnet, dass man aus genomischer Egel-DNA das Hirudin-Strukturgen isoliert, oder aus Hirudin-mRNA eine komplementäre doppelsträngige Hirudin-DNA (Hirudin-ds cDNA) herstellt, oder dass man ein für besagte Aminosäuresequenz kodierendes Gen oder Fragmente davon mittels chemischer und enzymatischer Verfahren herstellt.

Die Gewinnung von genomischer Hirudin-DNA und von Hirudin-ds cDNA erfolgt beispielsweise nach an sich bekannten Methoden. So gewinnt man genomische Hirudin-DNA beispielsweise aus einer Egel-Genbank, welche das Hirudin-Gen enthält, indem man die Egel-DNA-Fragmente in einem Mikroorganismus kloniert und Klone, die Hirudin-DNA enthalten, identifiziert, beispielsweise durch Kolonie-Hybridisierung unter Verwendung eines radioaktiv markierten, Hirudin-DNA spezifischen Oligodesoxynucleotids, welches mindestens 15, vorzugsweise 15 bis 30, Desoxynucleotide enthält. Die so erhaltenen DNA-Fragmente enthalten neben dem Hirudin-Gen in der Regel weitere unerwünschte DNA-Bestandteile, welche durch Behandlung mit geeigneten Exo- oder Endonucleasen abgespalten werden können.

Doppelsträngige Hirudin-cDNA kann beispielsweise hergestellt werden, indem man aus geeigneten, vorzugsweise zur Hirudin-Bildung induzierten Egelzellen mRNA gewinnt, des so erhaltene mRNA-Gemisch in an sich bekannter Weise an Hirudin-mRNA anreichert, diese mRNA als Templat für die Herstellung von einsträngiger cDNA verwendet, daraus mit Hilfe einer RNA-abhängigen DNA-Polymerase die ds cDNA synthetisiert und diese in einen geeigneten Vektor kloniert. Klone, die Hirudin-cDNA enthalten, werden beispielsweise, wie oben beschrieben, durch Koloniehybridisierung unter Verwendung eines radioaktiv markierten, Hirudin-DNA spezifischen Oligodesoxynucleotids identifiziert.

Die auf diese Weise gewonnene genomische Hirudin-DNA oder die Hirudin-cDNA werden vorzugsweise am 5'- und am 3'-Ende mit chemisch synthetisierte Adapter-Oligodesoxynucleotiden verknüpft, welche die Erkennungssequenz für eine oder verschiedene Restriktionsendonuclease(n) enthalten und damit den Einbau in geeignete Vektoren erleichtern. Zusätzlich muss das Adaptermolekül für das 5'-Ende der Hirudin-DNA bzw. -cDNA noch das Translationsstartsignal (ATG) enthalten. Das Translationsstartsignal muss derart angeordnet sein, dass ihm das Codon für die erste Aminosäure des Hirudins direkt folgt.

Da die Struktur des natürlichen Hirudin-Gens nicht bekannt ist und die chemische Synthese eines für die Aminosäuresequenz des Hirudins kodierenden Gens ("Desulfatohirudin-Gen") auf Grund der modernen Synthesemöglichkeiten Vorteile, insbesondere in zeitlicher Hinsicht, bietet, ist die chemische Synthese bevorzugt.

Chemische Synthese eines Desulfatohirudin-Gens

Das Verfahren zur Herstellung eines Strukturgens für Desulfatohirudin der Formel I oder von Fragmenten davon ist dadurch gekennzeichnet, dass man Segmente des kodierenden und des komplementären Stranges des Desulfatohirudin-Gens chemisch synthetisiert und erhältliche Segmente enzymatisch in ein Strukturgen des Desulfatohirudins oder in Fragmente davon überführt.

Die DNAs enthalten zusätzlich zu den Codons für Desulfatohirudin Translations-Start- und Stopsignale, die eine Expression in geeigneten Mikroorganismen beispielsweise E. coli, ermöglichen, ferner Nukleotidsequenzen an den Enden, die für einen Einbau in einen Vektor geeignet sind.

Bevorzugt umfasst die DNA am 5'-Ende eine durch ein Restriktionsenzym schneidbare Nukleotidsequenz gefolgt vom Translations-Startsignal, Codons für die Aminosäuren von Hirudin, die gegebenenfalls an einer oder mehrenen Stellen das Schneiden durch ein Restriktionsenzym ermöglichen, ein Translations-Stopsignal und am 3'-Ende eine durch ein Restriktionsenzym schneidbare Nukleotidsequenz. Erfindungsgemäss anwendbare Restriktionsenzyme sind beispielsweise EcoRI, EcoRII, BamHI, HpaII, PstI, HinfI oder HindIII.

Eine bevorzugte doppelsträngige DNA besteht aus einer Nukleotidsequenz der Formel I und der komplementären Nukleotidsequenz

| | | | | | | | |
|---|---|---|---|---|---|---|---|
| 5' $(X')_n$ | Met<br>ATG | | | | | | |
| Val<br>GTX | Val<br>GTX | Tyr<br>TAY | Thr<br>ACX | Asp<br>GAY | Cys<br>TGY | Thr<br>ACX | Glu<br>GAM |
| Ser<br>QRS | Gly<br>GGX | Gln<br>CAM | Asn<br>AAY | Leu<br>YTZ | Cys<br>TGY | Leu<br>YTZ | Cys<br>TGY |
| Glu<br>GAM | Gly<br>GGX | Ser<br>QRS | Asn<br>AAY | Val<br>GTX | Cys<br>TGY | Gly<br>GGX | Gln<br>CAM |
| Gly<br>GGX | Asn<br>AAY | Lys<br>AAM | Cys<br>TGY | Ile<br>ATN | Leu<br>YTZ | Gly<br>GGX | Ser<br>QRS |
| Asp<br>GAY | Gly<br>GGX | Glu<br>GAM | Lys<br>AAM | Asn<br>AAY | Gln<br>CAM | Cys<br>TGY | Val<br>GTX |
| Thr<br>ACX | Gly<br>GGX | Glu<br>GAM | Gly<br>GGX | Thr<br>ACX | Pro<br>CCX | Lys<br>AAM | Pro<br>CCX |
| Gln<br>CAM | Ser<br>QRS | His<br>CAY | Asn<br>AAY | Asp<br>GAY | Gly<br>GGX | Asp<br>GAY | Phe<br>TTY |
| Glu<br>GAM | Glu<br>GAM | Ile<br>ATN | Pro<br>CCX | Glu<br>GAM | Glu<br>GAM | Tyr<br>TAY | Leu<br>YTZ |
| Gln<br>CAM | NON<br>TMK | $(X')_m$ 3' | | | | | |

worin die Nukleotidsequenz beginnend mit dem 5'-Ende dargestellt ist und zum besseren Verständnis die

Aminosäuren, für die jedes Triplett codiert, angegeben sind, und worin bedeuten

A Desoxyadenosyl,
T Thymidyl,
G Desoxyguanosyl,
C Desoxycytidyl,
X A, T, C oder G,
Y T oder C,

Z A, T, C oder G, wenn Y = C,
oder Z = A oder G, wenn Y = T,
Q 7 oder A,
R C und S = A, T, C oder G, wenn Q = T,
oder R = G und S = T oder C, wenn Q = A,
M A oder G,
L A oder C,
N T, C oder A
K A oder G, wenn M = A,
oder K = A, wenn M = G,

und $(X')_n$ und $(X')_m$ Nukleotidsequenzen mit n und m grösser als 3 und kleiner als 100, insbesondere grösser als 5 und kleiner als 15, bedeuten, welche durch ein Restriktionsenzym erkannt und geschnitten werden können.

Bevorzugt enthält die DNA-Sequenz am 5'-Ende eine durch EcoRI spaltbare, in der Mitte eine durch EcoRII spaltbare und am 3'-Ende eine durch BamHI spaltbare Nukleotidsequenz.

Die doppelsträngige DNA, enthält insbesondere von E. coli bevorzugte und für die Aminosäuren von Hirudin kodierende Tripletts. Solche Tripletts sind insbesondere für

| | |
|---|---|
| Glycin (Gly) | GGT |
| Cystein (Cys) | TGC |
| Valin (Val) | GTT |
| Leucin (Leu) | CTG |
| Serin (Ser) | TCT |
| Threonin (Thr) | ACC |
| Phenylalanin (Phe) | TTC |
| Tyrosin (Tyr) | TAC |
| Methionin (Met) | ATG |
| Asparaginsäure (Asp | ) GAC |
| Glutaminsäure (Glu) | GAA |
| Lysin (Lys) | AAA |
| Isoleucin (Ile) | ATC |
| Histidin (His) | CAC |
| Prolin (Pro) | CCG |
| Glutamin (Gln) | CAG |
| Asparagin (Asn) | AAC |

Bevorzugtes Stopsignal (NON) ist der Codon TAG.

Eine bevorzugtes für die Aminosäuresequenz von Hirudin kodierendes Gen in der oben dargestellten Weise ist die DNA der Formel II,

```
               MetValValTyrThrAspCysThrGluSerGlyGlnAsnLeuCysLeuCysGluGly
            CTGGAATTCATGGTTGTTTACACCGACTGCACCGAATCTGGTCAGAACCTGTGCCTGTGCGAAGGT
            GACCTTAAGTACCAACAAATGTGGCTGACGTGGCTTAGACCAGTCTTGGACACGGACACGCTTCCA
               (EcoRI)

SerAsnValCysGlyGlnGlyAsnLysCysIleLeuGlySerAspGlyGluLysAsnGlnCysVal
TCTAACGTTTGCGGTCAGGGTAACAAATGCATCCTGGGTTCTGACGGTGAAAAAAACCAGTGCGTT
AGATTGCAAACGCCAGTCCCATTGTTTACGTAGGACCCAAGACTGCCACTTTTTTTGGTCACGCAA
                                  (EcoRII)

ThrGlyGluGlyThrProLysProGlnSerHisAsnAspGlyAspPheGluGluIleProGluGlu
ACCGGTGAAGGTACCCCGAAACCGCAGTCTCACAACGACGGTGACTTCGAAGAAATCCCGGAAGAA
TGGCCACTTCCATGGGGCTTTGGCGTCAGAGTGTTGCTGCCACTGAAGCTTCTTTAGGGCCTTCTT

TyrLeuGlnNON
TACCTGCAGTAGGATCCTG
ATGGACGTCATCCTAGGAC
           (BamHI)              (II),
```

worin A, T, G, C die unter Formel I angegebenen Bedeutungen haben und zum besseren Verständnis die Aminosäuren, für die jedes Triplett kodiert, und die Schnittstellen für die Restriktionsenzyme angegeben sind.

Für die Herstellung eines Strukturgens für Desulfatohirudin nützliche doppelsträngige DNA-Fragmente desselben sind besonders solche, deren Enden durch Restriktionsenzyme geschnitten werden können. Solche doppelsträngigen DNA-Fragmente des Hirudin-Gens haben insbesondere 30 bis 70 Basenpaaren und sind beispielsweise solche der Formel III ($F_1$) und der Formel IV ($F_2$):

```
      MetValValTyrThrAspCysThrGluSerGlyGlnAsnLeuCysLeuCysGluGly
CTGGAATTCATGGTTGTTTACACCGACTGCACCGAATCTGGTCAGAACCTGTGCCTGTGCGAAGGT
GACCTTAAGTACCAACAAATGTGGCTGACGTGGCTTAGACCAGTCTTGGACACGGACACGCTTCCA
   (EcoRI)

SerAsnValCysGlyGlnGlyAsnLysCysIleLeuGlySer
TCTAACGTTTGCGGTCAGGGTAACAAATGCATCCTGGGTTCTG
AGATTGCAAACGCCAGTCCCATTGTTTACGTAGGACCCAAGAC
                                (EcoRII)

                                                 F1 (III)

 IleLeuGlySerAspGlyGluLysAsnGlnCysValThrGlyGluGlyThrProLysPro
CATCCTGGGTTCTGACGGTGAAAAAAACCAGTGCGTTACCGGTGAAGGTACCCCGAAACCG
GTAGGACCCAAGACTGCCACTTTTTTTGGTCACGCAATGGCCACTTCCATGGGGCTTTGGC
  (EcoRII)

GlnSerHisAsnAspGlyAspPheGluGluIleProGluGluTyrLeuGlnNON
CAGTCTCACAACGACGGTGACTTCGAAGAAATCCCGGAAGAATACCTGCAGTAGGATCCTG
GTCAGAGTGTTGCTGCCACTGAAGCTTCTTTAGGGCCTTCTTATGGACGTCATCCTAGGAC
                                                     (BamHI)

                     F2 (IV)
```

Methoden der Synthese von DNA sind von S.A. Narang (3) zusammenfassend dargestellt worden. Die bekannten Synthesetechniken erlauben die Herstellung von Polynukleotiden mit einer Länge von gegen 20 Basen in guter Ausbeute, hoher Reinheit und in verhältnismässig kurzer Zeit. Geeignet geschützte Nukleotide werden durch die Phosphodiestermethode (4), die noch effizientere Phosphotriestermethode (5) oder Phosphit-Triestermethode (6) miteinander verknüpft. Eine Vereinfachung der Synthese der Oligo- und Polynukleotide wird mit der Festphasen-Methode ermöglicht, bei der die Nukleotidketten an ein geeignetes

Polymer gebunden sind. Itakura et al. (7) verwenden bei der Festphasen-Synthese anstelle einzelner Nukleotide durch Phosphotriester-Methode geknüpfte Trinukleotide, die so in kurzer Zeit und guten Ausbeuten beispielsweise zu einem Polynukleotid mit 31 Basen kondensiert werden können. Die eigentliche doppelsträngige DNA kann aus chemisch hergestellten kurzen Segmenten enzymatisch aufgebaut werden. Khorana et al. (8) verwenden dazu überlappende Polynukleotid-Sequenzen aus beiden DNA-Strängen, die durch Basen-Paarung in richtiger Anordnung zusammengehalten und dann durch das Enzym DNA-Ligase chemisch verknüpft werden. Eine weitere Möglichkeit besteht darin, je eine Polynukleotid-Sequenz aus den beiden DNA-Strängen mit einem kurzen überlappenden Segment in Gegenwart der vier benötigten Desoxynukleosid-Triphosphate mit einer DNA-Polymerase, z.B. DNA-Polymerase I, Klenow-Fragment der Polymerase I, $T_4$ DNA-Polymerase, oder mit AMV (avian myeloblastosis virus) reverse Transcriptase, zu inkubieren. Dabei werden durch Basenpaarung die beiden Polynukleotid-Sequenzen in der richtigen Anordnung zusammengehalten und durch das Enzym mit den benötigten Nukleotiden zu einer vollständigen doppelsträngigen DNA ergänzt (9). Itakura et al. (10) beschreiben, wie basierend auf diesem Prinzip in Gegenwart von DNA-Polymerase I (Klenow-Fragment) aus 4 chemisch synthetisierten Fragmenten einer Länge von 39 bis 42 Basen ein 132 Basen-Paare langes Segment des humanen Leukozyten-Interferon $\alpha_2$-Gens aufgebaut werden kann, wobei 40 % Einsparung an chemischer Synthese gegenüber der oben beschriebenen Methode, bei der nur Ligase verwendet wird, erzielt werden.

Das Verfahren zur Herstellung von DNAs, welche ein Strukturgen des Desulfatohirudins enthalten und für eine Expression in Wirtszellen geeignet sind, und deren Enden einen Einbau in Vektoren ermöglichen, und von Fragmenten davon,ist au sich bikannt und dadurch gekennzeichnet, dass

a) ein geeignet geschütztes Desoxynukleosid an einen festen Träger gebunden wird,

b) geeignet geschützte Di-, Tri- oder Tetranukleotide nach der Phosphotriester- oder Phosphit-Methode hergestellt werden,

c) ein an den Träger gebundenes Desoxynukleosid oder Oligodesoxynukleotid mit geeignet geschützten Mono- oder (gemäss b) hergestellten) Di-, Tri- oder Tetranukleotiden nach der Phosphotriester-oder der Phosphit-Methode verknüpft wird,

d) nach c) erhältliche, an Träger gebundene Oligodesoxynukleotide mit einer Länge zwischen etwa 20 und etwa 70 Basen vom Träger abgespalten, gegebenenfalls gereinigt, von Schutzgruppen befreit und die freien 5'-terminalen Hydroxygruppen phosphoryliert werden,

e1) 2 Oligodesoxynukleotide einer Länge von je etwa 20 bis etwa 70 Basen aus dem kodierenden und aus dem komplementären Strang mit mindestens 3, vorzugsweise 8 bis 15, überlappenden Basenpaaren annelliert und mit einer DNA-Polymerase in Gegenwart der vier Desoxynukleosid-Triphosphate zu doppelsträngigen DNA-Segmenten (Fragmenten des Desulfatohirudin-Gens) ergänzt werden, und gegebenenfalls 2 doppelsträngige DNA-Segmente mit geeigneten gemäss d) phosphorylierten Enden mit einer Ligase zum Desulfatohirudin-Strukturgen verknüpft werden, oder 2 erhältliche doppelsträngige DNA-Segmente in geeignete Vektoren subkloniert, anschliessend gemäss d) phosphoryliert und mit einer Ligase zum Desulfatohirudin-Strukturgen verknüpft werden, oder

e2) alternativ je 2 Oligodesoxynukleotide aus dem kodierenden und aus dem komplementären Strang mit einer Länge von z.B. 20 bis 70 Basen und mit je mindestens 3, vorzugsweise 8 bis 15, überlappenden Basenpaaren annelliert, mit einer DNA-Polymerase in Gegenwart der vier Desoxynukleosid-Triphosphate aufgefüllt und mit Ligase zum Desulfatohirudin-Strukturgen verknüpft werden.

<u>Herstellung von Expressionsvektoren, welche ein für die Aminosäuresequenz von Hirudin kodierendes Gen enthalten</u>

Die Erfindung betrifft Expressionsvektoren, welche eine für Desulfatohirudin der Formel I kodierende DNA-Sequenz enthalten, die von einer Expressionskontrollsequenz derart reguliert wird, dass in einem mit diesen Expressionsvektoren transformierten Mikroorganismus besagtes Desulfatohirudin exprimiert wird.

Die Expressionsvektoren der vorliegenen Erfindung werden z.B. hergestellt, indem man in eine Vektor-DNA, welche eine Expressionskontrollsequenz enthält, eine für Desulfatohirudin der Formel I kodierende DNA-Sequenz derart einfügt, dass die Expressionskontrollsequenz besagte DNA-Sequenz reguliert.

Die Wahl eines geeigneten Vektors ergibt sich aus dem zur Transformation vorgesehenen Mikroorganismus Geeignete Mikroorganismen sind z.B. Hefen, z.B. Saccharomyces cerevisiae, und insbesondere Bakterienstämme, die über kein Restriktions- oder Modifikationsenzym verfügen, vor allem Stämme von Escherichia coli, z.B. E. coli X1776, E. coli HB101, E. coli W3110 Δ102, E. coli HB101/LM1035, E. coli JA221(30) oder E. coli K12 Stamm 294, Bacillus subtilis, Bacillus stearothermophilus, Pseudomonas, Haemophilus, Streptococcus und andere, Bevorzugte Mikrooganismen sind die genannten Stämme von E. coli, insbesondere E. coli HB101, E. coli JA221 und E. coli W3110Δ102.

Grundsätzlich sind alle Vektoren geeignet, welche die erfindungsgemässen heterologen, für die Aminosäuresequenz von Hirudin kodierenden DNA-Sequenzen in dem gewählten Mikroorganismus replizieren und exprimieren.

Beispiele für Vektoren, die zur Expression des Desulfatohirudin-Gens in einem E. coli-Stamm geeignet sind, sind Bacteriophagen, z.B. Derivate des Bacteriophagen λ, oder Plasmide, wie insbesondere das Plasmid colE1 und seine Derivate, z.B. pMB9, pSF2124, pBR317 oder pBR322. Die bevorzugten Vektoren der vorliegenen Erfindung leiten sich von Plasmid pBR322 ab. Geeignete Vektoren enthalten ein vollständiges Replicon und ein Markierungsgen, welches die Selektion und Identifizierung der mit den Expressionsplasmiden transformierten Mikroorganismen auf Grund eines phänotypischen Merkmals ermöglicht. Geeignete Markierungsgene verleihen dem Mikrooganismus beispielsweise Resistenz gegenüber Schwermetallen, Antibiotika und dergleichen. Weiterhin enthalten bevorzugte Vektoren der vorliegenden Erfindung ausserhalb der Replicon- und Markierungagen-Regionen Erkennungssequenzen für Restriktionsendonucleasen, so dass an diesen Stellen die für die Aminosäuresequenz von Hirudin kodierende DNA-Sequenz und gegebenenfalls die Expressions-Kontrollsequenz eingefügt werden können. Der bevorzugte Vektor, das Plasmid pBR322, enthält ein intaktes Replicon, gegen Tetracyclin und Ampicillin Resistenz verleihende Markierungsgene ($tet^R$ und $amp^R$) und eine Reihe von nur einmal vorkommenden Erkennungssequenzen für Restriktionsendonucleasen, z.B. PstI (schneidet im $amp^R$-Gen, das $tet^R$-Gen bleibt intakt), BamHI, HindIII, SalI (schneiden alle im $tet^R$-Gen, das $amp^R$-Gen bleibt intakt), NruI und EcoRI.

Mehrere Expressionskontrollsequenzen können zur Regulation der Desulfatohirudin-Expression eingesetzt werden. Insbesondere werden Expressionskontrollsequenzen stark exprimierter Gene der zu transformierenden Wirtszelle verwendet. Im Falle von pBR322 als Hybridvektor und E. coli als Wirtsmikroorganismus sind beispielsweise die Expressionskontrollsequenzen (welche unter anderem den Promotor und die ribosomale Bindungsstelle enthalten) des Lactoseoperons, Tryptophanoperons, Arabinoseoperons und dergleichen, des β-Lactamasegens, die entsprechenden Sequenzen des Phage λN-Gens oder des Phage fd-Schichtproteingens und andere geeignet. Während der Promotor des β-Lactamasegens (β-lac-Gen) bereits im Plasmid pBR322 enthalten ist, müssen die übrigen Expressionskontrollsequenzen in das Plasmid eingeführt werden. Bevorzugt als Expressionskontrollsequenz in der vorliegenden Erfindung ist diejenige des Tryptophanoperons (trp po).

Zur Replikation und Expression in Hefe geeignete Vektoren enthalten einen Hefe-Replikationsstart und einen selektiven genetischen Marker für Hefe. Hybridvektoren, die einen Hefe-Replikationsstart enthalten, z.B. das chromosomale autonom replizierende Segment (ars), bleiben nach der Transformation innerhalb der Hefezelle extrachromosomal erhalten und werden bei der Mitose autonom repliziert. Ferner können Hybridvektoren, die der Hefe-2μ-Plasmid-DNA homologe Sequenzen enthalten, verwendet werden. Solche Hybridvektoren werden durch Rekombination innerhalb der Zelle bereits vorhandenen 2μ-Plasmiden einverleibt oder replizieren autonom. 2μ-Sequenzen sind besonders für Plasmide mit grosser Transformationshäufigkeit geeignet und gestatten eine hohe Kopienzahl. Geeignete Markierungsgene für Hefe sind vor allem solche, die dem Wirt antibiotische Resistenz verleihen oder, im Fall von auxotrophen Hefemutanten, Gene, die Wirtsdefekte komplementieren. Entsprechende Gene verleihen z.B. Resistenz gegen das Antibiotikum Cycloheximid oder sorgen für Prototrophie in einer auxotrophen Hefemutante, z.B. das URA3-, LEU2-, HIS3- oder vor allem das TRP1-Gen. Vorzugsweise enthalten Hefe-Hybridvektoren weiterhin einen Replikationsstart und ein Markierungsgen für einen bakteriellen Wirt, insbesondere E. coli, damit die Konstruktion und die Klonierung der Hybridvektoren und ihrer Vorstufen in einem bakteriellen Wirt erfolgen kann. Für die Expression in Hefe geeignete Expressionskontrollsequenzen sind beispielsweise diejenigen des TRP1-, ADHI-, ADHII-, PHO3- oder PHO5-Gens, ferner im glykolytischen Abbau involvierte Promoter, z.B. der PGK- und der GAPDH-Promoter.

Insbesondere betrifft die Erfindung zur Replikation und phänotypischen Selektion befähigte Expressionsvektoren, welche eine Expressionskontrollsequenz und eine für die Aminosäuresequenz von Hirudin kodierende DNA-Sequenz enthalten, wobei besagte DNA-Sequenz mitsamt Transkriptionsstartsignal und -terminationssignal sowie Translationsstartsignal und -stopsignal in besagtem Expressionsplasmid unter Regulation besagter Expressionskontrollsequenz so angeordnet ist, dass in einer mit besagtem Expressionsplasmid transformierten Wirtszelle Desulfatohirudin exprimiert wird.

Um eine effektive Expression zu erreichen, muss das Desulfatohirudin-Gen richtig (in "Phase") mit der Expressionskontrollsequenz angeordnet sein. Es ist vorteilhaft, die Expressionskontrollsequenz in den Bereich zwischen dem Haupt-mRNA-Start und dem ATG der Genkodiersequenz, welche natürlich mit der Expressionskontrollsequenz verknüpft ist (z.B. die β-lac-Kodiersequenz bei Verwendung des β-lac-Promotors), mit dem Desulfatohirudin-Gen, welches vorzugsweise sein eigenes Translationsstartsignal (ATG) und Translationsstopsignal (z.B. TAG) mitbringt, zu verknüpfen. Dadurch wird eine effektive Transkription und Translation gewährleistet.

Beispielsweise wird ein Vektor, insbesondere pBR322, mit einer Restriktionsendonuclease geschnitten und, gegebenenfalls nach Modifikation des so gebildeten linearisierten Vektors, eine mit entsprechenden Restriktionsenden versehene Expressionakontrollsequenz eingeführt. Die Expressionskontrollsequenz enthält am 3'-Ende (in Translationsrichtung) die Erkennungssequenz einer Restriktionsendonuclease, sodass der die Expressionskontrollsequenz bereits enthaltende Vektor mit besagtem Restriktionsenzym verdaut und das mit passenden Enden versehene Desulfatohirudin-Gen eingesetzt werden kann. Dabei entsteht ein Gemisch von zwei Hybridplasmiden, welche das Gen in richtiger bzw. in falscher Orientierung enthalten. Vorteilhaft ist es, den die Expressionskontrollsequenz bereits enthaltenden Vektor noch mit einer zweiten Restriktionsendonuclease innerhalb der Vektor-DNA zu spalten und in das entstandene Vektor-Fragment das mit richtigen Enden versehene Desulfatohirudin-Gen einzusetzen. Alle Operationen am Vektor erfolgen vorzugsweise in einer Weise, dass die Funktion des Replicons und zumindest eines Markierungsgens nicht beeinträchtigt wird.

In einer bevorzugten Ausführungsform der vorliegenden Erfindung wird ein von pBR322 abgeleiteter Vektor, welcher eine Expressionskontrollsequenz, insbesondere diejenige vom Tryptophan-Operon (trp po), enthält, die am 3'-Ende (zwischen dem Haupt-mRNA-Start und dem ersten ATG) die Erkennungssequenz für eine, vorzugsweise kohäsive Enden bildende, Restriktionsendonuclease, z.B. EcoRI, trägt, mit der genannten Restriktionsendonuclease und im Vektor-DNA-Teil mit einer zweiten Restriktionsendonuclease, welche flache oder bevorzugt kohäsive Enden bildet, z.B. BamHI, verdaut, wonach der so linearisierte Vektor mit dem entsprechende Enden aufweisenden Desulfatohirudin-Gen (z.B. mit einem EcoRI-Ende vor dem ATG-Start und einem BamHI-Ende nach dem Translationsstop-Codon) verknüpft wird. Die Verknüpfung erfolgt in bekannter Weise durch Paarung der komplementären (kohäsiven) Enden und Ligierung, z.B. mit $T_4$-DNA-Ligase.

Das über den mRNA-Weg, aus genomischer DNA oder synthetisch gewonnene, mit entsprechenden kohäsiven (insbesondere EcoRI- und BamHI-) Enden versehene Desulfatohirudin-Gen kann vor dem Einbringen in ein Expressionsplasmid auch in einen Vektor, z.B. pBR322, kloniert werden, um grössere Mengen an Desulfatohirudin-Gen, beispielsweise für die Sequenzanalyse, zu gewinnen. Die Isolierung der Klone, welche das Hybridplasmid enthalten, wird beispielsweise mit einer Desulfatohirudin-Gen-spezifischen, radioaktiv-markierten Oligodesoxynucleotid-Probe (siehe oben) durchgeführt. Die Charakterisierung des Desulfatohirudin-Gens erfolgt beispielsweise nach dem Verfahren von Maxam und Gilbert (11).

In einer weiteren Ausführungsform der Erfindung werden zwei Teilstücke des Desulfatohirudin-Gens synthetisiert. Teilstück 1, welches den 1. Teil des Gens umfasst, enthält vor dem ATG und am Ende jeweils die Erkennungssequenz für kohäsive Enden bildende Restriktionsendonucleasen, z.B. vor dem ATG EcoRI und am Ende EcoRII. Teilstück 2, welches den hinteren Teil des Gens umfasst, besitzt entsprechende Erkennungssequenzen, am Anfang z.B. EcoRII und nach dem Translationsstopsignal (z.B. TAG) BamHI. Die Teilstücke werden an den äusseren Erkennungssequenzen gespalten (Teilstück 1 z.B. mit EcoRI und Teilstück 2 entsprechend mit BamHI) und in einen entsprechend zugeschnittenen Vektor (z.B. pBR322) subkloniert. Die Identifizierung der Klone, welche die Teilstücke enthalten, und die Charakterisierung der Teilstücke erfolgt wie oben beschrieben. Die Teilstücke werden dann mit den entsprechenden Restriktionsendonucleasen aus den Hybridvektoren herausgeschnitten (Teilstück 1 z.B. mit EcoRI und EcoRII und Teilstück 2 z.B. mit EcoRII und BamHI) und über ihre kohäsiven Enden, insbesondere EcoRII-Enden, ligiert, wobei das komplette Desulfatohirudin-Gen entsteht, das wie angegeben in eine Vektor-DNA eingefügt wird.

Transformation der Mikroorganismen

Die Erfindung betrifft auch ein Verfahren zur Herstellung eines transformierten Mikroorganismus, dadurch gekennnzeichnet, dass man einen Mikroorganismus mit einem Expressionsvektor, der eine von einer Expressionskontrollsequenz regulierte, für die Aminosäuresequenz von Hirudin kodierende DNA-Sequenz enthält, transformiert.

Geeignete Mikroorganismen sind beispielsweise die oben genannten, wie Stämme von Saccharomyces cerevisiae, Bacillus subtilis und insbesondere Escherichia coli. Die Transformation mit den erfindungsgemässen Expressionsplasmiden erfolgt beispielsweise wie in der Literatur beschrieben, so für S. cerevisiae (12), B. subtilis (13) und E. coli (14). Die Isolierung der transformierten Wirtszellen erfolgt vorteilhaft aus einem selektiven Nährmedium, dem das Biocid zugesetzt wird, gegen welches das im Expressionsplasmid enthaltene Markierungs-Gen Resistenz verleiht. Wenn, wie bevorzugt, die Expressionsplasmide das $amp^R$-Gen enthalten, wird dem Nährmedium demgemäss Ampicillin zugesetzt. Zellen, welche das Expressionsplasmid nicht enthalten, werden in einem solchen Medium abgetötet.

Die Erfindung betrifft ebenfalls die auf dem genannten Weg erhältlichen transformierten Mikrooganismen.

Kultivierung der transformierten Mikroorganismen und Gewinnung von Desulfatohirudin

Die transformierten Mikrooganismen werden zur Herstellung von biologisch aktiven Desulfatohirudin der Formel I verwendet. Das Verfahren zur Herstellung dieser Verbindung ist dadurch gekennzeichnet, dass die transformierten Mikroorganismen kultiviert und besagtes Desulfatohirudin aus den Wirtszellen freigesetzt und isoliert wird.

Unter "biologisch aktivem Desulfatohirudin" wird ein Desultfatohirudin-Produkt verstanden, welches eine Thrombin-inhibierende Wirkung und eine positive Reaktion mit anti-Hirudin-Antikörpern zeigt und welche; die Primärstruktur von Desulfatohirudin der Formel I aufweist.

Die Erfindung betrifft vor allem ein Verfahren zur Herstellung von Desulfatohirudin der Formel I und von Salzen davon, dadurch gekennzeichnet, dass mit einem Expressionsvektor, welcher eine von einer Expressionskontrollsequenz regulierte, für besagte Hirudin-Verbindung kodierende DNA-Sequenz enthält, transformierte Mikrooganismen in einem flüssigen Nährmedium, welches assimilierbare Kohlenstoff-und Stickstoffquellen enthält, kultiviert werden und das Produkt aus den Wirtszellen freigesetzt und isoliert wird, und, falls gewünscht, ein erhaltenes Salz in das freie Polypeptid und ein erhaltenes Polypeptid in ein Salz desselben überführt wird.

In den verfahrensgemäss erhältlichen Verbindungen mit Hirudin-Aktivität, insbesondere in den Verbindungen der Formel XIV, sind die Cystein-Reste Cys in gleicher Anordnung wie im natürlichen Hirudin paarweise durch Disulfid-Brücken verknüpft.

Die Kultivierung der erfindungsgemässen transformierten Mikrooganismen erfolgt in an sich bekannter Weise. So können für die Kultivierung der erfindungsgemässen, transformierten Wirtsmikroorganismen verschiedene Kohlenstoffquellen verwendet werden. Beispiele bevorzugter Kohlenstoffquellen sind assimilierbare Kohlenhydrate, wie Glucose, Maltose, Mannit oder Lactose, oder ein Acetat, das entweder allein oder in geeigneten Gemischen verwendet werden kann. Geeignete Stickstoffquellen sind beispielsweise Aminosäuren, wie Casaminosäuren, Peptide und Proteine und ihre Abbauprodukte, wie Trypton, Pepton oder Fleischextrakte; weiterhin Hefeextrakte, Malzextrakt, wie auch Ammoniumsalze, z.B. Ammoniumchlorid, -sulfat oder -nitrat, die entweder allein oder in geeigneten Mischungen verwendet werden können. Anorganische Salze, die ebenfalls verwendet werden können, sind z.B. Sulfate, Chloride, Phosphate und Carbonate von Natrium, Kalium, Magnesium und Calcium.

Weiterhin enthält das Medium z.B. wachstumsfördernde Substanzen, wie Spurenelemente, z.B. Eisen, Zink, Mangan und dergleichen, und vorzugsweise Substanzen, die einen Selektionsdruck ausüben und das Wachstum von Zellen, die das Expressionsplasmid verloren haben, verhindern. So wird dem Medium beispielsweise Ampicillin zugesetzt, wenn das Expressionsplasmid ein $amp^R$-Gen enthält. Ein solcher Zusatz von antibiotisch wirksamen Substanzen bewirkt auch, dass kontaminierende, Antibiotika-empfindliche Mikroorganismen abgetötet werden.

Die Kultivierung erfolgt nach an sich bekannten Verfahren. Die Kultivierungsbedingungen, wie Temperatur, pH-Wert des Mediums und Fermentationszeit, werden so ausgewählt, dass maximale Hirudin-Titer erhalten werden. So wird ein E. coli- oder ein Hefe-Stamm bevorzugt unter aeroben Bedingungen in submerser Kultur unter Schütteln oder Rühren bei einer Temperatur von etwas 20 bis 40 °C, vorzugsweise etwa 30 °C, und einem pH-Wert von 4 bis 9, vorzugsweise bei pH 7, während etwa 4 bis 20 h, vorzugsweise 8 bis 12 h, kultiviert. Dabei sammelt sich das Expressionsprodukt intrazellulär an.

Wenn die Zelldichte einen ausreichenden Wert erreicht hat, wird die Kultivierung abgebrochen und das Produkt aus den Zellen des Mikroorganismus freigesetzt. Zu diesem Zweck werden die Zellen zerstört, z.B. durch Behandlung mit einem Detergens, wie SDS oder Triton, oder mit Lysozym oder einem gleichartig wirkenden Enzym lysiert. Alternativ oder zusätzlich kann man mechanische Kräfte, wie Scherkräfte (z.B. X-Presse, French-Presse, Dyno-Mill) oder Schütteln mit Glasperlen oder Aluminiumoxid, oder abwechselndes Einfrieren, z.B. in flüssigem Stickstoff, und Auftauen, z.B. auf 30 ° bis 40 °C, sowie Ultraschall zum Brechen der Zellen anwenden. Die resultierende Mischung, die Proteine, Nucleinsäuren und andere Zellbestandteile enthält, wird nach der Zentrifugation in an sich bekannter Weise an Proteinen angereichert. So wird z.B. der grösste Teil der Nicht-Protein-Bestandteile durch Polyäthylenimin-Behandlung abgetrennt und die Proteine einschliesslich Desulfatohirudin z.B. durch Sättigen der Lösung mit Ammoniumsulfat oder mit anderen Salzen ausgefällt. Bakterielle Proteine können auch mittels Ansäuern mit Essigsäure (z.B. 0,1 %, pH 4-5) ausgefällt werden. Eine weitere Anreicherung an Desulfatohirudin kann durch Extraktion des essigsauren Ueberstandes mit n-Butanol erreicht werden. Weitere Reinigungsschritte umfassen bespielsweise chromatographische Verfahren, wie Ionenaustauschchromatographie, Gelpermeationschromatographie, Partitionschromatographie, HPLC, Umkehrphasen-HPLC und dergleichen. So erfolgt die Auftrennung der Mischbestandteile durch Dialyse, nach Ladung mittels Gel- oder trägerfreier Elektrophorese, nach Molekülgrösse mittels einer geeigneten Sephadex-Säule, durch Affinitätschromatographie, z.B. mit Antikörpern, insbesondere

monoklonalen Antikörpern, oder mit Thrombin gekuppelt an einen geeigneten Träger zur Affinitätschromatographie, oder durch weitere, insbesondere aus der Literatur bekannte Verfahren.

Beispielsweise umfasst die Isolierung des exprimierten Desulfatohirudins die folgenden Stufen:
Abtrennung der Zellen aus der Kulturlösung mittels Zentrifugation;
Herstellung eines Rohextrakts durch Zerstören der Zellen, z.B. durch Behandlung mit einem lysierenden Enzym und/oder abwechselndem
Einfrieren und Wiederauftauen;
Abzentrifugieren der unlöslichen Bestandteile;
Ausfällen der DNA durch Zugabe von Polyäthylenimin;
Fällung der Proteine durch Ammoniumsulfat;
Affinitätschromatographie des gelösten Niederschlags an einer monoklonalen Anti-Desulfatohirudin-Antikörper-Säule oder einer Thrombin-Säule;
Entsalzung der so gewonnen Lösung mittels Dialyse oder Chromatographie an Sephadex G25 oder Sephadex G10.

Alternativ können nach Abtrennen der DNA die bateriellen Proteine mittels 1%iger Essigsäure ausgefällt werden, aus dem sauren Ueberstand die Hirudin-Verbindung mit n-Butanol extrahiert oder der saure Ueberstand direkt der Ionenaustausch-Chromatographie (z.B. an Diäthylaminoäthylcellulose DEAE 53) unterworfen werden. Weitere Reinigungsschritte schliessen Gelfiltration an Sephadex G50 (oder G75), und Umkehrphasen-HPLC ein. Entsalzung erfolgt wieder an Sephadex G25.

Zum Nachweis der Hirudin-Aktivität kann der Test mit Anti-Hirudin- oder Anti-Desulfatohirudin-Antikörpern (z.B. aus Kaninchen erhältliche, oder aus Hybridomazellen erhältliche monoklonale Antikörper), der Thrombin-Test (15) oder der Blutgerinnungstest (16) herangezogen werden.

Die erfindungsgemäss herstellbare Verbindung der Formel I, kann nicht nur in freier Form, sondern auch in Form ihrer Salze, insbesondere ihrer pharmazeutisch annehmbaren Salze, vorliegen. Da sie mehrere Aminosäurereste mit freien Aminogruppen enthält, kann die erfindungsgemässe Verbindung z.B. in Form von Säureadditionssalzen vorliegen. Als Säureadditionssalze kommen insbesondere physiologisch verträgliche Salze mit üblichen, therapeutisch anwendbaren Säuren in Betracht; als anorganische Säuren sind die Halogenwasserstoffsäuren, wie die Chlorwasserstoffsäure, aber auch Schwefelsäure und Phosphor- bzw. Pyrophosphorsäure zu nennen; als organische Säuren sind in erster Linie Sulfonsäuren, wie die Benzol- oder p-Toluolsulfonsäure oder Niederalkansulfonsäuren, wie Methansulfonsäure, sowie Carbonsäuren, wie Essigsäure, Milchsäure, Palmitin- und Stearinsäure, Aepfelsäure, Weinsäure, Ascorbinsäure und Citronensäure, geeignet. Da die Hirudin-Verbindung auch Aminosäurereste mit freien Carboxylgruppen enthalten, kann sie auch als Metallsalz, insbesondere als Alkalimetall- oder Erdalkalimetallsalz, z.B. Natrium-, Kalium-, Calcium- oder Magnesiumsalz, oder auch als Ammoniumsalz, abgeleitet von Ammoniak oder einer physiologisch verträglichen, organischen stickstoffhaltigen Base, vorliegen. Da sie aber zugleich freie Carboxylgruppen und freie Aminogruppen enthält, kann sie auch als inneres Salz vorliegen.

Je nach Arbeitsweise erhält man die erfindungsgemässe Verbindung in freier Form, in Form von Säureadditionssalzen oder Salzen mit Basen. Aus den Säureadditionssalzen und den Salzen mit Basen kann in an sich bekannter Weise, beispielsweise durch Einstellen des pH-Wertes auf den isoelektrischen Punkt, die freie Verbindung gewonnen werden. Von letzterer wiederum lassen sich durch Umsetzen mit Säuren bzw. Basen, z.B. mit solchen, die die obengenannten Salze bilden, und Eindampfen oder Lyophilisieren therapeutisch annehmbare Säureadditionssalze bzw. Salze mit Basen gewinnen.

Das gemäss der vorliegenden Erfindung erhältliche Desulfatohirudin weist wertvolle pharmakologische Eigenschaften auf und kann, wie aus Blutegeln extrahiertes Hirudin, prophylaktisch oder insbesondere therapeutisch angewendet werden.

Die erfindungsgemäss herstellbare Desulfatohirudin-Verbindung erweist sich hinsichtlich ihrer biologischen Aktivität als zumindest äquivalent zu natürlichem Hirudin. Sie weist beispielsweise einen $K_I$-Wert von ca. $10^{-11}$ H auf. Desulfatohirudin ist vollständig spezifisch für Thrombin und zeigt keinerlei Wechselwirkungen mit anderen Proteinasen des Blutgerinnungssystems. Die aktive Toxizität ist äusserst gering; so sind in der Ratte bei Dosen von z.B. 1 g/kg keinerlei toxische Anzeichen festzustellen. Desgleichen werden keinerlei Hypersensitivitäts- und Allergie-Reaktionen beobachtet.

Die erfindungsgemäss herstellbare Desulfatohirudin-Verbindung kann daher in Analogie zu natürlichem Hirudin zur Therapie und Prophylaxe von Thrombosen und Thromboembolien, einschliesslich zur Prophylaxe von postoperativen Thrombosen, zur akuten Schock-Therapie (z.B. bei septischem oder polytraumatischem Schock), zur Therapie von Verbrauchskoagulopathien, bei Hämodialysen, Hämoseparationen und im extrakorporalen Kreislauf verwendet werden.

Insbesondere betrifft die Erfindung die in den Beispielen beschriebenen Expressionsvektoren, enthaltend eine fur die Aminosauresequenz von Hirudin kodierende DNA-Sequenz, mit solchen Expressionsvektoren transformierte Mikroorganismen, die in den Beispielen beschriebenen Verfahren zu ihrer Herstellung und das in den Beispielen beschriebene Verfahren zur Herstellung von Desulfatohirudin mit Hilfe der transformierten Mikroorganismen.

Die folgenden Beispiele und Zeichnungen dienen zur Illustration der Erfindung und sollen sie in keiner Weise einschränken.

Experimenteller Teil

Die in den Beispielen verwendeten Abkürzungen haben die folgende Bedeutung:

| | |
|---|---|
| TNE | Lösung, die 100 mM NaCl, 50 mM Tris•HCl pH 7.5 und 5 mM EDTA enthält, |
| SDS | Natriumdodecylsulfat |
| EDTA | Aethylendiamintetraessigsäure |
| DTT | 1,4-Dithiothreitol (1,4-Dimercapto-2,3-butandiol) |
| BSA | Rinder-Serumalbumin |
| EtBr | Ethidiumbromid |
| Tris | Tris-(hydroxymethyl)-aminomethan |
| Tris•HCl | Monohydrochlorid von Tris |

Beispiel 1: Herstellung des geschützten Nucleosid-Polystyrol-Harzes

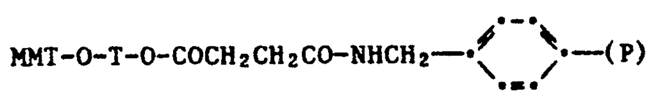

2,57 g (5 mMol) 5'-(4-Methoxytrityl)-thymidin (MMT-O-T-OH) in 20 ml abs. Pyridin werden mit 750 mg Bernsteinsäureanhydrid und 910 mg 4-Dimethylaminopyridin versetzt und 16 Stunden bei Raumtemperatur belassen. Nach Einengen der Pyridin-Lösung wird in 200 ml Essigsäureäthylester aufgenommen, zweimal mit je 200 ml 0,1 M Phosphatpuffer unter Zusatz von 10 ml gesättigter Kochsalz-Lösung ausgeschüttelt, nochmals mit gesättigter Kochsalzlösung gewaschen, getrocknet, eingeengt und Hexan zugetropft. Das ausgefallene Produkt wird abgetrennt und zweimal mit Aether zerrieben, dann in 300 ml Essigsäureäthylester gelöst und bei 0 °C mit 180 ml 0,1 M Kaliumhydrogensulfat von pH 2,5 ausgeschüttelt. Nach zweimaligem Waschen mit Wasser wird die Essigesterlösung mit Natriumsulfat getrocknet, filtriert, mit 0,5 ml Pyridin versetzt, eingeengt und tropfenweise mit Hexan verdünnt. Das ausgefallene Bernsteinsäurederivat wird abfiltriert.

1,0 g dieser Verbindung werden zusammen mit 190 mg N-Hydroxysuccinimid in 4 ml Essigsäureäthylester und 2 ml Dimethylformamid gelöst und bei 0 °C mit 370 mg N,N'-Dicyclohexylcarbodiimid versetzt. Nach Stehen über Nacht im Kühlschrank wird der ausgefallene N,N'-Dicyclohexylharnstoff abfiltriert, das Filtrat mit Essigsäureäthylester verdünnt, mit kaltem 0,1 M Natriumhydrogencarbonat und Wasser extrahiert, getrocknet und im Vakuum zur Trockne eingedampft. Der Rückstand wird mit Essigsäureäthylester an Kieselgel chromatographiert. DC: $R_f$ 0,45 in Dichlormethan-Methanol (9:1).

100 mg dieses N-Succinimidoyl-bernsteinsäureesters werden mit 1 g Aminomethyl-polystyrol (Amingehalt 110 $\mu$Mol/g) in 2 ml Dichlormethan und 4 ml Dimethylformamid 20 Stunden gerührt. Das Polymer-Harz wird abfiltriert und mit Dimethylformamid, Methanol, Dichlormethan und Methanol ausgewaschen. Nach dem Trocknen werden die nicht umgesetzten Aminogruppen acetyliert, indem das Harz in 6 ml Pyridin mit 1 ml Essigsäureanhydrid und 100 mg 4-Dimethylaminopyridin 30 min. gerührt wird. Das Polymer-Harz wird mit Dichlormethan, Dimethylformamid, Methanol und Dichlormethan ausgewaschen und bis zur Gewichtskonstanz getrocknet. Die spektroskopische Methoxytrityl (MMT)-Bestimmung ergibt eine Beladung von 57 $\mu$Mol/g.

Beispiel 2:

Analog Beispiel 1 wird das folgende geschützte Nucleosid-Polystyrol-Harz hergestellt:

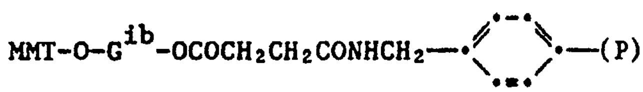

aus 5'-(4-Methoxytrityl)-N-isobutyryl-deoxyguanosin, Beladung 32 $\mu$Mol/g.

Beispiel 3: Synthese des Trinukleotids

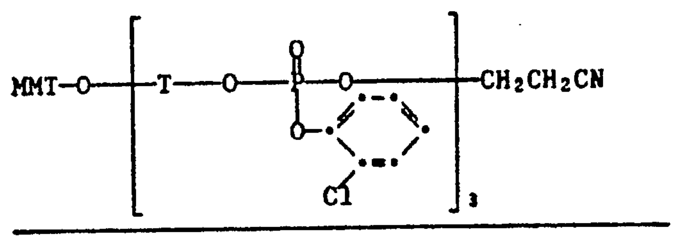

a) Synthese des Dinukleotids:

7.73 g (15 mMol) 5'-(4-Methoxytrityl)-thymidin (MMT-O-T-OH) werden zweimal mit abs. Pyridin eingedampft. Der Rückstand wird in 20 ml abs. Tetrahydrofuran gelöst und zu 80 ml einer 0,2 M Lösung von 2-Chlorphenyl-di-(1-benzotriazolyl)-phosphat in Tetrahydrofuran unter Rühren und Feuchtigkeitsausschluss getropft und das Reaktionsgemisch 1 Stunde bei Raumtemperatur gerührt. Die erhaltene Lösung des 2-Chlorphenyl-1-benzotriazolyl-5'-(4-methoxytrityl)-thymidin-3'-phosphats wird in drei Teile geteilt.

$\alpha$) Hydrolyse zu Triethylammonium-2-chlorphenyl-5'-(4-methoxytrityl)-thymidin-3'-phosphat:

Zu einem Drittel der obigen Lösung von 2-Chlorphenyl-1-benzotriazolyl-5'-(4-methoxytrityl)-thymidin-3'-phosphat werden unter Kühlung 100 ml 0,5 M Triäthylammoniumbicarbonat gegeben. Nach 15 min. wird mit Dichlormethan extrahiert. Die Dichlormethan-Lösung wird mit Wasser gewaschen, eingeengt und tropfenweise mit Petrolether versetzt. Die erhaltene Fällung wird abgenutscht, mit Aether-Petrolether 1:1 ausgewaschen und im Vakuum getrocknet. DC: $R_f$ 0,35 in Dichlormethan-Methanol-Wasser (75:22:3).

$\beta$) Veresterung zu 2-Cyanoäthyl-2-chlorphenyl-5'-(4-methoxytrityl)-thymidin-3'-phosphat und Abspaltung der 4-Methoxytrityl-Schutz-gruppe:

Zu einem Drittel der Lösung von 2-Chlorphenyl-1-benzotriazolyl-5'-(4-methoxytrityl)-thymidin-phosphat werden 1,3 ml 2-Cyanoäthanol und 2 ml Pyridin gegeben. Das Gemisch wird über Nacht bei Raumtemperatur belassen. Die Lösungsmittel werden im Vakuum abdestilliert und der Rückstand in Essigsäureäthylester gelöst und mehrmals mit 0,1 M Phosphatpuffer pH 7 und Wasser ausgeschüttelt. Die organische Phase wird getrocknet, eingeengt und in Hexan eingetropft. Der Niederschlag wird abfiltriert, in 50 ml Dichlormethan-Methanol 7:3 gelöst und bei 0 °C mit einer Lösung von 3,8 g p-Toluolsulfonsäuremonohydrat in 75 ml Dichlormethan-Methanol 7:3 versetzt. Nach 2 Stunden wird die Reaktionalösung mit Dichlormethan verdünnt und mit einer kalten Natriumhydrogencarbonat-Lösung ausgeschüttelt. Die organische Phase wird eingeengt und mit Hexan versetzt. Das ausgefallene 2-Cyanoethyl-2-chlorphenyl-thymidin-3'-phosphat wird an Kieselgel mit Dichlormethan-Methanol 96:4 chromatographiert. DC: $R_f$ von 0,45 in Dichlormethan-Methanol (9:1).

$\tau$) Kondensation zum 5'-(4-Methoxytrityl)-3'-cyanoäthyl)-bis-thymidin-dinukleotid:

2,2 g 2-Cyanoäthyl-2-chlorphenyl-thymidin-3'-phosphat werden zweimal durch Eindampfen mit abs. Pyridin entwässert, in 20ml abs. Tetrahydrofuran gelöst und zum restlichen Drittel der Lösung von 2-Chlorphenyl-1-benzotriazolyl-5'-(4-methoxytrityl)-thymidin-3'-phosphat gegeben. Nach 18 Stunden bei Raumtemperatur werden zur Reaktionslösung unter Eiskühlung 10 ml Wasser und 200 ml Essigsäureäthylester zugegeben. Die organische Phase wird mehrmals mit Natriumhydrogencarbonat und Wasser gewaschen, über Natriumsulfat getrocknet und auf ein kleines Volumen eingeengt. Das im Phosphat-Teil und am 5'- bzw. 3'-Ende geschützte Dinukleotid wird durch Eintropfen in Aether-Hexan 1:1 gefällt. DG: $R_f$ 0,48 in Dichlormethan-Methanol (9:1).

b) Synthese des Trinukleotids:

1,17 g (1 mMol) des oben beschriebenen vollgeschützten Dinukleotids werden in 30 ml Dichlormethan-Methanol 7:3 gelöst und unter Eiskühlung mit einer Lösung von 1,9 g p-Toluolsulfonsäuremonohydrat in 20 ml Dichlormethan-Methanol 7:3 versetzt. Nach 2 Stunden wird eiskalte Natriumhydrogencarbonat-Lösung zugegeben und mit Dichlormethan extrahiert. Die organische Phase wird getrocknet, eingeengt und in Hexan eingetropft. Das ausgefallene rohe Dinukleotid mit freier 5'-Hydroxygruppe wird an Kieselgel mit einem Gradienten von 2-8% Methanol in Dichlormethan chromatographiert. DG: $R_f$ 0,33 in Dichlormethan-Methanol (9:1).

850 mg dieses 5'-Hydroxy-dinukleotids und 1,06 g Triethylammonium-2-chlorphenyl-5'-(4-methoxytrityl)-thymidin-3'-phosphat [vgl. Abschnitt a)α)] werden zweimal mit Pyridin eingedampft, dann in 10 ml abs. Pyridin gelöst und mit 560 mg 1-Mesitylensulfonyl-3-nitro-1,2,4-triazol (MSNT) versetzt. Nach 2 Stunden werden 2 ml eiskaltes Wasser zugegeben und nach einer weiteren Stunde mit Dichlormethan extrahiert. Die organische Phase wird mit gesättigtem Natriumhydrogencarbonat und Wasser gewaschen, getrocknet, eingeengt und mit Aether versetzt. Das ausgefallene Trinukleotid wird durch Chromatographie an Kieselgel gereinigt. $R_f$ 0,45 in Dichlormethan-Methanol (9:1).

Beispiel 4:

Analog Beispiel 3 werden folgende geschützte Trinukleotide der allgemeinen Formel

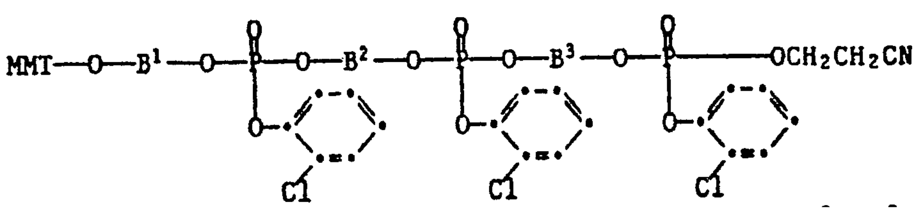

abgekürzt $B^1B^2B^3$, hergestellt. Für die Nukleoside $B^1$, $B^2$, B werden folgende Abkürzungen verwendet:

A = N-Benzoyl-deoxyadenosin
C = N-Benzoyl-deoxycytidin
G = N-Isobutyryl-deoxyguanosin
T = Thymidin

| Verbindung | $R_f$ [a)] | Verbindung | $R_f$ [a)] |
|---|---|---|---|
| TTT | 0,45 | ATG | 0,48 |
| TTC | 0,55 | ACT | 0,53 |
| TCT | 0,46 | ACC | 0,48 |
| TAC | 0,56 | ATT | 0,55 |
| TGC | 0,44 | ACA | 0,53 |
| TAG | 0,60 | AAC | 0,46 |
| TGT | 0,42 | AAA | 0,51 |
| TGA | 0,44 | AGT | 0,45 |
| CTT | 0,53 | AGG | 0,38 |
| CTG | 0,46 | GTT | 0,45 |
| CCT | 0,45 | GTC | 0,44 |
| CCC | 0,59 | GTG | 0,35 |
| CCG | 0,47 | GCA | 0,49 |
| CCA | 0,51 | GCG | 0,48 |
| CAT | 0,55 | GAT | 0,44 |
| CAC | 0,51 | GAC | 0,48 |
| CGC | 0,46 | GAG | 0,48 |
| CGA | 0,38 | GAA | 0,50 |
| CAG | 0,44 | GGC | 0,42 |
| CGG | 0,38 | GGT | 0,46 |
| CGT | 0,49 | GGG | 0,35 |
| | | GGA | 0,44 |

a) Dünnschichtchromatogramm auf Kieselgel in Dichlormethan-Methanol 9:1.

Beispiel 5: Synthese des DNA-Fragments einer Länge von 67 Basen von Base Nr. 96 bis Base Nr. 162 des DNA-Stranges (96/67)

a) Abspaltung der 2-Cyanäthylschutzgruppe der Trinukleotide:

10 $\mu$Mol der Trinukleotide aus Beispiel 3 oder 4 werden unter Feuchtigkeitsausschluss in 60 $\mu$l Pyridin-Acetonitril-Triäthylamin 1:1:1 gelöst. Nach 1 Stunde bei Raumtemperatur werden 0,7 ml peroxidfreier Aether zugetropft und der Niederschlag abzentrifugiert. Das rohe Triethylammoniumsalz wird in 50 $\mu$l Pyridin gelöst und nochmals mit 0.5 ml Aether ausgefällt, abzentrifugiert und 15 Stunden am Hochvakuum getrocknet.

b) Kupplung der teilgeschützten Trinukleotide mit der an PolystyrolHarz gebundenen Oligonukleotidkette:

Alle Operationen werden unter Feuchtigkeitsausschluss in einem Reaktionsgefäss mit 220 $\mu$l Inhalt und mikroprozessorgesteuerter Lösungsmittel- und Reagens-Zugabe durchgeführt. 13 mg (0,74 $\mu$Mol) des

Thymidin-Polystyrol-Harzes (Beispiel 1) werden im Reaktionsgefäss vorgelegt und folgenden Operationen unterworfen:

1. Methylenchlorid, 2 ml/min., 4 min.
2. Methylenchlorid-Isopropanol (85:15), 2 ml/min., 2 min.
3. Zinkbromid 1 M und 1,2-4-Triazol 0,02 M in Methylenchlorid-Isopropanol (85:15), 2 ml/min., 2-3,5 min.
4. Methylenchlorid-Isopropanol (85:15), 2 ml/min., 4 min.
5. Triäthylammoniumacetat 0,5 M in DMF, 2 ml/min., 5 min.
6. Molekularsieb-getrocknetes Pyridin, 2 ml/min., 3 min.
7. Tetrahydrofuran (Peroxid-frei, Molekularsieb-getrocknet), 2 ml/min., 3 min.
8. Stickstoffstrom, 10 min.
9. Injektion 10 μMol Trinukleotid GTC (Trimethylammoniumsalz aus Abschnitt a)) und 8,9 mg (30 μMol) 1-Mesitylensulfonyl-3-nitro-1,2,4-triazol (MSNT) gelöst in 150 μl Pyridin
10. 45° C, 20 min.
11. Pyridin, 2 ml/min., 4 min.
12. Acetanhydrid 5 % und 4-Dimethylaminopyridin 2,5 % in Pyridin, 2 ml/min., 4 min.
13. Pyridin, 2 ml/min., 4 min.
14. Pyridin-Isopropanol (1:1), 2 ml/min., 3 min.

Alle 14 Operationen werden 21 mal wiederholt, wobei bei der 9. Operation anstelle von GTC jeweils folgende Trinukleotide in Form ihrer Triäthylammoniumsalze (Abschnitt a)) in der angegebenen Reihenfolge eingesetzt werden: GCA, ACC, GAA, CCC, TAC, AGG, TGA, CGG, TAC, CGT, GTG, CCA, AAA, AAA, TGA, CGG, TGA, TTC, GGG, CCT, CAT. Die mittlere Kupplungsausbeute beträgt 97 %. Das Endprodukt hat folgende Struktur:

**MMT-CATCCTGGGTTCTGACGGTGAAAAAAACCAGTGCGTTACCGGTGAAGGTACCCCGAAACCGCAG**

**TCT-Polystyrol**

c) Abspaltung des DNA-Fragments vom Träger und Abspaltung der Schutzgruppen:

40,0 mg (ca. 0,60 μMol) DNA-Syntheseharz 96/67 werden mit 66 mg (0,40 mMol) o-Nitrobenzaldoxim und 50 μl (0,40 mMol) 1,1,3,3-Tetramethylguanidin in 400 μl 95%igem Pyridin während 3 Stunden bei 50° C und während 12 Stunden bei Raumtemperatur gehalten. Nach dem Abblasen des Pyridins mit Stickstoff wird der Rückstand mit 1.6 ml wässerigem Ammoniak (33 %) versetzt und im geschlossenen Gefäss während 24 Stunden bei 50° C gehalten.

Die abgetrennte flüssige Phase wird im Vakuum vom Ammoniak befreit und 3 mal mit je 3 ml peroxidfreiem Diäthyläther gewaschen. Nach dem Abtrennen der niedermolekularen Bestandteile an einer Biogel P6 Säule (100-200 mesh, 3x66 cm, 0,01 Molar Trimethylammoniumhydrogencarbonat pH 7,5, 1,5 ml/min.) werden 285 ODs (260 nm) DNA isoliert.

Insgesamt 60 ODs werden an einer HPLC-Säule aufgetrennt (PRP-1/Hamilton, 250 x 4,6 mm). Gradient (Lösung A: 0,05 M Triäthylammoniumacetat pH 7,0; Lösung B: Lösung A:Acetonitril 1:1): 30 % B in A → 60 % B in A in 20 min. bei 50° C und 2 ml/min. Der lipophile Hauptpeak (Retentionszeit ca. 14 min.) wird gesammelt, an einer DE52-Cellulose (Whatman) Säule aufkonzentriert, eluiert und mit Aethanol gefällt. Zur Abspaltung der 4-Methoxytrityl-Schutzgruppe wird der Niederschlag in 50 μl Essigsäure/$H_2O$ (4:1) gelöst und 45 min. bei Raumtemperatur gehalten. Das Reaktionsprodukt wird lyophilisiert, mit Aethanol gefällt und zur Reinigung auf einem 8 % Polyacrylamidgel (7 M Harnstoff) elektrophoretisch aufgetrennt. Die der erwarteten DNA-Grösse entsprechende Bande wird ausgeschnitten und das Produkt elektroeluiert, an DE52-Cellulose aufkonzentriert und die DNA 96/67 der Struktur

**5'-CATCCTGGGTTCTGACGGTGAAAAAAACCAGTGCGTTACCGGTGAAGGTACCCCGAAACCG**

**CAGTCT-3'**

mit Aethanol gefällt.

Beispiel 6:

Analog Beispiel 5 werden folgende DNA-Fragmente (5'-3') hergestellt:

**1/58**
**CTGGAATTCATGGTTGTTTACACCGACTGCACCGAATCTGGTCAGAACCTGTGCCTGT**

**46/64 komplementär**
**CAGAACCCAGGATGCATTTGTTACCCTGACCGCAAACGTTAGAACCTTCGCACAGGCACAGGTT**

**154/64 komplementär**
**CAGGATCCTACTGCAGGTATTCTTCCGGGATTTCTTCGAAGTCACCGTCGTTGTGAGACTGCGG**

Beispiel 7: Phosphorylierung der Fragmente 1/58, 46/64 komplementär, 96/67 und 154/64 komplementär

Die Phosphorylierung und die radioaktive Markierung an den 5'-Enden erfolgt mit [$\gamma$-$^{32}$P]ATP und $T_4$ Polynukleotid-Kinase (Boehringer) wie beschrieben (18).

Beispiel 8: Polymerisation zu Duplex II (Teilstück $F_2$ des Desulfatohirudin-Gens)

Je 50 pMol Fragment 96/67 kinasiert und Fragment 154/64 kinasiert werden in 24 $\mu$l Wasser gelöst, die Lösung während 3 min. auf 90° C erwärmt und innerhalb von 5 min. auf 12° C abgekühlt. Nach Zugabe von 4 $\mu$l Endo-R Puffer (0,1 Molar Tris•HCl pH 7,5, 66 mM $MgCl_2$, 66 mM $\beta$-Mercaptoäthanol, 0,6 M NaCl), 10 $\mu$l Deoxynucleosidtriphosphat-Gemisch (dATP, dCTP, dGTP, TTP, je $2 \cdot 10^{-3}$ Molar, mit $NH_3$ auf pH 7.0 eingestellt) und 2 $\mu$l (10 Einheiten) DNA-Polymerase I, Klenow-Fragment (Boehringer) wird während 30 min. bei 12° C inkubiert. Die Reaktion wird durch 3 minütiges Erhitzen auf 90° C gestoppt und das Gemisch bis zur Weiterverarbeitung bei -80° C aufbewahrt.

In analoger Weise werden die Fragmente 1/58 kinasiert und 46/64 kinasiert zum Duplex I (Teilstück $F_1$ des Desulfatohirudin-Gens) umgesetzt.

Die Duplexe I und II haben die folgenden Strukturen:

**Duplex I**

**CTGGAATTCATGGTTGTTTACACCGACTGCACCGAATCTGGTCAGAACCTGTGCCTGTGCGAAGGTTC**
**GACCTTAAGTACCAACAAATGTGGCTGACGTGGCTTAGACCAGTCTTGGACACGGACACGCTTCCAAG**

**TAACGTTTGCGGTCAGGGTAACAAATGCATCCTGGGTTCTG**
**ATTGCAAACGCCAGTCCCATTGTTTACGTAGGACCCAAGAC**

**Duplex II**

**CATCCTGGGTTCTGACGGTGAAAAAAACCAGTGCGTTACCGGTGAAGGTACCCCGAAACCGCAGTCTC**
**GTAGGACCCAAGACTGCCACTTTTTTTGGTCACGCAATGGCCACTTCCATGGGGCTTTGGCGTCAGAG**

**ACAACGACGGTGACTTCGAAGAAATCCCGGAAGAATACCTGCAGTAGGATCCTG**
**TGTTGCTGCCACTGAAGCTTCTTTAGGGCCTTCTTATGGACGTCATCCTAGGAC**

Die Herstellung der Teilstücke $F_1$ und $F_2$ des Desulfatohirudin-Gens ist im folgenden Schema I dargestellt:

Schema 5: Herstellung der Teilstücke $F_1$ und $F_2$ des Desulfatohirudin-Gens und der $F_1$-$F_2$-DNA

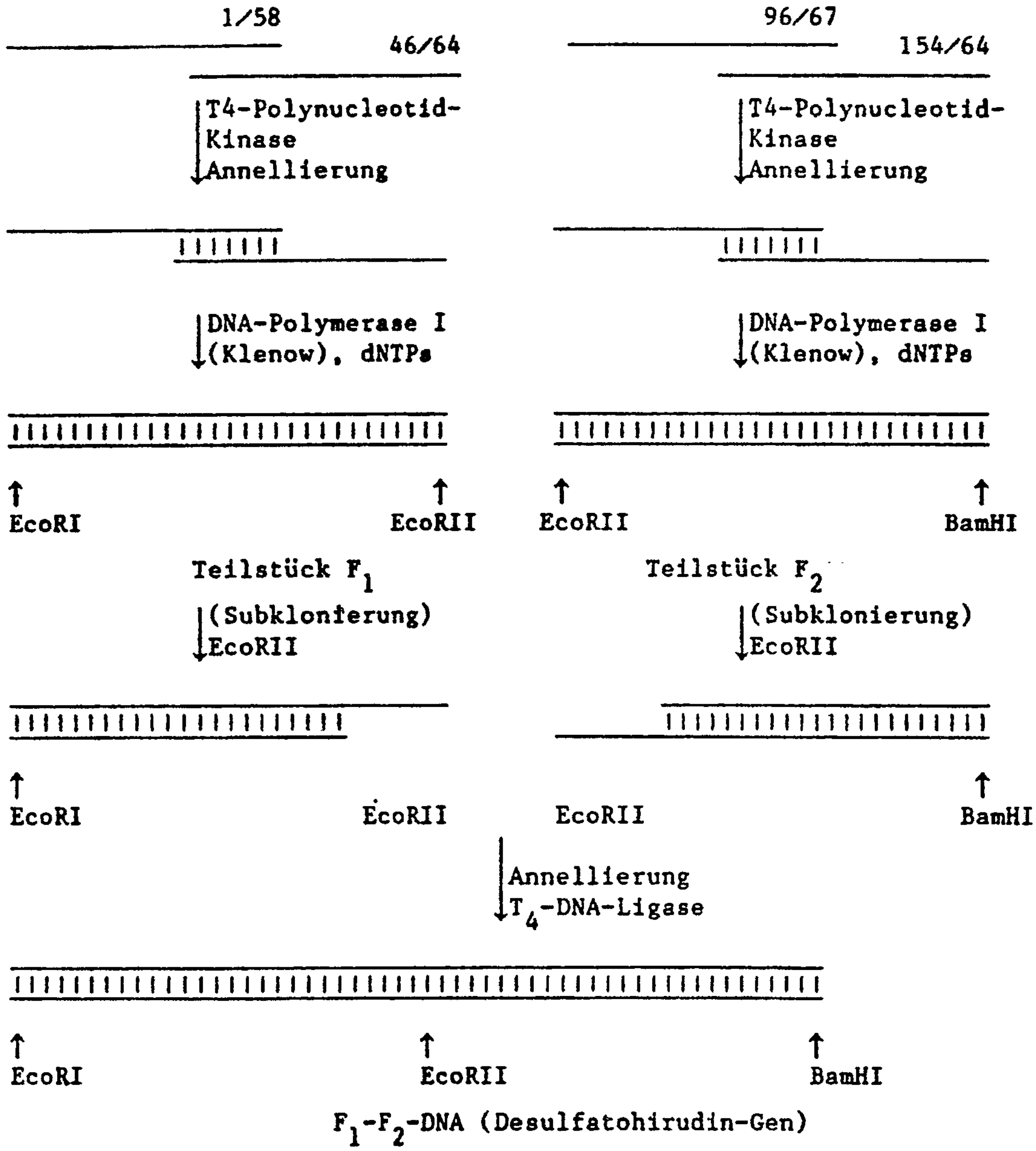


<u>Beispiel 9: Polymerisation und Ligation der Fragmente 1/58, 46/64 kinasiert, 96/67 kinasiert und 154/64; Herstellung des Desulfstohirudin-Gens</u>

Je 50 pMol Fragment 1/58, 46/64 kinasiert, 96/67 kinasiert und 154/64 werden in 48 µl Wasser gelöst, die Lösung während 3 min. auf 90°C erwärmt und innerhalb 5 min. auf 12°C abgekühlt. Nach Zugabe von 8 µl Endo-R Puffer (vgl. Beispiel 8), 20 µl Deoxynucleosidtriphosphat-Gemisch (daTP, dCTP, dGTF, TTP, je 0,002 Molar, mit $NH_3$ auf pH 7.0 eingestellt) und 2 µl (10 Einheiten) DNA-Polymerase I, Klenow-Fragment (Boehringer) wird während 30 min. bei 12°C inkubiert. Die Reaktion wird durch 3-minütiges Erhitzen auf 90°C gestoppt und die DNA nach Phenol/Chloroform-Extraktion durch Aethanolfällung isoliert. Das entstandene DNA-Gemisch wird in 100 µl Ligase-Puffer (66 mM Tris•HCl pH 7.5. 6,6 mM $MgCl_2$, 10 mM Dithiothreitol, 5 mM ATP) gelöst, mit 50 Einheiten (2 µl) T4 DNA-Ligase (Biolabs) versetzt und während 20 Stunden bei 20°C inkubiert. Die Reaktion wird durch 5-minütiges Erhitzen auf 70°C gestoppt und die DNA nach Phenol/Chloroform-Extraktion durch Aethanolfällung isoliert. Nach Auftrennung des Gemisches an einem 8 % Polyacrylamidgel (denaturierend) durch Elektrophorese wird das Ligationsprodukt mit 217 Basenpaaren elektroeluiert, an einer DE52-Cellulose-Säule aufkonzentriert und nach Elution durch Ethanolfällung isoliert.

Das Desulfatohirudin-Gen hat die folgende Struktur:

CTGGAATTCATGGTTGTTTACACCGACTGCACCGAATCTGGTCAGAACCTGTGCCTGTGCGAAGGT
GACCTTAAGTACCAACAAATGTGGCTGACGTGGCTTAGACCAGTCTTGGACACGGACACGCTTCCA

TCTAACGTTTGCGGTCAGGGTAACAAATGCATCCTGGGTTCTGACGGTGAAAAAAACCAGTGCGTT
AGATTGCAAACGCCAGTCCCATTGTTTACGTAGGACCCAAGACTGCCACTTTTTTTGGTCACGCAA

ACCGGTGAAGGTACCCCGAAACCGCAGTCTCACAACGACGGTGACTTCGAAGAAATCCCGGAAGAA
TGGCCACTTCCATGGGGCTTTGGCGTCAGAGTGTTGCTGCCACTGAAGCTTCTTTAGGGCCTTCTT

TACCTGCAGTAGGATCCTG
ATGGACGTCATCCTAGGAC

Die Herstellung des Desulfatohirudin-Gens aus den vier Fragmenten ist im folgenden Schema II dargestellt:

Schema 6: Herstellung des Hirudin-Gens aus den vier synthetischen Fragmenten

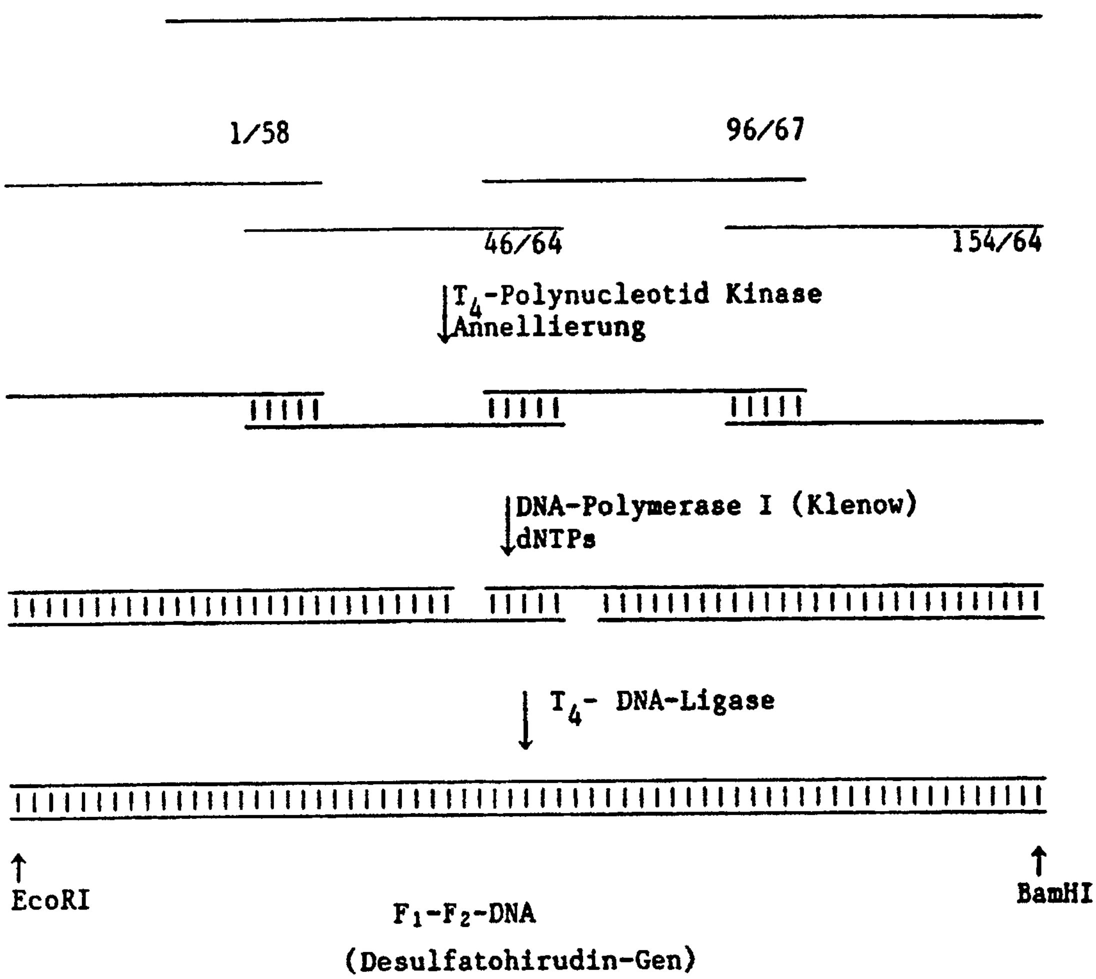


Beispiel 10: Herstellung des Plasmids pML 300, enthaltend die $F_1$-DNA des Desulfatohirudin-Gens (Fig. 1)

a) Herstellung des linearisierten Vektors pBR322/EcoRI/PvuII.

5 $\mu$g pBR322 Plasmid-DNA werden mit 5 Einheiten PvuII Restriktionsendonuclease (Biolabs) in 200 ml einer Lösung aus 100 $\mu$g/ml Gelatine für 1 Stunde bei 37° C verdaut. Anschliessend wird diese Lösung auf

100 mM Tris•HCl (pH 7.5), 50 mM NaCl eingestellt und die DNA mit 30 Einheiten EcoRI-Restriktionsendonuclease (Biolabs) für 2 Stunden bei 37° C verdaut. Dann wird die Lösung auf 50 mM Tris-HCl pH 8 eingestellt und bei einer DNA-Konzentration von 10 µg/µl mit 2 Einheiten intestinaler alkalischer Kälber-Phosphatase (Boehringer) während 30 min. bei 37° C inkubiert. Das Enzym wird durch Erhitzen der Lösung während 60 min. bei 65° C inaktiviert. Dann wird die Lösung auf TNE eingestellt, mit 1 Volumen Phenol und Chloroform extrahiert und die verdaute DNA mit 2 Volumen Alkohol bei -20° C über Nacht ausgefällt.

Der aus der pBR322 DNA herausgeschnittene Vektor (pBR322/EcoRI/-PvuII, 2297 Basenpaare) wird durch Gelelektrophorese an 1%iger niedrig schmelzender Agarose(Biorad) in Tris-Acetat-EDTA Puffer pH 8 vom kleinen DNA-Fragment (2067 Basenpaare) abgetrennt. Nach Anfärben der DNA im Agarosegel mit EtBr wird die Stelle des Gels, welche die DNA-Bande des pBR322/EcoRI/PvuII Vektors(= 2297 Basenpaare) enthält, aus dem Gel ausgeschnitten und bei 65° C 10 min. verflüssigt. Zu dieser DNA-Lösung werden 20 Volumina TNE hinzugefügt, die DNA nach Mueller et al. (19) durch DE-52 Chromatographie gereinigt, mit Phenol/Chloroform extrahiert und die DNA bei -20° C über Nacht mit Alkohol ausgefüllt. Der DNA-Niederschlag wird in 50 µl 0,01 M Tris•HCl (pH 8), 0,1 mM EDTA gelöst und bis zur Verwendung bei -20° C aufbewahrt. 1,4 µg (= 3,2 pMol Enden) DNA werden erhalten.

b) Herstellung der $F_1$-DNA/EcoRI

24 ng (= 1,3 pMol Enden) der chemisch synthetisierten $F_1$-DNA (siehe Beispiel 8) werden mit 5 Einheiten EcoRI Restriktionsendonuclease (Biolabs) in 50 µl 100 mM Tris•HGl (pH 7.5), 50 mM NaCl und 100 µg/ml Gelatine für 30 min. bei 37° C verdaut. Danach werden der Lösung 0,06 µg (= 0,14 pMol Enden) des linearisierten Vektors pBR322/EcoRI/PvuII (Beispiel 10a) zugesetzt. Anschliessend wird das Enzym durch Erhitzen auf 65° C nach 10 min inaktiviert, die Lösung auf TNE eingestellt und mit Phenol/Chloroform extrahiert. Die DNA wird mit Alkohol präzipitiert. Die ausgefällte DNA wird unter Alkohol bei -20° C bis zur Weiterverarbeitung gelagert.

c) Ligierung der pBR322/EcoRI/PvuII Vektor-DNA mit der $F_1$-DNA/EcoRI und Konstruktion des Plasmids pML300.

Der in Beispiel 10b) erhaltene DNA-Niederschlag, welcher die beiden genannten DNA-Bruchstücke enthält, wird in 20 µl einer Lösung von 50 mM Tris•HCl (pH 7.8), 10 mM $MgCl_2$, 10 mM DTT, 0.5 mM ATP, 100 µg/ml Gelatine gelöst und mit 25 Einheiten/µl $T_4$ DNA-Ligase (Biolabs) bei 15° C für 3 Stunden behandelt. Auf diese Weise entsteht in der Lösung das rekombinierte Plasmid pML300, welches die $F_1$-DNA enthält.

d) Transformation von E. coli HB101 mit dem Plasmid pML300

Die für die Transformation notwendigen mit Calcium vorbehandelten E. coli HB101 Zellen werden, wie von Handel et. al. (14) beschrieben, hergestellt.

Die unter c) erhaltene Lösung, welche das rekombinierte Plasmid pML300 enthält, wird für 10 min. auf 65° C erhitzt, um die $T_4$-DNA Ligase zu inaktivieren und anschliessend auf 37° C abgekühlt. 10 µl dieses Reaktionsgemisches werden zu 150 µl Calcium-behandelten E. coli HB101-Zellen in 10 mM $MgCl_2$ und 10 mM Tris•HCl (pH 7.5) in einem Gesamtvolumen von 200 µl gegeben.

Danach wird diese Mischung für 30 min. in Eis gekühlt, 2 min. auf 42° C erwärmt und dann 50 min. in 1 ml L-Medium (vgl. Beispiel 18) bei 37° C stehen lassen. Darauf wird die Mischung in Aliquoten à 0.2 ml auf 5 Agarplatten (McConkey-Agar, Difco), welche 60 µg/ml Ampicillin (Serva) enthalten, ausgestrichen. Die Agarplatten werden hernach bei 37° C für 16-18 Stunden gehalten. 484 Ampicillin-resistente Kolonien des transformierten E. coli HB 101 werden erhalten.

e) Screening der Kolonien, die $F_1$-DNA enthalten

470 transformierte Kolonien (Beispiel 10d) werden auf Nitrozellulosefilter B85 (Schleicher und Schüll) abgedrückt. Nach Grunstein und Hogness (20) werden die Kolonien lysiert und ihre denaturierte DNA auf dem Filter fixiert. Anschliessend erfolgt eine Vorhybridisierung der Filter in 20 ml (pro Filter) 4xSET [= Lösung von 30 mM Tris•HCl (pH 8), 150 mM NaCl, 1 mM EDTA], 0.1 % (g/v) Ficoll 400 (Pharmacia), 0.5 % SDS, 50 µg/ml denaturierte Kalbsthymus-DNA für 4 Stunden bei 64° C. Danach werden die Nitrozellulosefilter in 20 ml (pro Filter) 5xSET (g/v) Ficoll 400, 0.2 % SDS und 50 µg/ml denaturierter Kalbsthymus-DNA für 16 Stunden bei 64° C mit der $^{32}$P-radioaktiv markierten Probe (ca. $10^3$-$10^4$ Cerencov cpm pro Filter)

behandelt. Als Probe wird das Oligonucleotid 46/64 komplementär (vgl. Beispiel 6) verwendet.

Anschliessend werden die Filter in 2xSET, 0.2 % SDS bei Raumtemperatur zweimal gewaschen, danach zweimal in 2xSet, 0.5% SDS bei 60 °C (zuerst 30 min., dann 60 min. lang). Dann werden die Filter zwischen 3 MM Papier (Whatman) getrocknet und bei -80 °C auf einen Röntgen-Film (Fuji) mit einer Verstärkerfolie (Intensifying screen, Ilford) für 1-2 Tage gelegt.

Das erhaltene Autoradiogramm zeigt 71 positive Kolonien (Klone), die zur Weiterarbeit verwendet werden können, eine davon erhielt die Bezeichnung pML300.

Beispiel 11: Herstellung des Plasmids pML305, welches die $F_2$-DNA des Desulfatohirudin-Gens enthält (Fig. 2).

a) Herstellung des linearisierten Vektors pBR322/BamHI/NruI

5 µg pBR322 Plasmid-DNA werden mit 30 Einheiten BamHI Restriktionsendonuclease 30 min. bei 37 °C in einer Lösung von 100 mM NaCl, 6 mM Tris•HCl (pH 7.9), 6 mM $MgCl_2$ und 100 µg/ml Gelatine verdaut. Anschliessend werden der Lösung 15 Einheiten PvuII Restriktionsendonuclease zugefügt und 2 Stunden bei 37 °C verdaut.

Das Reaktionsgemisch wird für 10 min. auf 70 °C erwärmt, um die Enzyme zu inaktivieren. Danach werden die beiden DNA-Fragmente durch Gelelektrophorese an 1%iger niedrigschmelzender Agarose in Tris-Acetat EDTA Puffer pH 8 voneinander getrennt. (siehe Beispiel 10a).

Die DNA-Bande des pBR322 BamHI/PvuII Vektors (= 2672 Basenpaare) wird ausgeschnitten, verflüssigt und nach Mueller et al. (19) durch DE-52 Chromatographie gereinigt. 0,75 µg (= 1,5 pMol Enden) DNA werden erhalten.

b) Herstellung der $F_2$-DNA/BamHI

25 µg (= 1,3 pMol Enden) der chemisch synthetisierten $F_2$-DNA (Beispiel 8) werden mit 16 Einheiten BamHI Restriktionsendonuclease (Biolabs) in 20 µl 150 mM NaCl, 6 mM Tris•HCl (pH 7.9), 6 mM $MgCl_2$ und 100 µg/ml Gelatine für 30 min. bei 37 °C verdaut. Anschliessend werden der Lösung 60 ng (= 96 nMol Enden) des linearisierten Vektors pBR322/BamHI/PvuII (Beispiel 11a) zugesetzt, die ganze Lösung auf TNE eingestellt und mit Phenol/Chloroform extrahiert und die DNA mit 2 Volumen Alkohol ausgefällt. Die ausgefällte DNA wird bis zur Weiterverarbeitung unter Alkohol bei -20 °C gelagert.

c) Ligierung der pBR322/BamHI/PvuII Vektor-DNA mit der $F_2$-DNA/BamHI und Konstruktion des Plasmids pML305

Der unter Beispiel 11b) erhaltene DNA-Niederschlag, der die beiden genannten DNA-Bruchstücke enthält, wird in 20 µl einer Lösung von 50 mM Tris•HCl (pH 7.8), 10 mM $MgCl_2$, 10 mM DTT, 0.5 mM ATP, 100 µg/ml Gelatine gelöst und mit 15 Einheiten/µl $T_4$ DNA-Ligase (Biolabs) bei 15 °C für 3 Stunden behandelt. Auf diese Weise entsteht in der Lösung das rekombinierte Plasmid pML305, welches die $F_2$-DNA enthält.

d) Transformation von E. coli HB101 mit dem Plasmid pML305

Die Transformation der mit Calcium behandelten E. coli HB101 Zellen erfolgt wie in Beispiel 10d) beschrieben. Es werden 10 µl des in Beispiel 12c) erhaltenen Reaktionsgemisches verwendet. 313 Ampicillin-resistente Kolonien werden erhalten.

e) Screening der Kolonien, die $F_2$-DNA enthalten

65 transformierte Kolonien (Beispiel 11d) werden auf $F_2$-DNA geprüft, wie in Beispiel 10e) beschrieben. Als radioaktive Probe wird das Oligonucleotid 154/64 komplementär (vgl. Beispiel 6) verwendet. Im Autoradiogramm werden 2 positive Kolonien erhalten, eine davon erhält die Bezeichnung pML305.

Beispiel 12: Charakterisierung der Klone pML300 und pML305

Die DNAs der rekombinierten Plasmide pML300 und pML305 werden nach Ish-Horowitz isoliert (21). Die Nucleotidsequenzen des $F_1$-DNA- und $F_2$-DNA-Inserts werden nach Maxam und Gilbert bestimmt (11). Zu

diesem Zweck werden je 10 µg Plasmid-DNA von pML300 mit EcoRI-Restriktionsendonuclease und 10 µg Plasmid-DNA von pML305 mit BamHI-Restriktionsendonuclease gespalten und die linearisierten DNAs durch Gelelution aus Agarosegel isoliert [vgl. Beispiel 10a)]. Anschliessend werden die isolierten DNAs mit alkalischer Phosphatase verdaut und über DE-52 chromatographiert (vgl. Beispiel 11a). Danach werden die DNAs am 5'-Ende mit [$\gamma$-$^{32}$P]ATP (spezifische Aktivität > 5000 Ci/mmol, Amersham) und $T_4$-Polynucleotid-Kinase (P-L-Biochemicals) radioaktiv markiert.

Die radioaktiv markierten DNAs werden dann mit einer zweiten Restriktionsendonuclease (EcoRII) gespalten. Die entstandenen DNA-Fragmente werden durch Gelelution aus Agarose isoliert. Im Falle von pML300 wird dann vom EcoRII-EcoRI *Fragment (ca. 109 Basenpaare) die Nucleotidsequenz der $F_1$-DNA, im Fall vom pML300 die der $F_2$-DNA im EcoRII-BamHI*-Fragment (ca. 122 Basenpaare) bestimmt.

Die Nucleocidsequenzen, welche für die $F_1$-DNA, und $F_2$-DNA bestimmt werden, sind identisch mit den im Beispiel 8 dargestellten.

Beispiel 13: Herstellung des Expressionsplasmids pML310

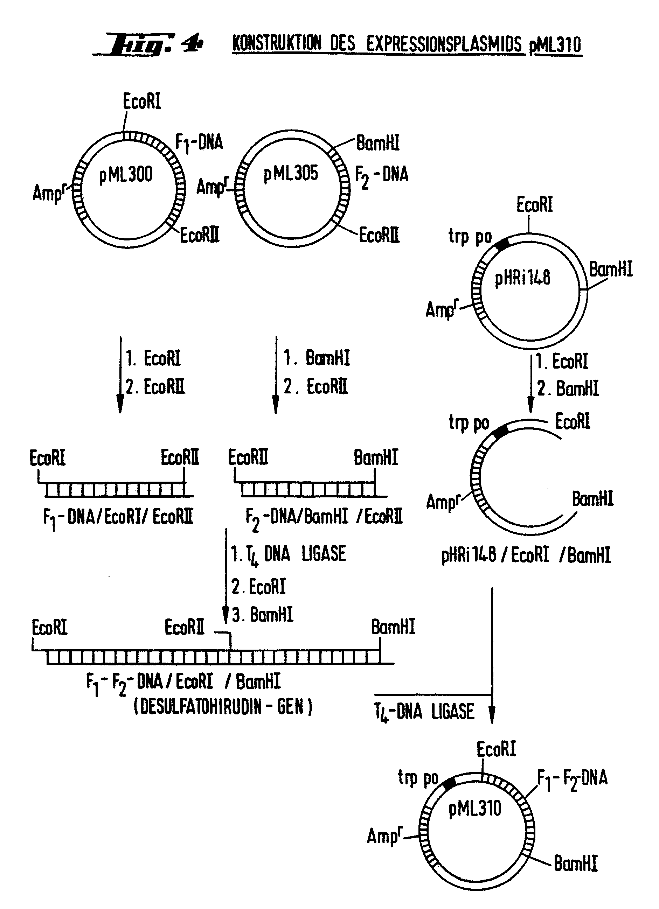

a) Konstruktion des linearisierten Vektors pHRi148/EcoRI/BamHI, welcher den trp-Promotor-Operator enthält (Fig. 3 und Fig. 4)

A. Konstruktion des Plasmids p159

10 µg Plasmid $pBRH_{trp}$ (22) werden mit 50 Einheiten EcoRI (Biolabs) gespalten während 60 min. bei 37° C. und das Verdauungsgemisch wird nach Phenolextraktion durch einen Saccharose-Dichtegradienten (5-23 %) in 50 mM Tris•HCl (pH 8,0), 1 mM EDTA in einem TST41 (Kontron AG) Rotor fraktioniert. Die Zentrifugation dauert 14 Stunden bei 40 000 upm und 15° C. 0,3 ml Fraktionen werden mit einem ISCO-Gradientkollektor bei 1 ml/min. gesammelt. Die Fraktionen, welche das kleinere Fragment enthalten, werden vereinigt, die Lösung wird auf TNE eingestellt und mit 2 Volumen Aethanol bei -20° C gefällt. Die DNA wird nach Zentrifugation in einer Eppendorf-Zentrifuge in 100 µl 10 mM Tris•HCl pH 7,5, 0,5 mM EDTA gelöst. 5 µg dieses DNA-Fragmentes werden mit 5 Einheiten BglII (Biolabs) 60 min. bei 37° C gespalten. Das Reaktionsgemisch wird mit Phenol und Chloroform extrahiert und die DNA wird mit 2 Volumen Aethanol bei -80° C während 10 min. inkubiert und die DNA durch Zentrifugation gesammelt und erneut in 50 µl 50 mM Tris•HCl (pH 8,0) gelöst. 2 µl von dieser Lösung werden entnommen (0,2 µg DNA) und bei einer DNA-Konzentration von 10 ng/µl in 50 mM Tris•HCl (pH 8,0) mit 1 Einheit intestinaler alkalischer Kälber-Phosphatase (Boehringer) während 30 min. bei 37° C inkubiert. Das Enzym wird durch Erhitzen der Lösung während 60 min. bei 65° C inaktiviert. 0,04 µg DNA wird entnommen und 5'-terminal mit 10 µCi [$\gamma$-$^{32}$P]-ATP (>5000 Ci/mmol, Amersham) und 5 Einheiten $T_4$ Polynucleotid-Kinase (P-L Biochemicals) in 20 µl Reaktionsvolumen in 50 mM Tris•HCl (pH 9,5), 10 mM $MgCl_2$, und 5 mM DTT, während 30 min. bei 37° C inkubiert. Die radioaktive Probe wird mit der nicht-markierten Probe (siehe oben) gemischt und die DNA-Fragmente werden durch einen 5-23 % Saccharose-Dichtegradienten in 50 mM Tris•HCl (pH 8,0). 1 mM EDTA in einem TST60 Rotor fraktioniert. Die Zentrifugation erfolgt während 5 Stunden bei 60 000 upm und 15° C. Es werden 0,2 ml Fraktionen gesammelt. Die Radioaktivität jeder Fraktion wird durch Messung der Cerencov-Strahlung bestimmt und die Fragmente damit identifiziert. Die gewünschten Fraktionen, welche das kleine DNA-Fragment enthalten, werden vereinigt, die DNA mit 2 Volumina Aethanol gefällt und nach Zentrifugation wieder in 20 µl 10 mM Tris•HCl pH 7,5, 0.5 mk EDTA gelöst.

Das $^{32}$P-markierte EcoRI-BglII DNA-Fragment wird mit 0,2 Einheiten TaqI (Biolabs) in 50 µl Volumen während 10 min. bei 37° C partiell gespalten. Das Reaktionsgemisch wird auf 0,2 % SDS, 10 % Glycerin, 10 mM EDTA, 0.05 % Bromphenol-Blau eingestellt und die DNA-Fragmente auf einem 6 % Polyacrylamidgel in Tris-Borat-EDTA (23) aufgetrennt. Auf dem Autoradiogramm wird die das gewünschte EcoRI-TagI enthaltende Bande (das grösste Teilfragment) identifiziert. Dieses Fragment (L, siehe Fig. 3) wird aus dem Gel extrahiert und gereinigt (19) und in 10 µl 10 mM Tris•HCl pH 7,5, 1 mM EDTA gelöst.

Als Akzeptor-Plasmid wird pBR322, welches mit ClaI und EcoRI gespalten ist, verwendet: 2 µg pBR322 werden mit 4 Einheiten ClaI (Biolabs) in 20 µl Reaktionsvolumen während 60 min. bei 37° C verdaut. Das Protein wird mit Phenol extrahiert und die DNA anschliessend mit 2 Volumina Aethanol bei -80° C während 10 min. gefällt. Die DNA wird durch Zentrifugation gesammelt und dann mit 10 Einheiten EcoRI (Biolabs) während 30 min. bei 37° C in 20 µl Reaktionsvolumen verdaut. Anschliessend wird die Lösung mit 2 Volumina 0,1 M Tris•HCl (pH 8,7) versetzt und mit 1 Einheit alkalischer Kälber-Phosphatase (Boehringer) während 30 min. bei 37° C inkubiert. Die Phosphatase wird anschliessend durch Inkubation bei 65° C

(* gibt das DNA-Ende an, das radioaktiv markiert ist).

während 60 min. inaktiviert.

100 ng des Akzeptor-Plasmids werden mit 5 µl Fragment L-DNA in 15 µl Reaktionsvolumen in 10 mM $MgCl_2$, 20 mM Tris•HCl (pH 7,8), 10 mM DTT, 0,5 mM ATP mit 30 Einheiten pro µl Reaktionsvolumen $T_4$ DNA-Ligase (Biolabs) während 2 Stunden inkubiert.

5 µl dieser Lösung werden zu einem Gemisch gegeben, das 150 ml mit Calciumchlorid behandelte E. coli HB101-Zellen (14) in 10 mM $MgCl_2$, 10 mM $CaCl_2$ und 10 mM Tris•HCl (pH 7,5) in einem Gesamtvolumen von 200 µl enthält. Das Gemisch wird 20 min. in Eis gekühlt, 1 min. auf 42° C erhitzt und 10 min. bei 20° C inkubiert. 1 ml Trypton-Medium [Trypton-Medium enthält 10 g Bacto-Trypton (Difco); 1 g Hefeextrakt (Difco); 1 g Glucose; 8 g NaCl und 294 mg $CaCl_2 \cdot 2H_2O$ in 1 l destilliertem Wasser] wird zugesetzt und das Gemisch 30 min. bei 37° C unter Schütteln bei 300 U/min. inkubiert. Die Mischung wird auf zwei Agarplatten (McConkey Agar, Difco; 0,6 ml/Platte), supplementiert mit 50 µg/ml Ampicillin (Sigma), plattiert. Die Platten werden 12 bis 17 Stunden bei 37° C inkubiert.

Die Plasmid-DNA von 10 verschiedenen Kolonien wird wie folgt isoliert:

Die Kolonien werden zur Inokulierung von 10 ml Trypton-Medium, supplementiert mit 50 µg/ml Ampicillin, wie oben, in einem 25 ml Erlenmeyerkolben verwendet. Die Kulturen werden 15 bis 18 Stunden bei 37° C und 300 U/min. geschüttelt. Die Zellen werden durch Zentrifugieren (Sorval, HS-4 Rotor, 10 min. bei 4000 U/min., 4° C) geerntet. Man erhält etwa 0,1 g Zellen, und diese werden in 1 ml 50 mM Tris•HCl (pH 8,0) resuspendiert. 0,25 ml Lysozymlösung [10 mg/ml in 50 mM Tris•HCl (pH 8,0); Lysozym wird von Sigma vertrieben] werden zugesetzt und nach 10-minütiger Inkubation bei 0° C werden 0,15 ml 0,5 m EDTA (pH 7,5) zugegeben. Nach weiteren 10 min. bei 0° C werden 60 µl 2%iges Triton x-100 (Merck) zugegeben. Nach 30 min. bei 0° C wird die Probe 30 min. bei 15'000 U/min. und 4° C in einem Sorval SA-600 Rotor zentrifugiert. Der Ueberstand wird mit 1 Vol. Phenol (gesättigt in TNE) deproteinisiert. Die Phasen werden durch Zentrifugieren (Sorval HB-4 Rotor) während 10 min. bei 5000 U/min. und 4° C getrennt. Die obere Phase wird zweimal mit 1 Vol. Chloroform extrahiert. Pankreatische RNAse A (Sigma; 10 mg/ml in TNE 10 min. bei 85° C vorerhitzt) wird bis zu einer Endkonzentration von 25 µg/ml zugegeben und das Gemisch 40 min. bei 37° C inkubiert. Die Lösung wird dann auf 1M NaCl und 10 % Polyethylenglykol 6000 (Fluka, 20 min. bei 120° C im Autoklaven behandelt) eingestellt und 2 Stunden bei -10° C inkubiert. Das Präzipitat wird in einem Sorval HB-4 Rotor (20 min. bei 10 000 U/min., 0° C) gesammelt und erneut in 100 µl TNE gelöst. Die DNA-Lösung wird mit 1 Vol. Phenol extrahiert und die DNA wird mit 2 Vol. Aethanol 10 min. bei -80° C präzipitiert. Das Präzipitat wird durch Zentrifugieren in einer Eppendorf-Zentrifuge gesammelt und die DNA erneut in 20 µl 10 mM Tris•HCl (pH 7,5) und 0,5 mM EDTA gelöst. Aus einer 10 ml Kultur gewinnt man 8 bis 10 µg Plasmid-DNA.

Die Plasmid-DNAs werden nach Verdauung mit den folgenden Restriktionsenzymen analysiert:

Je 0,5 µg Plasmid-DNA wird mit HpaI (Biolabs) sowie mit HpaI (Biolabs) und EcoRI (Biolabs) mit ClaI (Biolabs) nach Standard-Vorschriften, gemäss Angaben des Enzym-Herstellers, gespalten. Die DNAs werden auf einem 1 % Agarose-Gel in 40 mM Tris•Acetat (pH 7,8) 1 mM EDTA und 0,5 µg/ml Ethidiumbromid fraktioniert. Die gewünschten Plasmide enthalten eine HpaI-Stelle und ergeben nach der 3-fachen Verdauung neben dem grossen DNA-Fragment 2 kleinere Fragmente, welche grösser sind als das kleine EcoRI-ClaI-Fragment von pBR322. Eines dieser Plasmide wird mit p159 bezeichnet (siehe Fig. 3).

B. Konstruktion des Plasmids pHRi145

2 µg p159-DNA werden mit 10 Einheiten EcoRI (Biolabs) während 30 min. bei 37° C verdaut. Die DNA wird mit Phenol extrahiert, mit Aethanol gefällt und nach Zentrifugation in 10 µl 10 mM Tris•HCl (pH 7,5), 0,5 mM EDTA gelöst. Die mit EcoRI verdaute DNA wird weiterhin mit 5 Einheiten DNA-Polymerase (Klenow-Fragment) (Boehringer) in 10 mM $MgCl_2$, 10 mM β-Mercaptoethanol, 50 mM NaCl, 0,1 mM dATP (P&L Biochemicals), 0,1 mM dTTP (P&L Biochemicals) während 15 min. bei 12° C behandelt. Die Polymerase wird anschliessend durch Inkubation bei 85° C während 5 min. inaktiviert. Das Reaktionsgemisch wird in 20 mM Tris•HCl (pH 7,8), 10 mM $MgCl_2$, 10 mM DTT, 0,5 mM ATP (Sigma) auf das 10fache verdünnt und mit 30 Einheiten $T_4$ DNA-Ligase pro µl Reaktionsgemisch während 1 Stunde bei 15° C inkubiert.

50 ng der DNA werden in E. coli transformiert (wie oben beschrieben) und auf McConkey-Agarplatten supplementiert mit 50 µg/ml Ampicillin, ausplattiert.

Die Plasmid-DNAs von 10 verschiedenen Kolonien wird, wie oben beschrieben, isoliert. Die Plasmid-DNAs werden analysiert durch Verdauung mit EcoRI. Die gewünschten Plasmide sind EcoRI-resistent. Die Analyse erfolgt wie oben beschrieben. Eines der gewünschten Plasmide wird als HRi145 bezeichnet (Fig. 3).

C. Konstruktion des Plasmids pHRi148

2 µg pHRi145-DNA werden mit 5 Einheiten ClaI (Boehringer) während 60 min. bei 37° C behandelt, dann mittels Phenol-Extraktion deproteiniert. Die DNA wird mit Aethanol gefällt und dann in 20 µl 10 mM Tris•HCl (pH 7,5), 0,5 mM EDTA gelöst. Die überhängenden Enden werden, wie oben beschrieben, mit DNA Polymerase I (Klenow-Fragment) aufgefüllt, mit der Aenderung, dass dATP und dTTP ersetzt werden durch dCTP (P&L Biochemicals) und dGTP (P&L Biochemicals). Die Polymerase wird inaktiviert durch Inkubation bei 85° C während 5 min. Das Reaktionsgemisch wird mit 2 Volumina 0,1 M Tris•HCl (pH 8,7) versetzt und mit 0,5 Einheiten Kälber-Phosphatase (Boehringer) während 30 min. bei 37° C inkubiert. Das Reaktionsgemisch wird deproteiniert durch Phenol-Extraktion. Die DNA wird mit Aethanol gefällt und in 8 µl 10 mM Tris•HCl (pH 7,5), 0,5 mM EDTA gelöst.

Ein chemisch synthetisierter DNA-Linker der Formel

**5'-GAATTCCATGGTACCATGGAATTC-3'**

wird am 5'-Ende phosphoryliert, indem 8 pMol des Linkers mit 5 µCi [$\gamma$-$^{32}$P)-ATP (5500 Ci•mmol$^{-1}$ Amersham) in 8 µl Reaktionsvolumen, welches 0,1 mM rATP (Sigma), 50 mM Tris•HCl (pH 9,5), 10 mM $MgCl_2$, 5 mM DTT und 2 Einheiten $T_4$ Polynucleotid-Kinase (P&L Biochemicals) enthält, während 30 min. bei 37° C inkubiert werden. Die Reaktion wird gestoppt durch Einfrieren bei -80° C.

Der radioaktiv markierte Linker wird anschliessend mit 1 µg ClaI und Phosphatase behandelt und mit pHRi145-DNA (siehe oben) in einem 20 µl Reaktionsvolumen ligiert, welches 0,5 mM rATP (Sigma), 10 mM DTT (Calbiochem), 20 mM Tris•HCl (pH 7,8), 1 mM $MgCl_2$ und 800 Einheiten $T_4$ DNA-Ligase (Biolabs) enthält. Die Inkubation erfolgt bei 15° C während 2 Stunden. Die Ligase wird inaktiviert durch Inkubation bei 85° C während 10 min. Anschliessend werden 2 Volumina Wasser hinzugegeben, die Kochsalz-Konzentration auf 10 mM eingestellt und 20 Einheiten KpnI (Biolabs) hinzugegeben während 30 min. bei 37° C. Nach der Extraktion mit Phenol und Chloroform wird die Mischung durch ein 0,9 % niedrigschmelzendes Agarose-Gel (Biorad) in 40 mM Tris•Acetat (pH 7,8), 1 mM EDTA und 0,5 µg/ml Ethidiumbromid fraktioniert. Die durch UV-Bestrahlung sichtbare Bande, welche die gleiche Mobilität wie eine Marker-DNA gleicher Grösse aufzeigt, wird mit einem Skalpell herausgeschnitten. Das Gelstück wird 5 min. bei 65° C geschmolzen und dann auf 37° C abgekühlt. Man erhält ein Volumen von ca. 20 µl. 5 µl dieser Lösung werden entommen und mit 400 Einheiten $T_4$-Ligase (Biolabs) in 10 µl Reaktionsvolumen, welches auf 0,5 mM ATP, 10 mM DTT, 10 mM $MgCl_2$, 20 mM Tris•HCl (pH 7,8) eingestellt wird, während 12 Stunden bei 15° C inkubiert. 1/10 Volumen einer Lösung mit 100 mM Tris•HCl (pH 7,5), 100 mM $CaCl_2$ und 100 mM $MgCl_2$ werden zum Ligase-Gemisch (solifidiziert bei 15° C) hinzugegeben und bei 65° C während 5 min. inkubiert. Die Lösung wird dann verwendet, um mit Calcium behandelte E. coli HB101-Zellen, wie oben beschrieben, zu transformieren. Es wird auf Mcconkey-Agarplatten, supplementiert mit 50 µg/ml Ampicillin, ausplattiert.

Die Plasmid-DNAs von 10 verschiedenen Kolonien werden isoliert, wie oben beschrieben, und die DNA wird der folgenden Restriktionsenzymanalyse unterworfen: Je 0,5 µg Plasmid DNA wird nacheinander mit KpnI (Biolabs), NcoI (Biolabs) und EcoRI (Biolabs) nach den Vorschriften des Enzym-Herstellers gespalten. Die Spaltprodukte werden auf 1 % Agarose-Gelen in 40 mM Tris•Acetat (pH 7,8), 1 mM EDTA, 0,5 µg/ml Ethidiumbromid fraktioniert. Alle Plasmide zeigen je eine dieser Enzymspaltstellen, wie gewünscht. Eines wird als HRi148 bezeichnet.

Das Plasmid HRi148 enthält einen Tryptophan-Promotor-Operator und eine ribosomale Bindungsstelle bis und mit ATG. Hirudin als auch andere heterologe Gene können direkt über die einmalig im Plasmid vorhandenen EcoRI, NcoI, KpnI-Stellen angekoppelt werden. Desweiteren ermöglicht diese Konstruktion das direkte Koppeln und Exprimieren von heterologen Genen, ohne dass das für die Initiation der Translation notwendige ATG auf dem entsprechenden Gen vorhanden sein muss. Dies kann leicht erreicht werden durch Spaltung mit NcoI und Auffüllen der überhängenden Enden mit DNA-Polymerase I, wie beschrieben, oder durch Spalten mit KpnI und Entfernen der überhängenden Enden durch Nuklease $S_1$. Das Plasmid HRi148 ist somit ein breit anwendbares Expressionsplasmid.

D. Herstellung des linearisierten Vektors pHRi148/EcoRI/BamHI

5 µg Plasmid-DNA von pHRi148 werden mit den Restriktionsendonucleasen EcoRI und BamHI, verdaut. Der herausgeschnittene Vektor pHRi148/EcoRI/BamHI wird mittels Dichtegradientenzentrifugation isoliert.

b) Herstellung der $F_1$-DNA/EcoRI/EcoRII und der $F_2$-DNA/BamHI/EcoRII

I. Herstellung der $F_1$-DNA/EcoRI/EcoRII

5 μg Plasmid DNA vom pML 300 werden zuerst mit 10 Einheiten EcoRI Restriktionsendonuclease in 50 μl einer Lösung von 100 mM Tris•HCl (pH 7,5), 50 mM NaCl und 100 μg/ml Gelatine für 1 Stunde bei 37° C verdaut. Ein Aliquot (0,5 μg) dieser linearisierten Plasmid DNA/EcoRI wird durch Gelelution aus einem Agarosegel isoliert (vgl. Beispiel 10a)) und mit [$\gamma$-$^{32}$P]ATP radioaktiv markiert (vgl. Beispiel 12). Die Hauptmenge der Plasmid DNA/EcoRI wird dann mit dieser radioaktiv markierten DNA vermischt, mit EcoRII Restriktionsendonuclease verdaut und das EcoRII-EcoRI* DNA Fragment (109 Basenpaare) durch Gelelektrophorese an 8 % Polyacrylamid getrennt. 200 μg der $F_1$-DNA/EcoRI*/EcoRII werden durch Gelelution isoliert.

II) Herstellung der $F_2$-DNA/BamHI/EcoRII

5 μg Plasmid DNA vom pML 305 werden mit 10 Einheiten BamHI Restriktionsendonuclease gespalten. Ein Aliquot (0,5 μg) dieser linearisierte Plasmid DNA/BamHI wird durch Gelelution aus einem Agarosegel isoliert (vgl. Beispiel 10a)) und mit [$\gamma$-$^{32}$P] radioaktiv markiert (vgl. Beispiel 12)). Die Hauptmenge der Plasmid DNA/BamHI wird dann mit dieser radioaktiv markierten DNA vermischt, mit EcoRII Restriktionsendonuclease verdaut und das EcoRII-BamHI* DNA Fragment (122 Basenpaare) durch Gelelektrophorese an 8 % Polyacrylamid getrennt. 250 μg der $F_2$-DNA/BamHI*/EcoRII werden isoliert.

c) Ligation der $F_1$-DNA mit der $F_2$-DNA und Konstruktion des Expressions-Plasmids pML310

10 ng (= 473 nMol Enden) $F_1$-DNA/EcoRI/EcoRII und 9 ng (= 495 nMol Enden) $F_2$-DNA/BamHI/EcoRII werden in einem Volumen von 20 μl mit $T_4$-DNA Ligase, wie bereits unter Beispiel 10c) beschrieben, behandelt. Anschliessend wird die Mischung mit Phenol/Chloroform extrahiert und die DNA mit Alkohol präzipitiert. Der DNA-Niederschlag wird dann wie in Beispiel 10c) beschrieben gelöst und mit EcoRI- und BamHI-Restriktionsendonuclease verdaut. Die Lösung wird dann auf TNE eingestellt und 30 ng (= 50 nMol Enden) der Vektor-DNA pHR148/EcoRI/BamHI (vgl. Beispiel 13aD) zugegeben. Anschliessend wird die Lösung abermals mit Phenol/Chloroform extrahiert und die DNA mit Alkohol ausgefällt. Die präzipitierte DNA-Mischung wird, wie in Beispiel 10c) angegeben, mit $T_4$-DNA Ligase (Biolabs) behandelt. Auf diese Weise entsteht in der Lösung ein rekombiniertes Plasmid, welches die $F_1$-$F_2$-DNA (Desulfatohirudin-Gen) als Insert enthält.

d) Transformation von E. coli HB101 mit dem Plasid pML 310

Die Transformation der mit Calcium behandelten E. coli HB101 Zellen erfolgt wie in Beispiel 10d) beschrieben. Es werden 10μl des in Beispiel 13c) erhaltenen Reaktionsgemisches verwendet. 420 Ampicillin resistente Kolonien werden erhalten.

e) Screening derKolonien, die $F_1$-$F_2$-DNA enthalten

18 transformierte Kolonien (Beispiel 13d) werden auf ihren $F_1$-$F_2$-DNA-Gehalt geprüft, wie in Beispiel 10e) beschrieben. Als radioaktive Probe wird eine Mischung der in den Beispielen 5 und 6 beschriebenen Oligodeoxynucleotide verwendet. Im Autoradiogramm werden 12 positive Kolonien erhalten, fünf davon erhalten die Bezeichnung pML310, pML311, pML312, pML313, pML314.

Beispiel 14: Charakterisierung des Klons pML310

Die Charakterisierung der $F_1$-$F_2$-DNA-Sequenz in dem rekombinierten Plasmid pML310 erfolgt durch Sequenzierung der $F_1$-$F_2$-DNA nach Maxam und Gilbert (11), wie in Beispiel 12 beschrieben. 10 μg Plasmid -DNA werden geprüft. Die Nucleotidsequenz der $F_1$-$F_2$-DNA ist mit der für das synthetische Desulfatohirudin-Gen beschriebenen identisch.

Beispiel 15: Herstellung des Expressionsplasmids pML 315

a. Ligation der $F_1$-DNA mit der $F_2$-DNA und Konstruktion des Expressionsplasmids pML315

24 $\mu$g (= 1,3 pMol Enden) bzw. 26 $\mu$g (= 1,3 pMol Enden) des chemisch synthetisierten $F_1$- bzw. $F_2$-DNA (siehe Beispiel 8 und Schema I) werden in einem Volumen von 20 $\mu$l mit 5 Einheiten EcoRII Restriktionsendonuclease (Biolabs) in 50 mM Tris-HCl (pH 8), 5 mM $MgCl_2$, 50 mM NaCl, 1 mM DTT, 100 $\mu$g/ml Gelatine für 30 min. bei 37° C verdaut. Anschliessend wird die Mischung mit Phenol/Chloroform extrahiert und die DNA mit Alkohol präzipitiert. Der DNA-Niederschlag wird dann wie in Beispiel 10c) beschrieben gelöst und mit $T_4$-DNA-Ligase drei Stunden bei 15° C behandelt. Danach wir die Mischung mit Phenol/Chloroform extrahiert und wie in Beispiel 10b) beschrieben gefällt. Der DNA-Niederschlag wird anschliessend gelöst (vgl. Beispiel 10c) und mit EcoRI- und BamHI-Restriktionsendonuclease für 30 min. bei 37° C verdaut. Danach werden der Lösung 70 ng (= 0.1 pMol Enden) des linearisierten Vektors pHRi148/EcoRI/BamHI (Beispiel 13aD) zugesetzt, die ganze Lösung auf TNE eingestellt und mit Phenol/Chloroform extrahiert und die DNA mit 2 Volumen Alkohol ausgefällt.

Der erhaltene DNA Niederschlag, der die beiden DNA Bruchstücke ($F_1$-$F_2$-DNA/EcoRI/BamHI und pHRi148/EcoRI/BamHI) enthält, wird in 20 $\mu$l einer Lösung von 50 mM Tris-HCl (pH 7.8), 10 mM $MgCl_2$, 10 mM DTT, 0,5 mM ATP, 100 $\mu$g/ml Gelatine gelöst und mit 15 Einheiten/$\mu$l $T_4$-DNA Ligase (Biolabs) bei 15° C für 3 Stunden behandelt. Auf diese Weise entsteht in der Lösung das rekombinierte Plasmid pML315, welches die $F_1$-$F_2$-DNA (= Desulfatohirudin-Gen) enthält.

b) Transformation von E. coli HB101 mit dem Plasmid pML315

10 $\mu$l des das Plasmid pML315 enthaltenden Gemisches (Beispiel 15a) werden für die Transformation der Calcium-behandelten E. coli HB101 Zellen verwendet. Ca. 236 Ampicillin-resistente Kolonien werden erhalten.

c) Screening der Kolonien, die $F_1$-$F_2$-DNA enthalten

Transformierte Kolonien (Beispiel 15b) werden auf das Vorhandensein von $F_1$-$F_2$-DNA geprüft, wie in Beispiel 13e) angegeben.

Es werden fünf positive Kolonien erhalten, welche die Bezeichnung pML315 - pML319 erhalten.

## Beispiel 16: Herstellung des Expressionsplasmids pML320

a. Ligation der synthetischen $F_1$-$F_2$-DNA mit der Vektor-DNA pHRi148/EcoRI/BamHI

20 $\mu$g des total synthetisch hergestellten Desulfatohirudin-Gens ($F_1$-$F_2$-DNA, vgl. Beispiel 9) werden in 20 $\mu$l einer Lösung von 50 $\mu$l 100 mM Tris•HCl (pH 7,5), 50 mM NaCl und 100 $\mu$g/ml Gelatine mit dem Restriktionsenzym EcoRI und anschliessend mit BamHI verdaut. Die Lösung wird auf TNE eingestellt, woraufhin 30 $\mu$g (= 50 nMol Enden) der Vektor-DNA pHRi148/EcoRI/BamHI (vgl. Beispiel 13aD) zugegeben wird. Die Lösung wird mit Phenol/Chloroform extrahiert und die DNA mit Alkohol ausgefällt. Der DNA-Niederschlag wird, wie in Beispiel 10c) angegeben, mit $T_4$-DNA-Ligase (Biolabs) behandelt. Auf diese Weise entsteht in der Lösung ein rekombiniertes Plasmid (pML320), welches die $F_1$-$F_2$-DNA (Hirudin-Gen) als Insert enthält.

b. Transformation von E. coli HB101 mit dem Plasmid pML320

Die Transformation der mit Calcium behandelten E. coli HB101 Zellen erfolgt wie in Beispiel 10d) beschrieben. Es werden 10$\mu$l des in Beispiel 16a) erhaltenen Reaktionsgemisches verwendet. 173 Ampicillin resistente Kolonien werden erhalten.

c. Screening der Kolonien, die $F_1$-$F_2$-DNA enthalten

11 transformierte Kolonien (Beispiel 16b) werden, wie in Beispiel 10e) beschrieben, auf ihren $F_1$-$F_2$-DNA-Gehalt geprüft. Als radioaktive Probe wird eine Mischung der in den Beispielen 5 und 6 beschriebenen Oligodesoxynucleotide verwendet. Im Autoradiogramm werden 14 positive Kolonien erhalten. Fünf davon erhalten die Bezeichnung pML320, pML321, pML322, pML323 und pML324.

## Beispiel 17: Charakterisierung der Klone pML315 und pML320

Die Charakterisierung der $F_1$-$F_2$-Sequenzen in den Plasmiden pML315 und pML320 erfolgt durch

Sequenzierung der $F_1$-$F_2$-DNA nach Maxam und Gilbert (11), wie in Beispiel 12 beschrieben. Die Nuclotidsequenzen der DNA-Inserts beider Plasmide sind mit derjenigen des synthetischen Hirudin-Gens identisch.

Beispiel 18: Synthese von Polypeptiden mit Hirudin-Aktivität durch E. coli-Zellen, die Plasmide mit rekombinierten Hirudin-Genen enthalten

a. Synthese von Polypeptiden mit Hirudin-Aktivität

Jeder der 15 Klone, die das rekombinierte Desulfatohirudin-Gen enthalten, nämlich

*E. coli* HB101 pML 310, *E. coli* HB101 pML 311, *E. coli* HB101 pML 312, *E. coli* HB101 pML 313, *E. coli* HB101 pML 314, *E. coli* HB101 pML 315, *E. coli* HB101 pML 316, *E. coli* HB101 pML 317, *E. coli* HB101 pML 318, *E. coli* HB101 pML 319, *E. coli* HB101 pML 320, *E. coli* HB101 pML 321, *E. coli* HB101 pML 322, *E. coli* HB101 pML 323, *E. coli* HB101 pML 324

wird auf die Bildung von Hirudin-Aktivität getestet.

Zu diesem Zweck werden die oben genannten Klone in 5 ml L-Medium über Nacht (16 Stunden) bei 37° C und 250 upm kultiviert. L-Medium ist wie folgt zusammengesetzt:

| | |
|---|---|
| Bacto Tryptone | 10 g |
| Bacto Hefe-Extrakt | 5 g |
| NaCl | 5 g |
| Glucose | 5 g |
| Ampicillin | 0,1 g |

1 ml dieser Uebernachtkultur wird am nächsten Tag in 25 ml M9-Medium übertragen. M9-Medium ist wie folgt zusammengesetzt:

| | |
|---|---|
| $Na_2HPO_4 \cdot 7H_2O$ | 13,25 g |
| $KH_2PO_4$ | 3,0 g |
| NaCl | 0,5 g |
| $NH_4Cl$ | 1,0 g |
| $CaCl_2 \cdot 2H_2O$ | 0,015 g |
| $MgSO_4 \cdot 7H_2O$ | 0,25 g |
| Casaminosäuren | 2,5 g |
| Vitamin $B_1$ | 0,0099 g |
| Glucose | 5,0 g |
| Ampicillin | 0,1 g |

Es wird bei 37° C und 250 upm solange kultiviert, bis die Bakteriensuspension eine optische Dichte ($OD_{623}$) von ca. 0,9.-1,0 erreicht hat. Anschliessend erntet man die Zellen (5 ml der wachsenden Kultur) und resuspendiert die Bakterien in 0.5 ml einer Lösung von 50 mM Tris•HCl (pH 8) und 30 mM NaCl. Danach wird die Suspension auf 1 mg/ml Lysozym (Boehringer) eingestellt und 30 min. in Eis gestellt. Durch abwechselndes Einfrieren der Suspension in flüssigem Stickstoff und Auftauen bei 37° C werden die

Bakterien zerstört. Dieser Vorgang wird 5 mal wiederholt, anschliessend wird die Mischung 30 min. bei 16000 upm bei 4° C zentrifugiert. Die Ueber stände werden auf Desulfatohirudin-Gehalt untersucht, indem man mit Antikörpern (vgl. Beispiel 18) versetzt, die Ueberstände im HPLC prüft oder die Hemmung von Thrombin (15) misst.

Folgende Resultate werden erhalten:

| Bakterienextrakt | Desulfatohirudin-Bildung |
|---|---|
| E. coli HB101 pML 310 | + |
| E. coli HB101 pML 311 | + |
| E. coli HB101 pML 312 | + |
| E. coli HB101 pML 313 | + |
| E. coli HB101 pML 314 | + |
| E. coli HB101 pML 315 | + |
| E. coli HB101 pML 316 | + |
| E. coli HB101 pML 317 | + |
| E. coli HB101 pML 318 | + |
| E. coli HB101 pML 319 | + |
| E. coli HB101 pML 320 | + |
| E. coli HB101 pML 321 | + |
| E. coli HB101 pML 322 | + |
| E. coli HB101 pML 323 | + |
| E. coli HB101 pML 324 | + |
| E. coli HB101 pHRi 148 (Kontrolle) | - |
| E. coli HB101 pBR322 (Kontrolle) | - |
| E. coli HB101 (Kontrolle) | - |

Beispiel 19 : Nachweis der Hirudin-Aktivität

Ca. 5-10 µl einer Probe, welche Polypeptide mit Hirudin-Aktivität enthält (vgl. Beispiel 18), werden auf 1 $cm^2$ Nitrocellulosepapier (NZ) (BIORAD) aufgetropft und 30 min. bei Raumtemperatur getrocknet. Danach wird das NZ für 1 Stunde bei 37° C in einer Lösung von 3 % Serumalbumin in 0,01 M Tris•HCl (pH 8) und 0,9 % NaCl inkubiert.

Anschliessend wird das NZ in einer Lösung von 0,01 M Tris•HCl (pH 8) und 0,9 % HaCl für 30 min. gewaschen. Dabei wird die Lösung 5 mal gewechselt. Danach wird das gewaschene NZ für 2 Stunden bei 25° C in einer Lösung von 3 % Serumalbumin in 0,01 M Tris•HCl (pH 8) und 0,9 % NaCl, die 2 µg/ml Antikörper (aus Kaninchen hergestellte, oder monoklonale Antikörper) gegen Hirudin enthält, behandelt. Danach wird das NZ, wie oben beschrieben gewaschen.

Anschliessend wird das NZ für 2-3 Stunden bei 25° C mit einer Lösung von 3 % Serumalbumin in 0,01 M Tris•HCl (pH 8) und 0,9 % NaCl, die 0,2 µCi/ml $^{125}$I-Protein A (sp. Aktivität 89,8 µCi/mg) (NEN) enthält, behandelt. Danach wird abermals das NZ, wie oben beschrieben, gewaschen, getrocknet und in einem γ-Zähler (Multi Gramma, 1260 gamma counter, LKB, Wallace) die gebundene Radioaktivität bestimmt, welche ein Mass für das auf dem NZ vorhandene Polypeptid mit Hirudin-Aktivität ist.

In einem alternativen Verfahren wird obige Probe einer SDS-Polyacrylamidgel-Elektrophorese (PAGE)

unterworfen [vgl. (24)]. Das PAGE-Elektropherogramm wird durch Elektro-Blotting auf NZ übertragen. Danach wird die NZ wie oben beschrieben behandelt und/oder über Nacht zusammen mit einem Röntgenfilm (Fuji) autoradiographiert. Stellen auf dem NZ, welche Polypeptide mit Hirudin-Aktivität enthalten, erscheinen auf dem Film als schwarze Flecken.

Beispiel 20: Isolierung und Reinigung von Desulfatohirudin mit Hilfe einer Thrombin-Säule

a. Herstellung der Polypeptidlösung für die Thrombin-Säule.

150 ml Kulturbrühe (erhalten gemäss Beispiel 18) werden auf 4° C abgekühlt und die Zellen durch Zentrifugation (5000 upm, 15 min., Sorvall RC 3B) abgetrennt. Der klare Ueberstand enthält keine Hirudin-Aktivität.

Anschliessend werden die Zellen in 12 ml Lyse Puffer (50 mM Tris•HCl pH 8, 30 mM NaCl) suspendiert. Dieser Mischung werden 15 mg Lysozym (Boehringer) zugesetzt und diese dann 30 min. bei 4° C aufbewahrt. Anschliessend werden die Zellen durch 4 maliges Einfrieren in flüssigem Stickstoff und anschliessendem Auftauen bei 37° C zerstört. Danach wird 30 min. bei 16000 upm 4° C zentrifugiert. Der Ueberstand enthält die Hirudin-Aktivität. In dem Ueberstand (15 ml) werden dann 7,7 g festes Ammoniumsulfat aufgelöst. Die trübe Mischung wird bei 4° C für 30 min. stehengelassen und dann zentrifugiert (s. oben). Das nasse Sediment wird in 1 ml 0,05 mM Tris•HCl pH 8 Puffer gelöst und man erhält die gewünschte Polypeptidlösung.

b. Reinigung von Hirudin auf einer Thrombin-Säule.

Die Thrombin-Säule (Bettvolumen 4 ml) ist mit 0,05 M Tris-HCl pH 8 äquilibriert. 5 ml-Portionen der oben erhaltenen Polypeptid-Lösung werden bei 4° C auf die Säule mit einer Fliessgeschwindigkeit von 7 ml/Stunde aufgetragen. Gewaschen wird dann mit 25 ml 0.05 M Tris•HCl (pH 8). Die ersten Fraktionen enthalten die nicht adsorbierten Polypeptide, welche verworfen werden. Anschliessend wird die Säule mit 10 ml1,5 M Benzamidin (Merck) [vgl. (25)] in 0,05 M Tris•HCl (pH 8) gewaschen und die erhaltenen Fraktionen auf Hirudin-Aktivität mit dem Thrombin-Test (15) geprüft. Die Fraktionen, welche die Polypeptide enthalten, werden durch Messung der $OD_{280nm}$ bestimmt. Fraktionen 25 und 26 enthalten die Hirudin-Aktivität; sie werden bei -20° C aufbewahrt oder bis zur Weiterverarbeitung im Eisbad. Die Hirudin-Aktivität beträgt in Fraktion 25 20 μg/ml und in Fraktion 26 52 μg/ml. Danach werden die Fraktionen dialysiert oder über Sephadex-G25 (Pharmacia) entsalzt. SDS-Polyacrylamidgel-Elektrophorese (24) des Produktes ergibt ein Molekulargewicht von ca. 7000-8000 Dalton und ganzzahlige Vielfache davon (Oligomere).

c. Herstellung der Thrombin-Säule

Affi-Gel 10 (Bio Rad) wird nach Angaben des Herstellers mit kaltem destilliertem Wasser und Kupplungspuffer pH 8,5 (0.1 M $NaHCO_3/Na_2CO_3$-Lösung) gewaschen. Eine 50 %ige Suspension der Gels in Kupplungspuffer (4 ml) wird in ein Plastikrohr übertragen, mit der gleichen Menge Thrombinlösung (120 mg in 4 ml Kupplungspuffer) (Calbiochem) vermischt und über Nacht bei 4° C rotiert. Danach wird das Gel mit Kupplungspuffer gewaschen. Zur Blockierung der noch freien aktiven Stellen wird das Gel mit 0,1 ml 1 M Aethanolamin-HCl (pH 8,0) pro Gel während 3 Stunden bei 4° C behandelt dann mit phosphatgepufferter Salzlösung, enthaltend 10 mM Natriumazid pro ml Gel gewaschen und darin bei 4° C gehalten. Der Kupplungsgrad wird durch Messung der Extinktion bei 280 nm bestimmt und beträgt 15 bis 30 mg Hirudin pro ml Gel.

4 ml des entstandenen Thrombin-Gels werden verwendet, um die Affinitätssäule herzustellen.

Beispiel 21: Transformation verschiedener E. coli-Stämme mit dem Plasmid pML310 und Kultivierung der transformierten Wirtszellen

In analoger Weise, wie in Beispiel 10d) beschrieben, werden die Stämme E. coli JA221, E. coli LM1035 und E. coli W3110Δ102 mit dem Plasmid pML310 transformiert. Transformierte Kolonien werden auf das Vorhandensein von $F_1$-$F_2$-DNA geprüft, wie in Beispiel 10e) beschrieben. Es werden 5, 3 bzw. 5 positive Kolonien erhalten, die die folgenden Bezeichnungen erhalten:

*E. coli* JA221/pML310/1
*E. coli* JA221/pML310/2
*E. coli* JA221/pML310/3
*E. coli* JA221/pML310/4
*E. coli* JA221/pML310/5
*E. coli* LM 1035/pML310/1
*E. coli* LM 1035/pML310/2
*E. coli* LM 1035/pML310/3
*E. coli* W3110Δ102/pML310/1
*E. coli* W3110Δ102/pML310/2
*E. coli* W3110Δ102/pML310/3
*E. coli* W3110Δ102/pML310/4
*E. coli* W3110Δ102/pML310/5

Die genannten Klone werden in einem modifizierten M9-Medium kultiviert, welches die folgende Zusammensetzung aufweist:

| | | |
|---|---|---|
| $Na_2HPO_4 \cdot 7H_2O$ | 9,0 | g |
| $KH_2PO_4$ | 3,0 | g |
| NaCl | 0,5 | g |
| $NH_4Cl$ | 3,5 | g |
| $CaCl_2 \cdot 2H_2O$ | 0,015 | g |
| $MgSO_4 \cdot 7H_2O$ | 0,25 | g |
| Casaminosäuren | 7,0 | g |
| Hefeextrakt | 5,0 | g |
| Vitamin $B_1$ | 0,0099 | g |
| Eisen-III-citrat | 0,006 | g |
| MOPS (3-Morpholinopropan-1-sulfonsäure) | 34,0 | g |
| Glucose | 20,0 | g |
| Ampicillin | 0,1 | g |

Es wird bei 37°C und 180 upm solange kultiviert, bis die Bakteriensuspension eine optische Dichte ($OD_{623}$) von ca. 1 erreicht hat. Anschliessend erntet man die Zellen (5 ml der wachsenden Kultur) und resuspendiert die Bakterien in 0,5 ml einer Lösung von 50 mM Tris•HCl (pH 8) und 30 mM NaCl. Danach wird die Suspension auf 1 mg/ml Lysozym (Boehringer) eingestellt und 30 min. in Eis gestellt. Durch abwechselndes Einfrieren der Suspension in flüssigem Stickstoff und Auftauen bei 37°C werden die Bakterien zerstört. Dieser Vorgang wird 5 mal wiederholt. Anschliessend wird die Mischung 30 min. bei

16000 upm bei 4°C zentrifugiert.

Alle Klone werden auf die Bildung von Polypeptiden mit Hirudin-Aktivität untersucht, in dem die Hemmung von Thrombin (15) gemessen wird. In allen Bakterienextrakten wird Hirudin-Aktivität (300-600 ng/ml Kulturlösung) festgestellt. Beispielsweise werden folgende Hirudin-Aktivitäten bestimmt:

| Stamm | Hirudin-Aktivität (µg/l Kulturlösung) |
|---|---|
| E. coli LM1035/pML310/1 | 300 |
| E. coli JA 221/pML310/1 | 500 |
| E. coli W3110Δ102/pML310/1 | 600 |

Beispiel 22: Fermentation des transformierten Stammes E. coli W3110Δ102/pML310/1 im 5000 l Fermenter und Aufarbeitung der Kulturbrühe

In analoger Weise, wie in Beispiel 21 beschrieben, werden in einem 5000 l Fermenter E. coli W3110Δ102/pML310/1-Zellen in 3000 l modifiziertem M9-Medium kultiviert, bis die Suspension eine optische Dichte ($OD_{623}$) von ca. 10-13 erreicht hat.

Die Kulturbrühe (pH 7,4) wird auf 10°C abgekühlt, und die Zellen werden mit einer Alfa-Laval BRPX-207 Entschlammvorrichtung behandelt. Der klare Ueberstand enthält keine Hirudin-Aktivität und wird verworfen. Während der Entschlammung wird der Schlammraum fortwährend mit Lysepuffer A (50 mM Tris•HCl, 30 mM NaCl, eingestellt mit HCl auf pH 8,0) teilentschlammt und am Schluss der Inhalt der Zentrifugenschale (7 1) unter voller Entschlammung mit Lysepuffer A ausgestossen. Die erhaltene Zellmasse wird mit Puffer A auf 375 l eingestellt und besitzt einen pH-Wert von 7,6. Nach dem Kühlen auf 5-10°C wird die Suspension durch eine Dyno-Mühle (Typ KD5) geleitet, welche mit 4,2 l Glasperlen mit einem Durchmesser von 0,5-0,75 mm ausgerüstet ist. Dabei werden die Zellen zerstört. Die so erhaltene Suspension wird mit Essigsäure auf einen Essigsäuregehalt um 2 %(v/v) eingestellt und über Nacht bei 10°C gerührt. Die Suspension mit einem pH von 3,9 wird mit der oben beschriebenen Technik entschlammt. Der klare Ueberstand von 300 l wird in einem Fallfilmverdampfer (Stundenleistung 60 l Wasser) auf 35 l aufkonzentriert. Das leicht trübe Konzentrat wird zentrifugiert und der so erhaltene klare Ueberstand auf einer Ultrafiltrationsanlage DDS = Lab 35, welche mit GR 81 PP Membrane bestückt ist (Fläche 2,5 $m^2$), gegen 2 % Essigsäure oder 0.02 M Tris-HCl pH 7.5 Puffer diafiltriert. Das Endvolumen beträgt 31 l.

Ein aliquoter Teil von 2 l dieser klaren Proteinlösung wird auf eine Sephadex G-50 F-Säule (KS 370 Pharmacia) mit einem Bettvolumen von 96 l aufgetragen, wobei die Säule mit 2 % Essigsäure äquilibriert ist. Die in 15 l Eluat enthaltene Hauptfraktion wird mittels Ultrafiltration eingeengt und anschliessend gegen Wasser diafiltriert. Die so erhaltene klare wässrige Lösung wird lyophilisiert. Die Aufarbeitung kann, wie in den folgenden Beispielen beschrieben, durchgeführt werden.

Beispiel 23: Kultivierung des Stammes E. coli W3110Δ102/pML310/1 im Labormassstab

Der Stamm E. coli W3110Δ102/pML310/1 wird bei 37°C und 200 upm 24 Stunden in Schüttelkolben inkubiert. Das Medium weist folgende Zusammensetzung auf (g/l):

| | |
|---|---|
| $Na_2HPO_4 \cdot 7H_2O$ | 9,0 g |
| $KH_2PO_4$ | 3,0 |
| NaCl | 0,5 |
| $CaCl_2$ | 0,015 |
| $MgSO_4 \cdot 7H_2O$ | 0,25 |
| Eisen(III)-citrat | 0,006 |
| MOPS | 40 |
| $NH_4Cl$ | 3,5 |
| Casamino Säuren | 7,0 |
| Hefeextrakt | 5,0 |
| Cerelose (Glucose) | 20 |
| Ampicillin | 0,1 |

Das Medium hat einen pH-Wert von 6,54. Während der Kultivierung wird eine optische Dichhte ($OD_{605nm}$) von 14,9 erreicht.

Die Zellen (50 ml der wachsenden Kultur) werden geerntet und in 5 ml einer Lösung von 50 mM Tris • HCl (pH 8.0) und 30 mM NaCl resuspendiert. Danach wird die Suspension mit 5 g gekühlten Glasperlen (Durchmesser 0,5 mm), oder durch Zugabe von Lysozym (Boehringer), Endkonzentration 1 mg/ml, während 30 bis 45 Minuten in Intervallen auf einem Multi-Tube Vortexer bei hoher Intensität geschüttelt. Zu 0,5 ml der Mischung werden 0,5 ml 2 % Essigsäure zugegeben und die Mischung wird 20 min bei 4° C und 12'000 upm zentrifugiert. Die überstehende Lösung wird auf Hirudin-Aktivität (Hemmung von Thrombin) geprüft. Der Thrombin-Hemmtest wird wie folgt durchgeführt:

Der Test beruht auf der Bestimmung der enzymatischen Abspaltung von p-Nitroanilin aus dem Substrat Chromozym TH (Tosyl-glycyl-prolylarginin-4-nitroanilid-acetat). Das gebildete p-Nitroanilin wird kolorimetrisch bei 405 nm gemessen (Micro-Elisa Auto Reader von Dynatech, Kloten). Der Test wird in Mikrotiterplatten mit flachem Boden (Costar, Serocluster, Catalog Nr. 3590) durchgeführt. Die Reaktionszeit beträgt 2 h bei 37° C im Brutschrank. Für den Test werden 20 µl der zu bestimmten Lösung mit 30 µl Füllpuffer (0,2 M Tris•HCl pH 7,5, 1 M NaCl, 100 µg/ml Rinderserumalbumin) verdünnt und mit 20 µl Enzym-Lösung [10 mg lyophilisiertes Thrombin aus Humanplasma (Boehringer) in 1 ml bidestilliertem Wasser gelöst und ca. 1:400 mit obigem Füllpuffer verdünnt] und mit 150 µl Substrat-Lösung [20 ng Chromozym TH (Boehringer) in 4 ml bidestilliertem Wasser gelöst und 1:15 mit obigem Füllpuffer verdünnt] versetzt. Alle Lösungen werden gegen eine Blank-Lösung, bestehend aus 70 µl Füllpuffer (s.o.) und 150 µl Substrat-Lösung und gegen eine Enzym-Kontrollösung, bestehend aus 50 µl Füllpuffer, 20 µl Enzym-Lösung und 150 µl Substrat-Lösung gemessen. Der $OD_{405nm}$-Wert der Enzym-Kontrollösung nach 2 h bei 37° C ergibt 100 % Aktivität und sollte ca. 0,8 ± 0,2 betragen. Die Thrombin-Hemmung (in %) der Proben errechnet sich nach der Formel %-Thrombin-Hemmung =

$$100 - \frac{100 \text{ x } OD_{405nm} \text{ der Probe}}{OD_{405nm} \text{ der Enzym-Kontrollösung}}$$

Die überstehende Lösung weist eine Thrombin-Hemmung von 49,7 %/20 µl auf.

Beispiel 24: Reinigungsverfahren zur Anreicherung von Hirudin-Verbindungen aus Kulturbrühen

In analoger Weise wie in Beispiel 22 beschrieben, wird der Stamm E. coli W3110Δ102/pML310/1 im Fermenter kultiviert. Dann werden die Zellen aufgeschlossen, abzentrifugiert und der aufkonzentrierte Ueberstand nach Essigsäurebehandlung ultrafiltriert oder dialysiert. Solcherart gewonnene Lösungen können beispielsweise mittels Ionenaustauschchromatographie gereinigt werden.

a) Anionenauatauschchromatographie über eine MONO Q Säule (Pharmacia) zur Gewinnung von Thrombin hemmenden Fraktionen)

8,5 ml Proteinlösung (20 mM Tris • HCl pH 7,5/150 mM NaCl) werden auf die Mono Q Säule mittels eines Chromatographiesystems der Fa. Pharmacia ("fast protein liquid chromatography" FPLC) aufgetragen und bei folgenden Bedingungen eluiert.

Experimentelle Bedingungen:

Säule: Mono Q, 4,6mm x 150 mm. Puffer A: 20 mM Tris • HCl pH 7,5; Puffer B: 20 mM Tris • HCl/l M NaCl. Linearer Salzgradient: A: während 9 ml mit 15 % Puffer B eluieren, während 10,7 ml auf 70 % Puffer B steigern. AUFS 1.0 bei 280 nm, 1 ml Fraktionen.

Im Ausschlussvolumen (Fraktionen 1-10) wird ein grosser Proteingipfel von den nachfolgend eluierenden Hirudin-Verbindungen abgetrennt. Die Fraktionen 17-23 zeigen im Thrombin-Test eine Thrombin-Hemmung zwischen 20 und 100 %, wobei die Hauptfraktion (Fraktionen 20 und 21) mit 92 % Hemmung, bei einer Konzentration von ca. 500 mM NaCl eluiert.

b) Anionenaustauschchromatographie an einer DEAE Cellulose-Säule zur Anreicherung von Hirudin Verbindungen.

10 ml eines Dialysats wird der Anionenaustauschchromatographie an DE 53 (Fa. Whatman) bei pH 7,5 unterworfen (Chromatographiebedingungen: Säule 1 x 5 cm (10 ml). Elutionspuffer A: 20 mM Ammoniumacetat, 100 mM NaCl, pH 7,5. Elutionspuffer B: 20 mM Ammoniumacetat 1 M NaCl, pH 4,0. Linearer Salzgradient je 4 Säulenvolumen Puffer A, resp. B). Hirudin-Verbindungen eluieren ab 0,4 M NaCl. Der Nachweis gelingt mit dem Thrombin-Hemmtest.

c) Entsalzung an P-6 DG "Desalting Gel" (Fa. Bio-Rad)

110 ml Dialysat (20 mM Tris • HCl, pH 7,5, konzentrierte Proteinlösung aus 2,6 l Ueberstand) wurden in analoger Weise wie in Beispiel 24b beschrieben an DE 53 getrennt. Hirudin-Verbindungen eluieren zwischen Fraktionen 46 und 52 (150 ml), getrennt von verschiedenen, rascher eluierenden nicht-aktiven Proteingipfel, und werden am Rotationsverdampfer auf ca. 12 ml konzentriert. Die so erhaltene, klare Proteinlösung wird auf eine Biogel P-6 DG Säule aufgetragen und wässerig eluiert.

Experimentelle Bedingungen:

Säule 2,5 x 9 cm, stationäre Phase 45 ml Biogel P-6 DG Desalting Gel, Durchflussgeschwindigkeit 1,15 ml/min, 4,6 ml Fraktionen. Der Salzgehalt kann durch Leitfähigkeitmessung oder Testierung mittels Silbernitrat (AgCl-Fällung) überprüft werden. Die Hauptaktivität eluiert zwischen den Fraktionen 7 bis 10, wobei der Nachweis mittels dem Thrombin Hemmtest gelingt. Die Fraktionen 8-10 (13.5 ml) werden vereinigt, am Rotationsverdampfer auf 1 ml aufkonzentriert und die klare Lösung auf eine Sephadex G 50 fine-Säule (Pharmacia) mit einem Bettvolumen von 160 ml aufgetragen, wobei die Säule mit 1 % Essigsäure äquilibriert ist.

d) Gelfiltration an Sephadex G50 fine (Fa. Pharmacia)

Experimentelle Bedingungen: Säule: Sephadex G50 fine (31 g), 2,5 cm x 64 cm, Volumen 310 ml; Lösungsmittel: 1 % Essigsäure; Durchflussgeschwindigkeit 43 ml/h, 3,8 ml Fraktionengrösse. AUFS: 0,1 bei 282 nm. Papiervorschub 3,0 cm/h. Der Nachweis der eluierten Aktivität erfolgt mit dem Thrombin-Hemmtest. Die in 50 ml Eluat (Fraktionen 39-52) enthaltene Hauptfraktion eluiert rascher als nicht-aktive Nebenkomponenten. (Fraktionen 53 bis 80). Die Thrombin-Hemmung der aktiven Fraktionen beträgt zwischen 80 und 100 % Hemmung.

e) Kationenaustauschchromatographie an einer CM-Cellulose zur Voranreicherung von aktiven Lysaten

10 ml Dialysat [20 mM Tris • HCl, Konzentrat (1:20) aus 200 ml Ueberstand, pH 2,0 eingestellt mit 3 ml

4N HCl] wurden auf einem Kationentauscher (CM-Cellulose) getrennt. Hirudinverbindungen eluieren im Ausschlussvolumen zwischen Fraktionen 4-8. Der Nachweis erfolgt mittels des Thrombin-Hemmtests. Die Fraktionen 5 und 6 hemmen zu 89 %, respektive 88 % pro 20 µl Eluat.

Experimentelle Bedingungen:

Säule: 1,5 x 8 cm, stationäre Phase: 19 ml CM-Cellulose (los MZ-3146). Durchflussgeschwindigkeit:43 ml/h, 3,8 ml Fraktionen. Linearer Salzgradient Puffer A: 20 mM $NH_4OAc$, pH 2 eingestellt mit 4N HCl, Puffer B: 200 mM $NH_4OAc$ pH 6.5, je 5 Säulenvolumen, AUFS 1,0 bei 282nm.

Beispiel 25: Fermentation des transformierten Stammes E. coli W3110Δ102/pML310/1 und Aufarbeitung der Kulturbrühe

In analoger Weise wie in Beispiel 22 beschrieben, werden in einem Fermenter E. coli W3110Δ102/pML310/1 Zellen in 8 l Medium L während 5 h kultiviert, wobei die Suspension eine optische Dichte ($OD_{623}$) von ca. 5 erreicht hat.

Die aus der Kulturbrühe (pH 7,2) abzentrifugierten Zellen werden auf 10° C abgekühlt, durch Lysozymbehandlung und durch mehrmaliges (4x) abwechselndes Einfrieren in flüssigem Stickstoff und Auftauen auf ca. 30° C aufgeschlossen. Die so erhaltene Suspension (2,5 l) wird mit 1,5 % Essigsäure (2,4 l) verdünnt und 30 min. bei 4° C gerührt. Die ausgefallenen bakteriellen Proteine und andere Zellbestandteile werden für 30 min. bei 4° C bei 9000 Upm abzentrifugiert. Der klare Ueberstand von 3,2 l wird in einem Rotationsverdampfer (Fa. Büchi) auf 320 ml aufkonzentriert. Das Konzentrat wird über Nacht gegen 20 mM Tris • HCl pH 7.5 bei 4° C dialysiert und das leicht trübe Dialysat nochmals zentrifugiert. Das Endvolumen beträgt 300 ml. Die klare Proteinlösung (0,02 M Tris • HCl pH 7,0) wird auf eine Diäthylaminoäthylcellulose-(DE53 der Fa. Whatman) (20 g) Säule mit einem Bettvolumen von 20 ml aufgetragen, wobei die Säule mit Puffer A äquilibriert ist (Puffer A: 20 mM Ammoniumacetat/100 mM NaCl, pH 7,5). Vorerst wird mit 1 Säulenvolumen (45 ml) bei konstantem Fluss (43 ml/h) gewaschen und anschliessend mit einem linearen Salzgradienten mit je fünf Säulenvolumen Puffer A, respektive Puffer B eluiert (Puffer B: 20 mM Ammoniumacetat/4 M NaCl, pH 5,0). Die in 100 ml Eluat enthaltene, im Thrombin-Hemmtest aktive Hauptfraktion (Fr. 24-26) wird über Nacht bei 4° C gegen 20 mM Tris • HCl pH 7,5 dialysiert und anschliessend am Rotationsverdampfer bei 35° C auf 5 ml Endvolumen aufkonzentriert.

Die so erhaltene klare wässerige Lösung wird auf eine Sephadex G-50 fine Säule (Pharmacia) mit einem Bettvolumen von 160 ml aufgetragen, wobei die Säule (2,5 x 64 cm Biorad) mit 5 % Essigsäure äquilibriert ist. Die in 50 ml Eluat (Fraktionen 5-7, Fluss 50 ml/h, 25 min./Fraktion) enthaltene Hauptaktivität wird am Rotationsverdampfer eingeengt und anschliessend einer "Reverse Phase" HPLC-Trennung unterworfen. Ein Aliquot (500 µl) der einzelnen Fraktionen wird am "speed vac" Konzentrator (Fa. Savant) 1:10 konzentriert und mittels der "Reversed Phase"-HPLC-Analyse auf ihren Gehalt an Desulfatohirudin-Verbindungen untersucht. Die Lösungen enthalten Desulfatohirudin-Verbindungen. Fraktion 6 (18 ml) enthält prozentual den geringsten Anteil an Nebenkomponenten und wird mittels semipräparativer "Reversed Phase"-HPLC weiter gereinigt.

Experimentelle Bedingungen:

Vydac 218 TP 510 RP-HPLC-Säule, 10 x 250 mm; Aliquote Anteile der Fraktion 6 (200 µl 1:10 konzentriert) pro Trennung; AUFS 0,5 bei 220 nm; Durchflussgeschwindigkeit: 2 ml/min. Eluent: A:0.1 % Trifluoressigsäure, B:Acetonitril/Wasser 8:2 + 0.07 % Trifluoressigsäure, 1 min 16 % B, dann während 40 min auf 64 % B steigen. Die so erhaltenen Fraktionen werden 1:1 mit Wasser verdünnt und lyophilisiert. Der Rückstand besteht aus reinen Desulfatohirudin-Verbindungen.

Beispiel 26: Analyse des Produktgemisches aus der Fermentation von E. coli W3110Δ102/pML310/1

Die gemäss Beispiel 25 erhaltenen, im Thrombin-Hemmtest aktiven Fraktionen werden einer HPLC-Analyse unter zwei verschiedenen Bedingungen unterworfen.

a) Experimentelle Bedingungen Nr. 1:

Nucleosil (MN) C18, 5 µm RP-HPLC Säule, 4,6 x 120 mm; 50 µl Hirudin-Verbindungen pro Trennung, Att. 6 bei 214 nm; Durchflussgeschwindigkeit 1,2 ml/min; Eluent: A:0,1 % Trifluoressigsäure, B:

Acetonitril/Wasser 8:2 mit 0.07 % Trifluoressigsäure, 2 min 16 % B, dann während 30 min. auf 64 % B steigern. Gegendruck: 107 bar.

In einminütigem Abstand werden Fraktionen genommen (insgesamt 30), die dem Thrombin-Hemmtest und dem Antikörpertest (vgl. Beispiel 19) unterworfen werden. Fraktionen 12-15 erweisen sich als aktiv. Ausserdem werden die Aminosäurezusammensetzung, der N-Terminus und der C-Terminus für die aktive Hirudin-Verbindung der Fraktion 14 bestimmt. Diese Fraktion (Nr. 14) wird zudem mit Desulfatohirudin verglichen, welches analog wie in Beispiel 28 beschrieben durch Umsetzung von natürlichem Hirudin aus Blutegel mit dem Enzym Arylsulfatase erhalten wird. Die Resultate sind in der folgenden Tabelle 1 zusammengefasst:

**Tabelle 1:**

| Fraktion | Retentionszeit (min) | Thrombin-Hemmung | (%) | Antikörper Test | Δ (cpm) |
|---|---|---|---|---|---|
| Fr 12 | 11,75 | + | 35 | + | |
| Fr 13 | 12,50 | ++ | | + | |
| Fr 14 | 12,80 | ++ | 79 | + | 13850 |
| Fr 15 | 13,14 | + | 27 | + | 12440 |
| Kontrolle | - | - | 9 | - | 250 |
| Desulfato-Hirudin | 12.8 | ++ | 86 | + | |

Fraktionen des Kontrollstammes E. coli W3110Δ102 ("Kontrolle")) sind weder im Thrombin-Hemmtest noch im Antikörpertest aktiv. Solche Fraktionen werden erhalten durch Kultivierung des nicht-transformierten Stammes E. coli W3110Δ102 unter den gleichen Bedingungen, wie in Beispiel 22 beschrieben, und durch Aufarbeitung der Kulturbrühe gemäss Beispiel 25.

b.) Experimentelle Bedingungen Nr. 2:

RP-HPLC Säule, Vydac 218 TP 5415 4,6 x 150 mm; 100 μl (1:10 Konz.) Fraktion 6 (vgl. Beispiel 25) pro Trennung; AUFS 0,1 bei 214 nm; Durchflussgeschwindigkeit 1,2 ml/min; Eluent: A: 0,1 % Trifluoressigsäure, B: Acetonitril/Wasser 8:2 + 0.07 % Trifluoressigsäure. Gradient: 2 min 16 % B, dann während 3 min. auf 20 % B steigern, dann während 15 min auf 25 % B steigern, dann während 27 min. auf 64 % B steigern. Gegendruck 160 bar.

In der folgenden Tabelle 2 sind die im Thrombin-Hemmtest und im Antikörpertest aktiven Fraktionen aufgelistet. Die Tabelle enthält weiterhin die Konzentrationen an NaCl in mM, die zur Eluierung der Fraktionen von einer Mono Q-Säule (Anionenaustauschchromatographie) erforderlich sind (vgl. Beispiel 24a), sowie die langwelligsten UV-Absorptionsbanden einiger Fraktionen. Alle Fraktionen zeigen zudem eine positive Reaktion mit anti-Hirudin-Antikörpern (vgl. Beispiel 30).

Tabelle 2

| Fraktion Nr. | Retentionszeit (min.) RP-HPLC | Thrombin-Hemmung % | Antikörper Test Δ/cpm | FPLC | UV($\lambda_{max}$) mM NaCl | Ausbeute µg |
|---|---|---|---|---|---|---|
| 1 | 15,63 | 97,8 ± 2 | 16'196 | 350 | 223s,274 | 4 |
| 2 | 16,83 | 91,9 ± 2 | 14'157 | 390 | 275 | 1.6 |
| 3 | 18,43 | 79,9 ± 2 | 9'257 | | | 1 |
| 4 | 19,6 | 81,7 ± 5 | 11'877 | 420 | | 1.5 |
| 5 | 20,6 | 50,4 ± 5 | 7'647 | 460 | | 1.5 |
| 6 | 21,53 | 66,1 ± 5 | 9'584 | 500 | 273 | 1.5 |
| Kontrolle | - | 0 | 508 | | --- | - |
| PBS-Puffer | - | - | 66 | | | - |
| Desulfato-Hirudin | 15,45 | 97,3 ± 2 | 15'400 | 350 | 274 | ca. 4 |

Beispiel 27: Charakterisierung von Desulfatohirudin-Verbindungen aus der Fermentation des Stammes E. coli W3110Δ102/pML310/1

I. Die Hirudin Verbindung aus Fraktion 14 (vgl. Beispiel 26 unter "Experimentelle Bedingungen Nr. 1", Tabelle 1) oder Fraktion 1 (vgl. Beispiel 26 "Experimentelle Bedingungen Nr. 2", Tabelle 2) wird wie folgt charakterisiert:

a) Bestimmung der Aminosäurezusammensetzung

Ca. 2,5 µg Hirudin-Verbindung werden mit 6 N HCl bei 110°C während 24 h hydrolysiert und dann nach Chang et al. (DABS-Cl-Methode) (Zitat 31) analysiert. Das Hydrolysat weist die folgende Zusammensetzung auf.

| Aminosäure | Hydrolisat | | Aminosäure | Hydrolisat | |
|---|---|---|---|---|---|
| Asp | 9,8 | (9) | Ile | 1,8 | (2) |
| Thr | 4,2 | (4) | Leu | 3,9 | (4) |
| Ser | 4,4 | (4) | Tyr | 1,5 | (2) |
| Glu | 12,6 | (13) | Phe | 1 | (1) |
| Pro | 3,7 | (3) | His | 1,1 | (1) |
| Gly | 9,3 | (9) | Lys | 3,4 | (3) |
| Val | 3,5 | (4) | Met | 0 | (0) |
| Cystin | 2,2 | (3) | Total | | (65) |

b) Partielle Sequenzanalyse

5 $\mu$g (750 pMol) der Hirudin-Verbindung wird einer klassischen Sequenzanalyse nach Edman unterworfen und die N-terminalen Phenylthiohydantoin (PTH)-aminosäuren mittels RP-HPLC bestimmt.

Ergebnis:

| Cyclus | 1 | | | | 5 | | | | | 10 |
|---|---|---|---|---|---|---|---|---|---|---|
| Aminosäure | Val | Val | Tyr | Thr | Asp | n.b. | Thr | Glu | Ser | Gly |

| Cyclus | 11 | | | | 15 | | |
|---|---|---|---|---|---|---|---|
| Aminosäure | Gln | Asn | Leu | n.b. | Leu | n.b. | Glu |

(n.b. nicht bestimmt).

Die N-terminale Sequenz der untersuchten biosynthetischen Hirudin-Verbindung ist somit identisch mit derjenigen des natürlichen Hirudins (Zitat 1).

c) Bestimmung der C-terminalen Aminosäuren mittels Carboxypeptidase Y-Abbau

Der zeitabhängige Abbau der C-terminalen Aminosäuren mit Carboxypeptidase Y ergibt als C-Terminus

60 65

- Ile - Pro - Glu - Glu - Tyr - Leu - Gln

Die Bestimnung der Aminosäuren erfolgte mit dem Aminosäureanalysator. Es ergibt sich, dass $Tyr^{63}$ nicht sulfatiert ist (Desulfato-hirudin!).

Bei dem Hauptprodukt der Fermentation des transformierten Stammes E. coli W3110Δ102/pML310/1 handelt es sich, wie die Strukturdaten und der Vergleich mit aus natürlichem Hirudin gewonnenen Desulfatohirudin beweisen, um Desulfatohirudin.

II. Die Struktur der übrigen Fermentationsprodukte ist noch nicht vollständig geklärt. Bei den Fraktionen

2-6 (vgl. Tabelle 2) handelt es sich um definierte Verbindungen, die anhand der HPLC- und FPLC-Daten sowie des UV-Absorptionsmaximums ausreichend definiert sind. Mit Sicherheit handelt es sich bei diesen Verbindungen um Desulfatohirudin-ähnliche Verbindungen, die sich von Desulfatohirudin durch eine strukturelle Mikroheterogenität (z.B. unterschiedliche verknüpfte S-S-Brücken) oder durch Modifikation am N-bzw. C-Terminus unterscheiden (z.B. N-terminaler Methionyl-Rest oder N-terminal acyliert, wie formyliert oder acetyliert).

Auch bei der Fermentation der Stämme E. coli LM1035/pML310/1, E. coli JA221/pML 310/1 und E. coli HB101/pML310 kann Desulfatohirudin als Hauptprodukt identifiziert werden.

Beispiel 28: Herstellung von Desulfatohirudin aus natürlichem Hirudin zu Vergleichszwecken

Natürliches Hirudin wird zu 2 mg/ml Puffer (0,1 M Ammoniumacetat, pH 5,5) gelöst. Zu 20 $\mu$g Hirudin (10 $\mu$l Lösung) werden 100 $\mu$l einer entsalzten Arylsulfatase (Boehringer)-Lösung gegeben, die 16 $\mu$g Arylsulfatase enthält. Man inkubiert bei 25° C und verfolgt die Umsetzung mittels HPLC-Analyse. Nach 6 Stunden Inkubation sind über 90 % des Hirudins desulfatiert. Die Retentionszeit des gebildeten Desulfatohirudins beträgt unter den in Beispiel 26 ("Experimentelle Bedingungen a":) beschriebenen Bedingungen 12,8 min. und unter den in Beispiel 26 ("Experimentelle Bedingungen b") beschriebenen Bedingungen 15,45 min.

Die Aminosäurezusammensetzung, die Sequenzanalyse mittels Edman-Abbau und die Bestimmung der C-terminalen Aminosäuren durch Carboxypeptidase Y-Abbau ergeben das für Desulfatohirudin erwartete Ergebnis.

Beispiel 29: Expression von Desulfatohirudin-Verbindungen in Hefe (S. cerevisiae)

Die Expression von Fremdproteinen in Hefe erfordert ein definiertes Expressionssystem. Es soll einen starken Hefe-Promoter, bevorzugt einen induzierbaren Hefe-Promoter, und geeignete Transkriptionsstopsignale auf einem Plasmidvektor enthalten, mit dem Hefezellen transformiert werden können. Der Vektor enthält auch DNA-Sequenzen, bevorzugt Hefe 2$\mu$ Sequenzen, die extrachromosomale Replikation mit hoher Kopienzahl gewährleisten. Er soll ausserdem einen Selektionsmarker für Hefe, bevorzugt das Hefe LEU2 Gen, und pBR322 DNA Sequenzen mit Replikationsursprung und Selektionsmarker, bevorzugt das Ampicillin-Resistenzgen, für die Verwendung in E. coli enthalten. Solche Vektoren sind als sog. Shuttle-Vektoren für die Vermehrung in E. coli und Hefe geeignet.

Ein Expressionssystem, das diese Voraussetzungen erfüllt, ist in der Europäischen Patentanmeldung Nr. 100,561 beschrieben und mit Beispielen für effiziente Expression in Hefe belegt. Fremdgene werden unter der Kontrolle des induzierbaren PHO5 Promoters der sauren Phosphatase aus Hefe exprimiert. PHO5 Promoter, Fremdgen und PHO5 Transcriptionsstop sind in Serie in dem Hefe Vektor pJDB207 integriert, der auch DNA Sequenzen des Hefe 2$\mu$ Plasmids, das Hefe LEU2 Gen, das Ampicillin Resistenzgen und den E. coli Replikationsursprung enthält.

Das Expressionsplasmid pJDB207R/PHO5-HIR wird wie folgt hergestellt:

a) Isolierung des pJDB207 Vektor Fragments:

6 $\mu$g des Plasmids pJDB207R/IF($\alpha$-3) (EP 100,561) werden mit der Restriktionsendonuclease BamHI komplett verdaut. Die entstehenden DNA Fragmente (6,85 kb und 1,15 kb) werden mit Aethanol gefällt und in 400 $\mu$l 50 mM Tris•HCl pH 8.0 aufgenommen. 4,5 Einheiten Kälberdarm-Phosphatase (Boehringer, Mannheim) werden zugegeben. Die Mischung wird 1 Stunde bei 37° C inkubiert, danach wird die Phosphatase bei 65° C für 1,5 Stunden inaktiviert. Die Lösung wird auf 150 mM NaCl gebracht und dann auf eine DE52 (Whatman) Anionenaustauschersäule (100 $\mu$l Volumen) aufgetragen, die vorher mit 10 mM Tris•HCl pH 7,5, 150 mM NaCl, 1 mM EDTA äquilibriert wurde. Nach einem Waschgang mit demselben Puffer wird die DNA mit 400 $\mu$l 1,5 M NaCl, 10 mM Tris•HCl pH 7,5, 1 mM EDTA eluiert und mit Aethanol gefällt. Das grosse 6,85 kb BamHI Fragment wird vom kleinen Fragment auf einem 1,2 %igen Agarosegel in Tris-Borat-EDTA Puffer pH 8,3 abgetrennt.

b) Isolierung des 534 bp PHO5 Promoter Fragments

6 $\mu$g des Plasmids p31/R (EP 100.561) werden mit den Restriktionsendonucleasen EcoRI und BamHI verdaut. Die entstehenden 3 DNA Fragmente werden auf einem 1,2 %igen Agarosegel in Tris-Borat-EDTA Puffer pH 8,3 getrennt. Das 534 bp BamHI-EcoRI Fragment wird isoliert. Es enthält den PHO5 Promoter

einschliesslich der Transkriptionsstartstellen.

c) Isolierung eines 206 bp DNA Fragments mit der für Hirudin kodierenden Sequenz

9 μg des Plasmids pML310 (vgl. Beispiel 13c) werden mit den Restriktionsenzymen BamHI und EcoRI komplett verdaut. Die entstehenden zwei DNA Fragmente werden auf einem 1,2 %igen Agarosegel in Tris-Borat-EDTA Puffer pH 8,3 getrennt. Das 206 bp EcoRI-BamHI Fragment wird isoliert.

d) Ligierung der DNA Fragmente:

Die drei in Abschnitt a-c (s.o.) erwähnten DNA Fragmente werden aus Agarosegelblöcken durch Elektroelution gewonnen, über DE52 (Whatman) Anionen-Austauscher gereinigt, mit Aethanol gefällt und in $H_2O$ resuspendiert.

0,1 pMol (0,45 μg) des 6,85 kb BamHI Vektor Fragmente, 0,3 pMol (0,1 μg) des 534 bp BamHI-EcoRI PHO5 Promoter Fragments und 0,25 pMol (34 ng) des 206 bp EcoRI-BamHI Fragments von pML310 werden in 15 μl 60 mM Tris-HCl pH 7,5, 10 mM $MgCl_2$, 10 mM DTT, 1 mM ATP mit 600 Einheiten T4 DNA Ligase (Biolabs) bei 15° C für 16 Stunden ligiert.

24 transformierte, $amp^R$ Kolonien werden getrennt in LB Medium mit 100 μg/ml Ampicillin kultiviert. Plasmid DNA wird nach der Methode von Holmes et al. (32) isoliert und durch einen HindIII/EcoRI Doppelverdau analysiert. Das Auftreten eines ca. 600 bp grossen EcoRI-HindIII Fragments zeigt an, dass bei dem betreffenden Klon das PHO5 Promoter-Hirudin DNA Fragment in der richtigen Orientierung zu den Transkriptionsstopsignalen auf dem Vektor vorliegt. Wie erwartet haben etwa 50 % der Klone die richtige Orientierung des Inserts. Einer dieser Klone wird als pJDB207R/PHO5-HIR bezeichnet.

e) Transformation von Sacchsromyces cerevisiae GRF18:

Saccharomyces cerevisiae Stamm GRF18 (α, his3-11, his3-15, leu2-3, leu2-112, $can^R$) wird mit dem Plasmid pJDB207R/PHO5-HIR nach dem Protokoll von Hinnen et al. (12) transformiert. Für transformierte Hefezellen wird auf Agarplatten mit Hefe-Minimalmedium ohne Leucin selektioniert. Eine transfozmierte Hefekolonie wird isoliert und als Saccharomyces cerevisiae GRF18/pJDB207R/PHO5-HIR bezeichnet.

f) Züchten von S. cerevisiae GRF18/pJDB207R/PHO5-HIR:

Zellen von S. cerevisiae GRF18/pJDB207R/PHO5-HIR werden in 20 ml Hefe-Minimalmedium (Difco Yeast Nitrogen Base ohne Aminosäuren mit Zusatz von 2 % Glukose und 20 mg/l L-Histidin) bei 30° C geschüttelt und bis zu einer Zelldichte von $3 \cdot 10^7$ Zellen/ml gezüchtet. Die Zellen werden in 0,9 % NaCl gewaschen und dann zum Animpfen von 50 ml Kulturen in low $P_i$-Minimalmedium benutzt. Low $P_i$-Minimalmedium wird entsprechend der Zusammensetzung des Difco Yeast Nitrogen Base Mediums (ohne Aminosäuren) angesetzt, enthält jedoch 0,03 g/l $KH_2PO_4$, 1 g/l KCl, 10 g/l L-Asparagin anstelle von $(NH_4)_2SO_4$, 2 % Glukose und 1 g/l L-Histidin. Die Kulturen werden bis zu einer Zelldichte von $4 \cdot 10^6$ Zellen/ml angeimpft und bei 30° C für 24 Stunden bei 200 upm geschüttelt.

g. Analyse des Produktgemisches aus der Fermentation von S. cerevisiae GRF18/pJDB207R/PHO5-HIR

Die Zellen (vgl. Beispiel 29f) werden wie üblich aufgeschlossen (vgl. z.B. Europäische Patentanmeldung Nr. 100,561). Der mit 1,5 % Essigsäure behandelte Ueberstand wird zentrifugiert und je 2 ml der klaren Lösung am "Speed Vac" Konzentrator und im Hochvakuum getrocknet. Die Proben werden einzeln in 100 μl Wasser gelöst und einer HPLC-Analyse (Bedingungen wie in Beispiel 26a) unterworfen. Die Thrombin-Hemmung der einzelnen Fraktionen wird getestet. Die Fraktion mit einer Retentionszeit von 12,8 min weist eine Thrombin-Hemmung von 89,6 % auf. Gemäss Aminosäurezusammensetzung handelt es sich um Desulfatohirudin.

Beispiel 30: Test-Kit mit monoklonalen anti-Hirudin-Antikörpern zur Bestimmung von Hirudin, kompetitiver Radio-Immunossay

a. Herstellung der monoklonalen Anti-Hirudin-Antikörper

A) Immunisierung von Mäusen

Reines Hirudin (3 mg) in lyophilisierter Form wird in wenig 0,1 % Essigsäure gelöst und dann mit phosphatgepufferter Kochsalzlösung auf 3 ml ergänzt. Der pH wird auf 7,2 eingestellt. Portionen dieser Antigen-Lösung werden mit gleichen Mengen komplettem Freund's Adjuvans oder phosphatgepufferter Salzlösung gemischt.

Weibliche Balb/c Mäuse (8 Wochen alt, erhalten von der Tierfarm Sisseln, Schweiz) werden durch Injektion von 100 µg Hirudin-Lösung in gepufferter Salzlösung intravenös injiziert. Vier Tage später wird die Milz für die Fusion entnommen.

B) Herstellung der Hybridoma und Antikörper-Test

Die Herstellung der Hybridomazellen erfolgt durch Verschmelzung der erhaltenen Milzzellen mit der Myeloma-Zellinie X63-Ag8.6.5.3 (26). Dabei werden $10^8$ Milzzellen und $10^7$ Myelomazellen eingesetzt. Die Fusion wird wie beschrieben (27) durchgeführt.

Die Bestimmung der anti-Hirudin-Aktivität in den Hybridoma-Ueberständen erfolgt mit Hilfe eines Radioimmunoaasays [RIA, (28)].

c) Isolierung und Reinigung der Anti-Hirudin-Antikörper aus Aszites

Balb/c Mäuse werden intraperitoneal mit 0,4 ml Pristan (Carl Roth) vorbehandelt. Nach einer Woche werden 2 bis $5x10^6$ klonierte Hybridoma-Zellen intraperitoneal injiziert. Aszitische Flüssigkeit wird von jeder Maus wiederholt genommen und bei -80° C gefroren. Die gesammelte Flüssigkeit wird aufgetaut und 30 min. bei 4° C mit 16 000 upm zentrifugiert. Das Fett wird abgesaugt und zum verbleibenden Debri-freien Ueberstand langsam unter Rühren bei 0° C 0,9 Volumenäquivalente einer gesättigten Ammoniumsulfatlösung zugetropft. Die erhaltene rohe Immunoglobulin-Fraktion wird unter Verwendung von 0,1 m Tris•HCl (pH 8.2) durch Sephacryl G 2000 (Pharmacia), gemäss den Angaben des Herstellers, gegeben. Aktive Fraktionen werden vereinigt und mit Amicon XM50-Filter (Amicon) konzentriert.

b. Test-Kit für kompetitiven Radio-Immunoassay

Eine nach Beispiel 30aC) hergestellte Lösung von anti-Hirudin-Antikörpern wird mit phosphatgepufferter Salzlösung (PBS-Lösung) auf eine Konzentration von 1 µg pro 100 µl verdünnt. 100 µl dieser Lösung werden 2 Stunden bei 37° C in Plastik-Röhrchen oder auf Plastik-Mikrotiterplatten inkubiert, wobei Antikörper unspezifisch auf die Plastikoberfläche adsorbiert werden. Zur Absättigung der noch freien aktiven Stellen auf der Plastikoberfläche wird mit einer Bovinserum-Albumin-Lösung (BSA-Lösung) nachbehandelt.

Verdünnungsreihen einer Probelösung oder der Standardlösung in BSA-Lösung werden mit je 50 µl einer Lösung von nach bekannter Weise (29) mit radioaktivem $^{125}$Jod markiertem Hirudin der Aktivität 10 000 cpm pro 50 µl versetzt und dann auf der Plastikoberfläche 2 Stunden bei 37° C und anschliessend 12 Stunden bei 4° C inkubiert. Die Röhrchen oder Mikrotiterplatten werden mit phosphatgepufferter Salzlösung gewaschen und die Radioaktivität gemessen. Die Konzentration an Hirudin in der Probelösung wird durch eine mit Standardlösung gemessene Eichkurve bestimmt.

Ein Test-Kit für den beschriebenen Radio-Immunoassay enthält:

| | |
|---|---|
| 2 ml | Lösung von anti-Hirudin-Antikörper aus Beispiel 30aC) mit einer Konzentration von 1 bis 10 mg pro ml, |
| 100 ml | phosphatgepufferte Salzlösung (PBS-Lösung) |
| 100 ml | 0,3 % Bovinserum-Albumin und 0,1% Natriumazid in PBS-Lösung (BSA-Lösung), |
| 2 ml | Lösung von radioaktivem Hirudin der Aktivität 200 000 cpm/ml, |
| 2 ml | Standardlösung enthaltend 100 ng/ml Hirudin, |
| 1 ml | -Röhrchen oder Mikrotiterplatten aus Plastik. |

Beispiel 31: Pharmazeutisches Präparat enthaltend eine Desulfatohirudinverbindung für parenterale Applikation

Eine Lösung, welche eine Desulfatohirudin-Verbindung gemäss Beispiel 27 enthält, wird gegen eine 0,9 %ige NaCl-Lösung dialysiert. Die Konzentration der Lösung wird danach durch Verdünnen mit der gleichen NaCl-Lösung auf 0,2 mg/ml oder 2 mg/ml eingestellt. Diese Lösungen werden durch Ultrafiltration (Membranen mit 0,22 µm Poren) sterilisiert.

Die sterilisierten Lösungen sind direkt verwendbar, z.B. für die intravenöse Verabreichung. In analoger Weise werden parenteral applizierbare Lösungen hergestellt, die Mischungen von Desulfatohirudin-Verbindungen gemäss Beispiel 27 enthalten.

Referenzen


1. J. Dodt et al., FEBS Letters 165, 180 (1984)
2. P. Walsmann et al., Pharmazie 36, 653 (1981)
3. S.A. Narang, Tetrahedron 39, 3 (1983)
4. K.L. Agarwal et al., Angew. Chem. 84, 489 (1972)
5. C.B. Reese, Tetrahedron 34, 3143 (1972)
6. R.L. Letsinger and W.B. Lunsford, J. Am. Chem. Sec. 98, 3655 (1976)
7. K. Itakura et al., J. Am. Chem. Soc. 103, 706 (1981)
8. H.G. Khorana et el., J. Biol. Chem. 251, 565 (1976)
9. S.A. Narang et al., Anal. Biochem. 121, 356 (1982)
10. K. Itakura et el., J. Biol. Chem. 257, 9226 (1982)
11. A.M. Maxam und W. Gilbert, Proc. Natl. Acad. Sci. USA 74, 560 (1977); siehe auch Meth. Enzym. 65, 499 (1980)
12. A. Hinnen et el., Proc. Natl. Acad. Sci. USA 75, 1929 (1978)
13. Anagnostopoulos et al., J. Bacteriol. 81, 741 (1961)
14. M. Mandel et el., J. Mol. Biol. 53, 159 (1970)
15. H.U. Bergmeyer (Hrsgb.), Methods of Enzymatic Analysis, Vol. II, 3. Auflage, S. 314-316, Verlag Chemie, Weinheim 1983.
16. F. Markwardt et el., Thromb. Haemostasis 47, 226 (1982)
17. Köhler und Milstein, Nature 256, 495 (1975)
18. Molecular Cloning, A Laboratory Manual (ed. T. Maniatis et el.), Cold Spring Harbor Lab., 1982, S. 125
19. W. Müller et el., J. Mol. Biol. 124, 343 (1978)
20. M. Grunstein und D.S. Hogness, Proc. Natl. Acad. Sci. USA 72, 3961 (1979)
21. Ish-Horowitz, in loc. cit. 18), S. 368
22. DE-OS 3 111 405 (Genentech)
23. A.C. Peacock et al., Biochemistry 6, 1818 (1967)
24. U.K. Laemmli, Nature 227, 680 (1970)
25. P. Walsmann, Pharmazie 36, 860 (1981)
26. J.F. Kearney et al., J. Immunol. 123, 1548 (1979)
27. S. Alkan et el., Mol. Immunol. 20, 203 (1983)
28. T. Chard, An Introduction to Radioimmunoassay and related Techniques, North-Holland Publ. Comp., Amsterdam 1978
29. A.E. Bolton und W.M. Hunter, Biochem. J. 133, 529 (1973)
30. J. Clarke und J. Carbon, J. Mol. Biol. 120, 517 (1978)
31. J.Y. Chang et el., Methods in Enzymology, 91, 41 (1983)
32. D.S. Holmes et al., Anal. Biochem. 114, 193 (1981)


## Ansprüche

**1.** Ein Expressionsvektor, welcher eine für Desulfatohirudin der Formel

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

kodierende DNA-Sequenz enthält, die von einer Expressionskontrollsequenz derart reguliert wird, dass besagtes Desulfatohirudin in einem mit diesem Expressionsvektor transformierten Mikroorganismus exprimiert wird.

**2.** Ein Expressionsvektor gemäss Anspruch 1, worin die Expressionskontrollsequenz diejenige des E. coli Tryptophanoperons ist.

3. Ein Expressionsvektor gemäss Anspruch 1, worin die Expressionskontrollsequenz diejenige des Hefe-PHO5-Gens ist.

4. Verfahren zur Herstellung eines Expressionsvektors gemäss einem der Ansprüche 1 bis 3, dadurch gekennzeichnet, dass man in eine Vektor-DNA, welche eine Expressionskontrollsequenz enthält, eine für Desulfatohirudin der Formel

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

kodierende DNA-Sequenz derart einfügt, dass die Expressionskontrollsequenz besagte DNA-Sequenz reguliert.

5. Ein Mikroorganismus, der mit einem Expressionsvektor gemäss einem der Ansprüche 1 bis 3 transformiert ist.

6. Ein transformierter Mikroorganismus gemäss Anspruch 5, ausgewählt aus einem Stamm von Escherichia coli, Bacillus subtilis oder Saccharomyces cerevisiae.

7. Ein transformierter Escherichia coli-Stamm gemäss Anspruch 5.

8. Ein transformierter Saccharomyces cerevisiae-Stamm gemäss Anspruch 5.

9. Verfahren zur Herstellung eines transformierten Mikroorganismus gemäss einem der Ansprüche 5-8, dadurch gekennzeichnet, dass man einen Mikroorganismus mit einem Expressionsvektor gemäss einem der Ansprüche 1-3 transformiert.

10. Verfahren zur Herstellung von biologisch aktivem Desulfatohirudin der Formel

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

( I )

und Salzen davon, dadurch gekennzeichnet, dass mit einem Expressionsvektor, welcher eine von einer Expressionskontrollsequenz regulierte, für besagte Hirudin-Verbindung kodierende DNA-Sequenz enthält, transformierte Mikroorganismen in einem flüssigen Nährmedium, welches assimilierbare Kohlenstoff- und Stickstoffquellen enthält, kultiviert werden und besagtes Desulfatohirudin aus den Wirtszellen freigesetzt und isoliert wird.

11. Verfahren gemäss Anspruch 10, dadurch gekennzeichnet, dass man ein erhaltenes freies Desulfatohirudin in ein Salz desselben oder ein erhaltenes Desulfatohirudin-Salz in die freie Verbindung überführt.

Patentansprüche für folgenden Vertragsstaat: AT

1. Verfahren zur Herstellung eines Expressionsvektors, welcher eine für Desulfatohirudin der Formel

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

kodierende DNA-Sequenz enthält, die von einer Expressionskontrollsequenz derart reguliert wird, dass besagtes Desulfatohirudin in einem mit diesem Expressionsvektor transformierten Mikroorganismus exprimiert wird, dadurch gekennzeichnet, dass man in eine Vektor-DNA, welche eine Expressionskontrollsequenz enthält, eine für besagtes Desulfatohirudin kodierende DNA-Sequenz derart einfügt, dass die Expressionskontrollsequenz besagte DNA-Sequenz reguliert.

2. Verfahren gemäss Anspruch 1, dadurch gekennzeichnet, dass die Expressionskontrollsequenz diejenige des E. coli Tryptophanoperons ist.

3. Verfahren gemäss Anspruch 1, dadurch gekennzeichnet, dass die Expressionskontrollsequenz diejenige des Hefe-PHO5-Gens ist.

4. Verfahren zur Herstellung eines transformierten Mikroorganismus, der einen gemäss einem der Ansprüche 1-3 herstellbaren Expressionsvektor enthält, dadurch gekennzeichnet, dass man einen Mikroorganismus mit einem Expressionsvektor, der eine von einer Expressionskontrollsequenz regulierte, für Desulfatohirudin der Formel

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

kodierende DNA-Sequenz enthält, transformiert.

5. Verfahren gemäss Anspruch 4, dadurch gekennzeichnet, dass der Mikroorganismus ein Stamm von Escherichia coli, Bacillus subtilis oder Saccharomyces cerevisiae ist.

6. Verfahren gemäss Anspruch 4, dadurch gekennzeichnet, dass der Mikroorganismus ein Escherichia coli-Stamm ist.

7. Verfahren gemäss Anspruch 4, dadurch gekennzeichnet, dass der Mikroorganismus ein Saccharomyces cerevisiae-Stamm ist.

8. Verfahren zur Herstellung von biologisch aktivem Desulfatohirudin der Formel

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

(I),

und Salzen davon, dadurch gekennzeichnet, dass mit einem Expressionsvektor, welcher eine von einer Expressionskontrollsequenz regulierte, für besagte Hirudin-Verbindung kodierende DNA-Sequenz enthält, transformierte Mikroorganismen in einem flüssigen Nährmedium, welches assimilierbare Kohlenstoff- und Stickstoffquellen enthält, kultiviert werden und besagtes Desulfatohirudin aus den Wirtszellen freigesetzt und isoliert wird.

9. Verfahren gemäss Anspruch 8, dadurch gekennzeichnet, dass man ein erhaltenes freies Desulfatohirudin in ein Salz desselben oder ein erhaltenes Desulfatohirudin-Salz in die freie Verbindung überführt.

**Claims**

1. An expression vector that contains a DNA sequence coding for desulphatohirudin of the formula

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln ,

which sequence is regulated by an expression control sequence in such a manner that the said desulphatohirudin is expressed in a microorganism transformed with that expression vector.

2. An expression vector according to claim 1, in which the expression control sequence is that of the E. coli tryptophan operon.

3. An expression vector according to claim 1, in which the expression control sequence is that of the yeast-PHO5 gene.

4. A process for the manufacture of an expression vector according to any one of claims 1 to 3, characterised in that a DNA sequence coding for desulphatohirudin of the formula

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

is inserted in such a manner into a vector DNA that contains an expression control sequence that the expression control sequence regulates the said DNA sequence.

5. A microorganism that is transformed with an expression vector according to any one of claims 1 to 3.

6. A transformed microorganism according to claim 5, wherein the microorganism is selected from a strain of Escherichia coli, Bacillus subtilis or Saccharomyces cerevisiae.

7. A transformed Escherichia coli strain according to claim 5.

8. A transformed Saccharomyces cerevisiae strain according to claim 5.

9. A process for the manufacture of a transformed microorganism according to any one of claims 5-8, characterised in that a microorganism is transformed with an expression vector according to any one of claims 1-3.

10. A process for the manufacture of biologically active desulfatohirudin of the formula

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

(I)

and salts thereof, characterised in that microorganisms transformed with an expression vector that contains a DNA sequence coding for the said hirudin compound and regulated by an expression control sequence are cultivated in a liquid nutrient medium that contains assimilable carbon and nitrogen sources, and the said desulphatohirudin is freed from the host cells and isolated.

**11.** A process according to claim 10, characterised in that a free desulphatohirudin obtained is converted into a salt thereof or a desulphatohirudin salt obtained is converted into the free compound.

Claims for the following Contracting State: AT

**1.** A process for the manufacture of an expression vector that contains a DNA sequence coding for desulphatohirudin of the formula

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln,

which sequence is regulated by an expression control sequence in such a manner that the said desulphatohirudin is expressed in a microorganism transformed with that expression vector, characterised in that a DNA sequence coding for the said desulphatohirudin is inserted in such a manner into a vector DNA that contains an expression control sequence that the expression control sequence regulates the said DNA sequence.

**2.** A process according to claim 1, characterised in that the expression control sequence is that of the E. coli tryptophan operon.

**3.** A process according to claim 1, characterised in that the expression control sequence is that of the yeast-PHO5 gene.

**4.** A process for the manufacture of a transformed microorganism that contains an expression vector that can be manufactured according to any one of claims 1-3, characterised in that a microorganism is transformed with an expression vector that contains a DNA sequence coding for desulphatohirudin of the formula

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

and regulated by an expression control sequence.

**5.** A process according to claim 4, characterised in that the microorganism is a strain of Escherichia coli, Bacillus subtilis or Saccharomyces cerevisiae.

**6.** A process according to claim 4, characterised in that the microorganism is an Escherichia coli strain.

**7.** A process according to claim 4, characterised in that the microorganism is a Saccharomyces cerevisiae strain.

**8.** A process for the manufacture of biologically active desuphatohirudin of the formula

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

(I)

and salts thereof, characterised in that microorganisms transformed with an expression vector that contains a DNA sequence coding for the said hirudin compound and regulated by an expression control sequence are cultivated in a liquid nutrient medium that contains assimilable carbon and nitrogen sources, and the said desulphatohirudin is freed from the host cells and isolated.

9. A process according to claim 8, characterised in that a free desulphatohirudin obtained is converted into a salt thereof or a desulphatohirudin salt obtained is converted into the free compound.

## Revendications

1. Vecteur d'expression qui contient une séquence d'ADN codant pour la désulfatohirudine de formule

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

qui est régulée par une séquence de commande d'expression de manière que la désulfatohirudine en question soit exprimée dans un microorganisme transformé avec ce vecteur d'expression.

2. Vecteur d'expression selon la revendication 1, dans lequel la séquence de commande d'expression est celle de l'opéron Tryptophane d' E. coli.

3. Vecteur d'expression selon la revendication 1, dans lequel la séquence de commande d'expression est celle du gène PHO5 de levure.

4. Procédé de préparation d'un vecteur d'expression selon l'une des revendications 1 à 3, caractérisé en ce qu'on introduit dans un ADN vecteur qui contient une séquence de commande d'expression une séquence d'ADN codant pour la désulfatohirudine de formule

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

de manière que la séquence de commande d'expression régule ladite séquence d'ADN.

5. Microorganisme qui est transformé avec un vecteur d'expression selon l'une des revendications 1 à 3.

6. Microorganisme transformé selon la revendication 5, choisi dans une soucne d' Escherichia coli, Bacillus subtilis ou Saccharomyces cerevisiae.

7. Souche d' Escherichia coli transformée selon la revendication 5.

8. Souche de Saccharomyces cerevisiae transformée selon la revendication 5.

9. Procédé de préparation d'un microorganisme transformé selon l'une des revendication 5-8, caractérisé en ce qu'on transforme un microorganisme avec un vecteur d'expression selon l'une des revendications 1-3.

**10.** Procédé de préparation de désulfatohirudine biologiquement active de formule

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

(I)

et de ses sels, caractérisé en ce qu'on cultive des microorganismes transformés avec un vecteur d'expression qui contient une séquence d'ADN régulée par une séquence de commande d'expression, codant pour le composé d'hirudine mentionné, dans un milieu nutritif liquide qui contient des sources de carbone et d'azote assimilables, et en ce qu'on libère et isole la désulfatohirudine mentionnée à partir des cellules-hôtes.

**11.** Procédé selon la revendication 10, caractérisé en ce qu'on transforme une désulfatohirudine libre obtenue en un de ses sels ou un sel de désulfatohirudine obtenu en le composé libre.

Revendications pour l'Etat contractant suivant: AT

**1.** Procédé de préparation d'un vecteur d'expression qui contient une séquence d'ADN codant pour la désulfatohirudine de formule

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

qui est régulée par une séquence de commande d'expression de manière que la désulfatohirudine mentionnée soit exprimée dans un microorganisme transformé avec ce vecteur d'expression, caractérisé en ce qu'on introduit dans un ADN de vecteur qui contient une séquence de commande d'expression une séquence d'ADN codant pour la désulfatohirudine en question de manière que la séquence de commande d'expression, ladite séquence d'ADN.

**2.** Procédé selon la revendication 1, caractérisé en ce que la séquence de commande d'expression est celle de l'opéron Tryptophane d' E. coli.

**3.** Procédé selon la revendication 1, caractérisé en ce que la séquence de commande d'expression est celle du gène PHO5 de levure.

**4.** Procédé de préparation d'un microorganisme transformé qui contient un vecteur d'expression que l'on peut préparer selon l'une des revendications 1-3, caractérisé en ce qu'on transforme un microorganisme avec un vecteur d'expression qui contient une séquence d'ADN régulée par une séquence de commande d'expression, codant pour la désulfatohirudine de formule

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

5. Procédé selon la revendication 4, caractérisé en ce que le microorganisme est une souche d' Escherichia coli, Bacillus subtilis ou Saccharomyces cerevisiae.

6. Procédé selon la revendication 4, caractérisé en ce que le microorganisme est une souche d' Escherichia coli.

7. Procédé selon la revendication 7, caractérisé en ce que le microorganisme est une souche de Saccharomyces cerevisiae.

8. Procédé de préparation de désulfatohirudine biologiquement active de formule

ValValTyrThrAspCysThrGluSerGlyGlnAsnLeuCysLeuCysGluGlySerAsnValCys
GlyGlnGlyAsnLysCysIleLeuGlySerAspGlyGluLysAsnGlnCysValThrGlyGluGly
ThrProLysProGlnSerHisAsnAspGlyAspPheGluGluIleProGluGluTyrLeuGln

(I)

et de ses sels, caractérisé en ce qu'on cultive des microorganismes transformés avec un vecteur d'expression, qui contient une séquence d'ADN régulée par une séquence de commande d'expression, codant pour le composé d'hirudine mentionné, dans un milieu nutritif liquide qui contient des sources de carbone et d'azote assimilables et en ce qu'on libère et isole la désulfatohirudine en question à partir des cellules-hôtes.

9. Procédé selon la revendication 8, caractérisé en ce qu'on transforme une désulfatohirudine libre obtenue en un de ses sels, ou un sel de désulfatohirudine obtenu en le composé libre.

Fig. 1 KONSTRUKTION DES PLASMIDS pML300

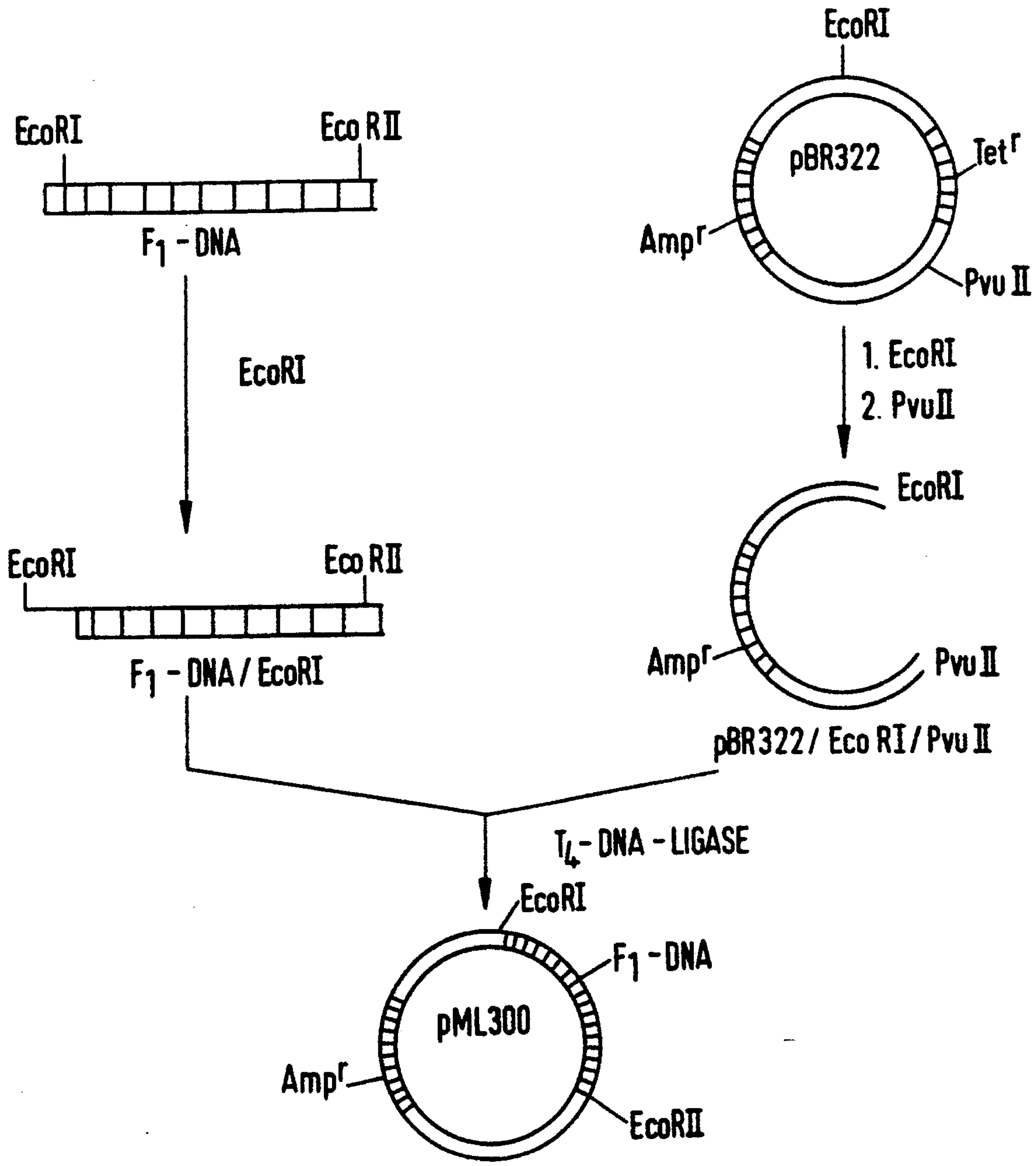

Fig. 2 KONSTRUKTION DES PLASMIDS pML305

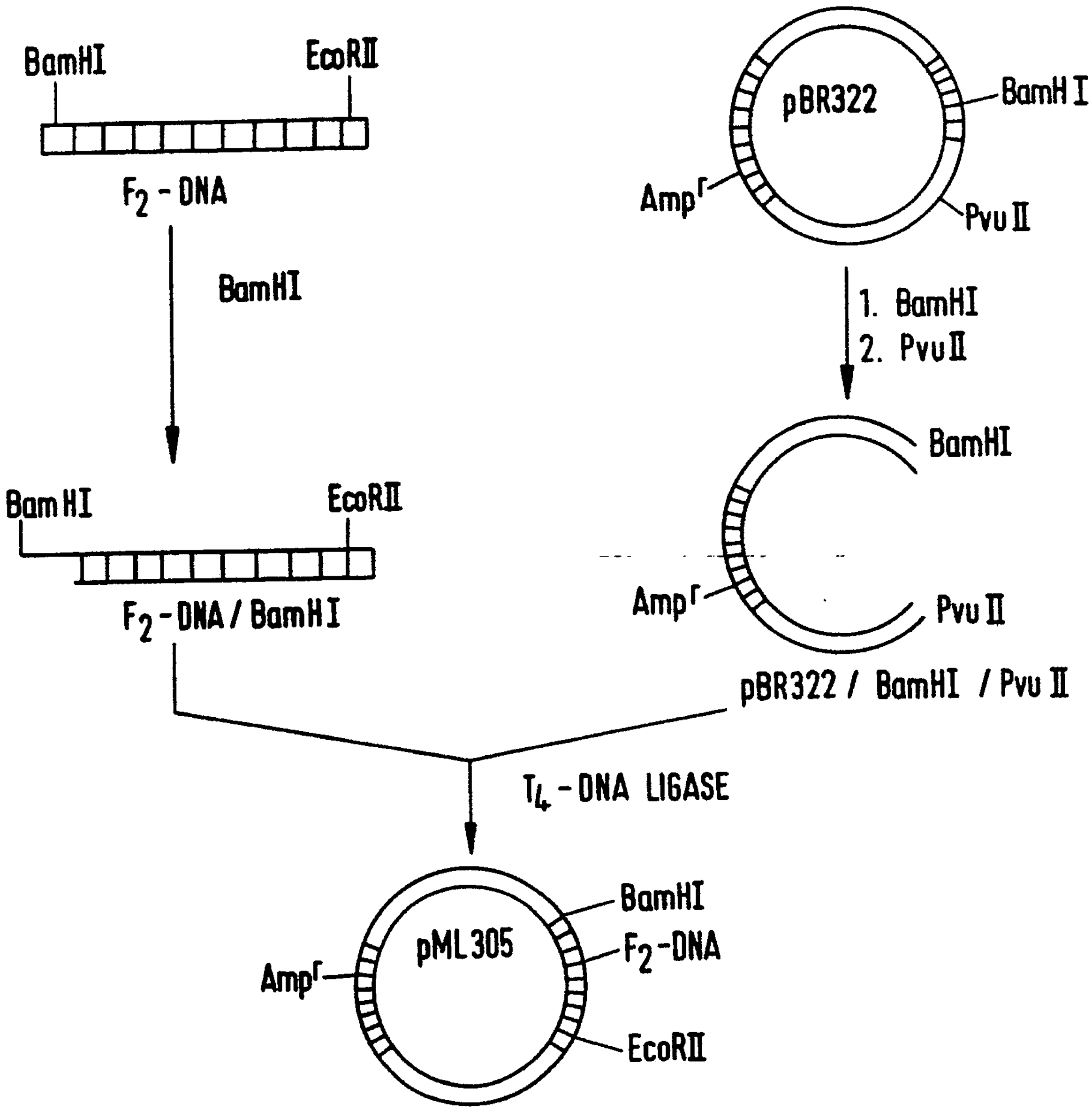

Fig. 3 KONSTRUKTION DES PLASMIDS pHRi148

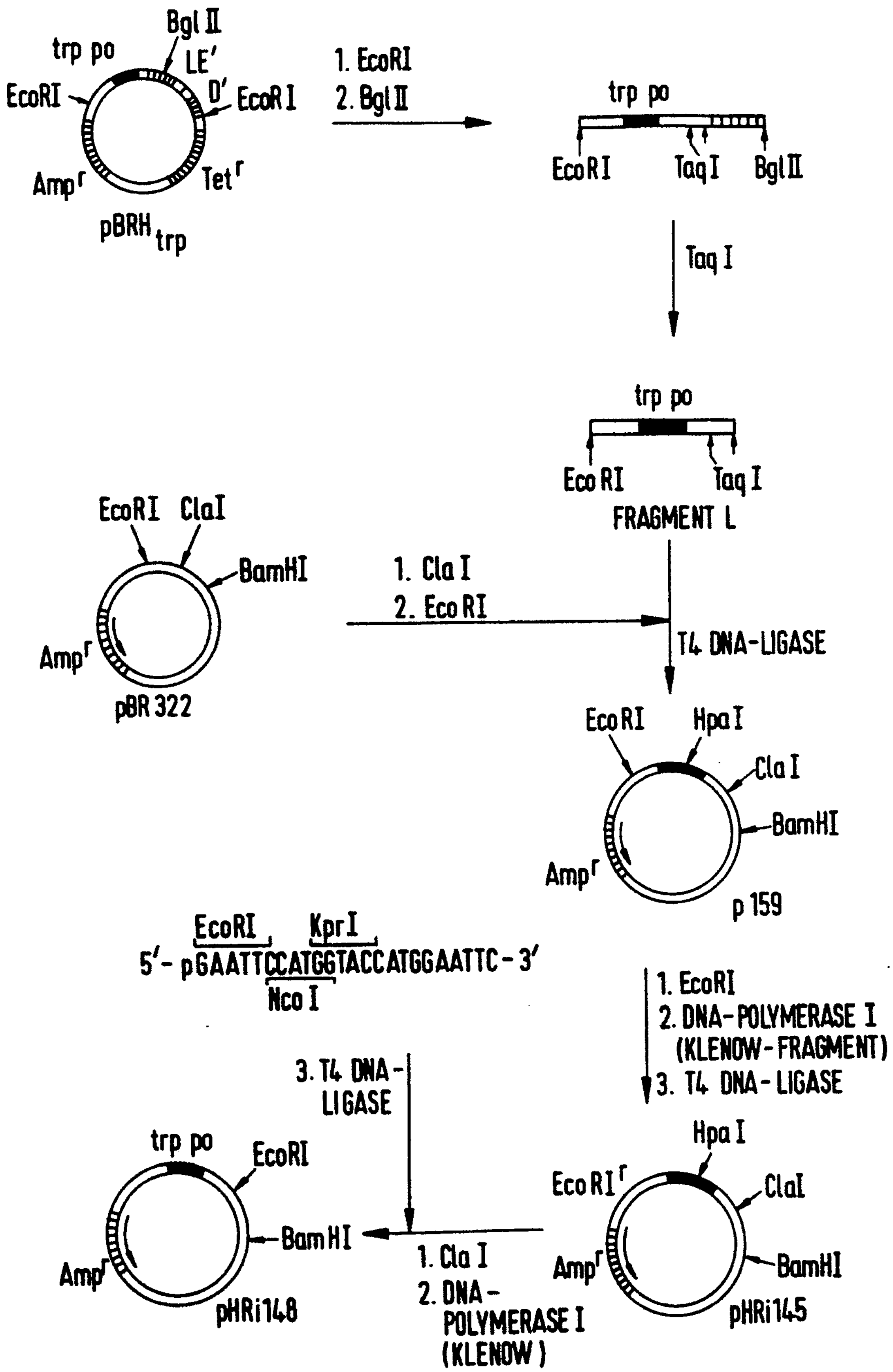

Fig. 4 KONSTRUKTION DES EXPRESSIONSPLASMIDS pML310